# EUROPEAN PATENT APPLICATION

(11) **EP 4 825 973 A1**
(43) Date of publication of application: **21.10.2026**
(21) Application number: 25738595.5
(22) Date of filing: 10.01.2025
(51) Int. Cl.: C12N 15/86, A61K 39/00, A61P 35/00, C07K 19/00, C12N 15/62

(54) **VIRAL PARTICLE, AND PREPARATION METHOD THEREFOR AND USE THEREOF**

(30) Priority: 10.01.2024 CN 202410037635
(71) Applicant: Shenzhen Genocury Biotech Co., Ltd, Shenzhen, Guangdong 518000 (CN)
(72) Inventor: HUANG, Ke, Shenzhen, Guangdong 518110 (CN); LI, Yuhang, Shenzhen, Guangdong 518110 (CN)
(74) Representative: Vogelbruch, Keang
(86) International application number: PCT/CN2025/071920
(87) International publication number: WO 2025/149068

(57) **Abstract**

A viral particle. The surface of the viral particle comprises a T cell activation primary signaling molecule and a T cell activation secondary signaling molecule; the envelope glycoprotein of the viral particle is subjected to a first mutation, so that the receptor binding ability of the viral glycoprotein is weakened or lost with respect to its receptor binding ability before the first mutation; and the envelope glycoprotein of the viral particle can also be subjected to a second mutation, so that the viral particle has enhanced resistance to complement-mediated inactivation or is not subjected to complement-mediated inactivation. The viral particle has improved specificity for targeted activation, stimulation and transduction of non-activated T cells, and is thus more applicable for the preparation of CAR-T cells in subjects.

## Description

### TECHNICAL FIELD

The present invention relates to the field of gene vectors, and in particular to a viral particle, and a preparation method therefor and use thereof.

### BACKGROUND

In the fields of genetic engineering and cell therapy, lipid-nanoparticles ("LNPs"), virus-like particles ("VLPs"), adenovirus, adeno-associated virus, retroviral vectors ("RVVs") and lentiviral vectors ("LVVs") and other vectors are commonly used gene vectors.

Lentiviral vectors and retroviral vectors can integrate exogenous payload genes, such as chimeric antigen receptor ("CAR") genes and the like, into the genome of host cells, enabling stable expression of CAR genes in host cells, and are thus widely used for the preparation of CAR-T cells *in vitro* and *in vivo*.

By deleting virulence genes of HIV (Human Immunodeficiency Virus), such as genes env, vif, vpr, vpu and nef, and performing multiple attenuations, self-inactivating, replication-defective and biosafe lentiviral vectors or retroviral vectors are constructed.

However, how to improve the efficiency and specificity of targeted activation and transduction of T cells *in vitro* and *in vivo* in patients, the efficiency of membrane surface expression of CAR molecule in T cells, and the killing efficiency of the prepared CAR-T cells remains a technical problem to be solved in the technical field of CAR-T cell therapy.

### SUMMARY

In view of the above, to solve at least one of the above technical problems, one aspect of the present invention provides a viral particle,
(a) the surface of the viral particle comprises a T cell activation primary signaling molecule and a T cell activation secondary signaling molecule; and
(b) the surface of the viral particle comprises a glycoprotein, wherein the glycoprotein is subjected to a first mutation, so that the ability of the glycoprotein to bind a glycoprotein receptor is weakened or lost with respect to the ability before the first mutation.

In some embodiments of the present invention, the viral particle targetedly activates non-activated T cells.

In some embodiments of the present invention, the viral particle is a lentiviral vector or a retroviral vector.

Non-activated T cells refer to T cells that do not proliferate, do not differentiate, are in a resting state, have not recognized antigens, and have not been activated or stimulated by a T cell activation primary signaling molecule or T cell activation primary and secondary signaling molecules, for example, T cells in the G0 phase of the cell cycle, resting/quiescent T cells, or naive T cells.

The T cell activation primary signaling molecule binds to a T cell surface protein and participates in T cell receptor ("TCR")-mediated T cell activation.

In some embodiments of the present invention, the T cell activation primary signaling molecule participates in converting TCR into an active PTK (protein tyrosine kinase), and the active PTK can phosphorylate a series of substrates, thereby producing a large number of downstream signals, which, when appropriately integrated (together with signals from other co-receptors), lead to T cell activation (Smith-Garvin JE, Koretzky GA, Jordan MS. T cell activation. Annu Rev Immunol. 2009; 27:591-619).

In some embodiments of the present invention, the T cell activation primary signaling molecule binds to a T cell endocytic receptor involved in mediating the generation and/or transmission of a T cell activation primary signal, such as CD3.

In some embodiments of the present invention, the T cell activation primary signaling molecule binds to a TCR/CD3 complex.

CD3 forms a TCR/CD3 complex with TCR and participates in the activation of helper T cells (CD4⁺ T cells) and cytotoxic T cells (CD8⁺ T cells).

In some embodiments of the present invention, the T cell activation primary signaling molecule binds to a TCR/CD3 complex or a TCR/CD3 complex subunit; and the TCR/CD3 complex subunit is at least one selected from the group consisting of CD3ε, CD3γ, CD3δ, TCRα and TCRβ.

In some embodiments of the present invention, the TCR/CD3 complex subunit is further selected from TCR γ and TCR ζ.

In some embodiments of the present invention, the T cell activation primary signaling molecule binds to human CD3 ε (Uniprot ID: P07766).

In some embodiments of the present invention, the T cell activation primary signaling molecule comprises an anti-CD3 antibody or an antigen-binding fragment thereof.

In some embodiments of the present invention, the anti-CD3 antibody or antigen-binding fragment thereof comprises an antibody or antigen-binding fragment thereof derived from UCHT1, OKT3, SP34, HuM291 or TR66, or a variant thereof, or a derivative thereof.

In some embodiments of the present invention, the anti-CD3 antibody is an scFv derived from UCHT1 (UCHT1-scFv), wherein the amino acid sequence of the UCHT1-scFv is as set forth in SEQ ID NO: 9; and the amino acid sequences of the HCDR1-3 regions of the UCHT1-scFv are as set forth in SEQ ID NOs: 81-83, respectively, and the LCDR1-3 regions of the UCHT1-scFv are as set forth in SEQ ID NOs: 84-86, respectively.

A T cell activation secondary signaling molecule, also known as a co-stimulatory signal molecule, binds to other T cell surface receptors to provide additional signals necessary for avoiding anergy and achieving effective T cell activation (Smith-Garvin JE, Koretzky GA, Jordan MS. T cell activation. Annu Rev Immunol. 2009; 27:591-619).

In some embodiments of the present invention, the T cell activation secondary signaling molecule binds to a T cell endocytic receptor involved in mediating the generation and/or transmission of a T cell activation secondary signal, such as CD28.

In some embodiments of the present invention, the T cell activation secondary signaling molecule binds to CD28.

In some embodiments of the present invention, the CD28 is human CD28 (Uniprot ID: P10747).

Although other cell surface receptors (co-stimulatory receptors) can also enhance activation signals through TCR, the co-stimulatory effect mediated by CD28 is stronger than that of other co-stimulatory receptors (Smith-Garvin JE, Koretzky GA, Jordan MS. T cell activation. Annu Rev Immunol. 2009; 27:591-619).

In some embodiments of the present invention, the T cell activation secondary signaling molecule is at least one selected from the group consisting of an anti-CD28 antibody or an antigen-binding fragment thereof and a CD28 ligand or a receptor-binding fragment thereof.

In some embodiments of the present invention, the CD28 ligand or receptor-binding fragment thereof comprises CD80 or a receptor-binding fragment thereof and CD86 or a receptor-binding fragment thereof.

In some embodiments of the present invention, the receptor-binding fragment of the ligand comprises at least one of the extracellular domain, functional fragment and derivative of the ligand.

In some embodiments of the present invention, the CD28 ligand or receptor-binding fragment thereof comprises CD80 or a receptor-binding fragment thereof and CD86 or a receptor-binding fragment thereof.

In some embodiments of the present invention, the CD80 receptor-binding fragment is a CD80 extracellular domain; and the CD86 receptor-binding fragment is a CD86 extracellular domain.

In some embodiments of the present invention, the anti-CD28 antibody or antigen-binding fragment thereof is an scFv derived from 15E8 (15E8-scFv), the amino acid sequence of the 15E8-scFv is as set forth in SEQ ID NO: 13; and the amino acid sequences of the HCDR1-3 regions of the 15E8-scFv are as set forth in SEQ ID NOs: 87-89, respectively, and the amino acid sequences of the LCDR1-3 regions of the 15E8-scFv are as set forth in SEQ ID NOs: 90-92, respectively.

In some embodiments of the present invention, the T cell activation secondary signaling molecule comprises an anti-CD28 antibody or an antigen-binding fragment thereof.

In some embodiments of the present invention, when the T cell activation secondary signaling molecule comprises a T cell activation secondary signaling molecule capable of binding to CD28, the T cell activation secondary signaling molecule can further comprise at least one ligand or receptor-binding fragment thereof selected from an ICOS (inducible costimulator) ligand (ICOSL) or a receptor-binding fragment thereof, a 4-1BB ligand (4-1BBL) or a receptor-binding fragment thereof, and an OX40 ligand (OX40L) or a receptor-binding fragment thereof.

Unlike CD28, which is stably expressed on both non-activated and activated T cells, ICOS is inducibly expressed on activated T cells (Hutloff A, Dittrich AM, Beier KC, Eljaschewitsch B, Kraft R, et al. ICOS is an inducible T-cell co-stimulator structurally and functionally related to CD28. Nature 1999; 397:263-6. [PubMed: 9930702]) (Smith-Garvin JE, Koretzky GA, Jordan MS. T cell activation. Annu Rev Immunol. 2009; 27:591-619). The lack of ICOS leads to impaired immune responses similar to, but less severe than, those in CD28 knockout models, suggesting that these two molecules may function in similar pathways (Coyle AJ, Lehar S, Lloyd C, Tian J, Delaney T, et al. The CD28-related molecule ICOS is required for effective T cell-dependent immune responses. Immunity 2000; 13:95-105. [PubMed: 10933398]) (Smith-Garvin JE, Koretzky GA, Jordan MS. T cell activation. Annu Rev Immunol. 2009; 27:591-619).

Co-stimulatory receptors outside the CD28 family, TNFR family members OX40 (CD134) and 4-1BB (CD137), provide co-stimulatory signals through binding to their ligands OX40L and 4-1BBL (Smith-Garvin JE, Koretzky GA, Jordan MS. T cell activation. Annu Rev Immunol. 2009; 27:591-619).

In some embodiments of the present invention, the T cell activation primary signaling molecule and/or T cell activation secondary signaling molecule is directly or indirectly linked to a transmembrane polypeptide and displayed on the surface of the viral particle.

In some embodiments of the present invention, the transmembrane polypeptide is selected from the transmembrane domains of the following proteins:

CD2, CD3, CD4, CD5, CD7, CD8, CD8α, CD8β, CD9, CD16, CD22, CD27, CD28, CD28H, CD30, CD33, CD37, CD40, CD45, CD64, CD80, CD86, CD84, CD154, CD166, CD226, CD244, 4-1BB, OX40, ICOS, ICAM-1, CTLA-4, PD-1, LAG-3, GITR, HVEM, DAP10, DAP12, TIM-1, LIGHT, ICOS, OX40, 2B4, BTLA, DNAM-1, DR3, FcERIγ, IL7, IL12, IL15, SLAM, KIR2DL4, KIR2DS1, KIR2DS2, NKG2C, NKG2D and CS1; and
preferably, the transmembrane polypeptide is a CD8α transmembrane domain.

In some embodiments of the present invention, the T cell activation primary signaling molecule and/or T cell activation secondary signaling molecule is indirectly linked to the transmembrane polypeptide through a linker domain and displayed on the surface of the viral particle.

In some embodiments of the present invention, the T cell activation primary signaling molecule and/or T cell activation secondary signaling molecule is indirectly linked to the transmembrane polypeptide through a linker domain and displayed on the surface of the viral particle;
preferably, the linker domain is selected from:
   (a) an immunoglobulin hinge region, wherein the immunoglobulin hinge region is selected from a wild-type or modified IgG1, IgG2, IgG3, IgG4, IgA and IgD hinge regions;
   (b) a hinge region, wherein the hinge region is selected from the wild-type or modified hinge regions of the following proteins: CD28, CD7, CD8, CD8α, CD8β, CD3, CD45, CD4, CD5, CD8, CD9, CD16, CD22, CD33, CD37, CD134, CD137, ICOS and CD154;
   (c) all or a portion of an Fc domain, wherein the Fc domain is one or more selected from a CH1 domain, a CH2 domain and a CH3 domain;
   (d) a stalk region of type II C-lectin, wherein the type II C-lectin is selected from the stalk regions of CD23, CD69, CD72, CD94, NKG2A and NKG2D; and
   (e) a flexible linker peptide; and
more preferably, the linker domain is a CD8α hinge region.

In some embodiments of the present invention, the linker domain is a human CD8α hinge region.

In some embodiments of the present invention, the human CD8α hinge region has at least about 85%, 86%, 87%, 88%, 89%, 90%, 91%, 92%, 93%, 94%, 95%, 96%, 97%, 98%, 99% or 100% sequence identity to SEQ ID NO: 10.

In some embodiments of the present invention, the T cell activation primary signaling molecule comprises, from N-terminus to C-terminus, the anti-CD3 antibody or antigen-binding fragment thereof, a CD8α hinge region and a CD8α transmembrane domain; and preferably, the anti-CD3 antibody is the UCHT1-scFv.

In some embodiments of the present invention, the T cell activation secondary signaling molecule comprises, from N-terminus to C-terminus, an anti-CD28 antibody or antigen-binding fragment thereof, a CD8α hinge region and a CD8α transmembrane domain; and preferably, the anti-CD28 antibody is the 15E8-scFv.

In some embodiments of the present invention, the T cell activation secondary signaling molecule is at least one selected from the group consisting of human CD80 or a receptor-binding fragment thereof and human CD86 or a receptor-binding fragment thereof;
preferably, the amino acid sequence of the human CD80 has at least about 85%, 86%, 87%, 88%, 89%, 90%, 91%, 92%, 93%, 94%, 95%, 96%, 97%, 98%, 99% or 100% sequence identity to SEQ ID NO: 93; and
preferably, the amino acid sequence of the human CD86 has at least about 85%, 86%, 87%, 88%, 89%, 90%, 91%, 92%, 93%, 94%, 95%, 96%, 97%, 98%, 99% or 100% sequence identity to SEQ ID NO: 95.

In some embodiments of the present invention, the receptor-binding fragment of the human CD80 comprises the extracellular domain and transmembrane domain of the human CD80; and the receptor-binding fragment of the human CD86 comprises the extracellular domain and transmembrane domain of the human CD86;
preferably, the amino acid sequence of the extracellular domain and transmembrane domain of the human CD80 has at least about 85%, 86%, 87%, 88%, 89%, 90%, 91%, 92%, 93%, 94%, 95%, 96%, 97%, 98%, 99% or 100% sequence identity to SEQ ID NO: 94; and
preferably, the amino acid sequence of the extracellular domain and transmembrane domain of the human CD86 has at least about 85%, 86%, 87%, 88%, 89%, 90%, 91%, 92%, 93%, 94%, 95%, 96%, 97%, 98%, 99% or 100% sequence identity to SEQ ID NO: 79.

In some embodiments of the present invention, the transmembrane polypeptide is the glycoprotein, and the glycoprotein is directly or indirectly linked to the T cell activation primary signaling molecule and/or the T cell activation secondary signaling molecule.

In some embodiments of the present invention, the glycoprotein is indirectly linked to the T cell activation primary signaling molecule and/or the T cell activation secondary signaling molecule through the first polypeptide linker.

In some embodiments of the present invention, the T cell activation primary signaling molecule is directly or indirectly linked to the T cell activation secondary signaling molecule.

In some embodiments of the present invention, the T cell activation primary signaling molecule is indirectly linked to the T cell activation secondary signaling molecule through the second polypeptide linker.

In some embodiments of the present invention, the polypeptide linker is a flexible linker peptide.

In some embodiments of the present invention, the flexible linker peptide is selected from a (G₄S)ₙ linker peptide, linker 1: GSTSGSGKPGSGEGSTKG (SEQ ID NO: 97) and linker 2: GSSGGSGGGGSGGGGSGGGGSSG (SEQ ID NO: 98), wherein, n = 1 to 4.

In some embodiments of the present invention,
(a) the glycoprotein is indirectly linked to the anti-CD3 antibody or antigen-binding fragment thereof through a (G₄S)ₙ linker peptide, and the anti-CD3 antibody or antigen-binding fragment thereof is indirectly linked to the anti-CD28 antibody or antigen-binding fragment thereof, a human CD86 extracellular domain or a human CD80 extracellular domain through a (G₄S)ₙ linker peptide; and/or
(b) the glycoprotein is indirectly linked to the anti-CD28 antibody or antigen-binding fragment thereof, a human CD86 extracellular domain or a human CD80 extracellular domain through a (G₄S)ₙ linker peptide, and the anti-CD28 antibody or antigen-binding fragment thereof, the human CD86 extracellular domain or the human CD80 extracellular domain is indirectly linked to the anti-CD3 antibody or antigen-binding fragment thereof through a (G₄S)ₙ linker peptide;

preferably, n = 3;
preferably, the anti-CD3 antibody or antigen-binding fragment thereof is the UCHT1-scFv;
preferably, the anti-CD28 antibody or antigen-binding fragment thereof is the 15E8-scFv.

In some embodiments of the present invention, the glycoprotein is indirectly linked to the UCHT1-scFv through a (G₄S)₃ linker peptide, and the UCHT1-scFv is indirectly linked to the 15E8-scFv through a (G₄S)₃ linker peptide.

In some embodiments of the present invention, the glycoprotein is indirectly linked to the 15E8-scFv through a (G₄S)₃ linker peptide, and the 15E8-scFv is indirectly linked to the UCHT1-scFv through a (G₄S)₃ linker peptide.

In some embodiments of the present invention, the glycoprotein is at least one selected from the group consisting of an envelope glycoprotein of a vesicular stomatitis virus strain and a variant thereof, an envelope glycoprotein of baboon endogenous retrovirus BaEV and a variant thereof, an envelope glycoprotein RD114 of feline endogenous retrovirus and a variant thereof, and an envelope glycoprotein GALV of gibbon ape leukemia virus and a variant thereof.

In some embodiments of the present invention, the glycoprotein is at least one selected from the group consisting of an envelope glycoprotein of a vesicular stomatitis virus strain and a variant thereof;
preferably, the envelope glycoprotein of the vesicular stomatitis virus strain and variants thereof comprise the following envelope glycoproteins and variants thereof: an envelope glycoprotein of a vesicular stomatitis virus Indiana strain and a variant thereof, an envelope glycoprotein of a vesicular stomatitis virus Cocal strain and a variant thereof, an envelope glycoprotein of a vesicular stomatitis virus Maraba strain and a variant thereof, an envelope glycoprotein of a vesicular stomatitis virus Morreton strain and a variant thereof, an envelope glycoprotein of a vesicular stomatitis virus Alagoas strain and a variant thereof, an envelope glycoprotein of a vesicular stomatitis virus New Jersey strain and a variant thereof, an envelope glycoprotein of a vesicular stomatitis virus Carajas strain and a variant thereof, an envelope glycoprotein of a vesicular stomatitis virus Chandipura strain and a variant thereof, an envelope glycoprotein of a vesicular stomatitis virus Eptesicus strain and a variant thereof, an envelope glycoprotein of a vesicular stomatitis virus Isfahan strain and a variant thereof, an envelope glycoprotein of a vesicular stomatitis virus Jurona strain and a variant thereof, an envelope glycoprotein of a vesicular stomatitis virus Malpais strain and a variant thereof, an envelope glycoprotein of a vesicular stomatitis virus Perinet strain and a variant thereof, an envelope glycoprotein of a vesicular stomatitis virus Piry strain and a variant thereof, an envelope glycoprotein of a vesicular stomatitis virus Radi strain and a variant thereof, an envelope glycoprotein of a vesicular stomatitis virus Rhinolopus strain and a variant thereof, and an envelope glycoprotein of a vesicular stomatitis virus Yug Bogdanovac strain and a variant thereof.

The envelope glycoprotein of a vesicular stomatitis virus strain, such as the envelope glycoproteins of the Indiana strain (VSV-G) and the Cocal strain (Cocal-G), can bind to a low density lipoprotein receptor ("LDL-R") widely expressed on the surface of various cells, and thus has a broad infectivity;
the extracellular domain of the envelope glycoprotein of the vesicular stomatitis virus Indiana strain comprises the amino acid sequence as set forth in SEQ ID NO: 1; the extracellular domain of the envelope glycoprotein of the vesicular stomatitis virus Cocal strain comprises the amino acid sequence as set forth in SEQ ID NO: 2.

In some embodiments of the present invention, the full-length protein of the wild-type VSV-G (including the VSV-G signal peptide) has the amino acid sequence as set forth in SEQ ID NO: 22,
wherein the amino acid sequence as set forth at sites 1 to 16 of SEQ ID NO: 22:
MKCLLYLAFLFIGVNC is the amino acid sequence of the signal peptide of the wild-type VSV-G.

In some embodiments of the present invention, the full-length protein of the wild-type Cocal-G (including the Cocal-G signal peptide) has the amino acid sequence as set forth in SEQ ID NO: 99,
wherein the sequence as set forth at sites 1 to 17 of SEQ ID NO: 99:
MNFLLLTFIVLPLCSHA is the amino acid sequence of the signal peptide of the wild-type Cocal-G.

Wild-type VSV-G extracellular domain is:

KFTIVFPHNQKGNWKNVPSNYHYCPSSSDLNWHNDLIGTALQVKMPKSHKAIQADG
WMCHASKWVTTCDFRWYGPKYITHSIRSFTPSVEQCKESIEQTKQGTWLNPGFPPQS
CGYATVTDAEAVIVQVTPHHVLVDEYTGEWVDSQFINGKCSNYICPTVHNSTTWHS
DYKVKGLCDSNLISMDITFFSEDGELSSLGKEGTGFRSNYFAYETGGKACKMQYCKH
WGVRLPSGVWFEMADKDLFAAARFPECPEGSSISAPSQTSVDVSLIQDVERILDYSLC
QETWSKIRAGLPISPVDLSYLAPKNPGTGPAFTIINGTLKYFETRYIRVDIAAPILSRMV
GMISGTTTERELWDDWAPYEDVEIGPNGVLRTSSGYKFPLYMIGHGMLDSDLHLSSK
AQVFEHPHIQDAASQLPDDESLFFGDTGLSKNPIELVEGWFSSWKSS (SEQ ID NO: 1).

Wild-type Cocal-G extracellular domain is:

KFSIVFPQSQKGNWKNVPSSYHYCPSSSDQNWHNDLLGITMKVKMPKTHKAIQADG
WMCHAAKWITTCDFRWYGPKYITHSIHSIQPTSEQCKESIKQTKQGTWMSPGFPPQN

CGYATVTDSVAVVVQATPHHVLVDEYTGEWIDSQFPNGKCETEECETVHNSTVWYS
DYKVTGLCDATLVDTEITFFSEDGKKESIGKPNTGYRSNYFAYEKGDKVCKMNYCK
HAGVRLPSGVWFEFVDQDVYAAAKLPECPVGATISAPTQTSVDVSLILDVERILDYSL
CQETWSKIRSKQPVSPVDLSYLAPKNPGTGPAFTIINGTLKYFETRYIRIDIDNPIISKM
VGKISGSQTERELWTEWFPYEGVEIGPNGILKTPTGYKFPLFMIGHGMLDSDLHKTSQ
AEVFEHPHLAEAPKQLPEEETLFFGDTGISKNPVELIEGWFSSWKST (SEQ ID NO: 2).

Wild-type VSV-G full-length protein is:

MKCLLYLAFLFIGVNCKFTIVFPHNQKGNWKNVPSNYHYCPSSSDLNWHNDLIGTAL
QVKMPKSHKAIQADGWMCHASKWVTTCDFRWYGPKYITHSIRSFTPSVEQCKESIEQ
TKQGTWLNPGFPPQSCGYATVTDAEAVIVQVTPHHVLVDEYTGEWVDSQFINGKCS
NYICPTVHNSTTWHSDYKVKGLCDSNLISMDITFFSEDGELSSLGKEGTGFRSNYFAY
ETGGKACKMQYCKHWGVRLPSGVWFEMADKDLFAAARFPECPEGSSISAPSQTSVD
VSLIQDVERILDYSLCQETWSKIRAGLPISPVDLSYLAPKNPGTGPAFTIINGTLKYFET
RYIRVDIAAPILSRMVGMISGTTTERELWDDWAPYEDVEIGPNGVLRTSSGYKFPLYM
IGHGMLDSDLHLSSKAQVFEHPHIQDAASQLPDDESLFFGDTGLSKNPIELVEGWFSS
WKSSIASFFFIIGLIIGLFLVLRVGIHLCIKLKHTKKRQIYTDIEMNRLGK (SEQ ID
NO:22).

Wild-type Cocal-G full-length protein is:

MNFLLLTFIVLPLCSHAKFSIVFPQSQKGNWKNVPSSYHYCPSSSDQNWHNDLLGITM
KVKMPKTHKAIQADGWMCHAAKWITTCDFRWYGPKYITHSIHSIQPTSEQCKESIKQ
TKQGTWMSPGFPPQNCGYATVTDSVAVVVQATPHHVLVDEYTGEWIDSQFPNGKCE
TEECETVHNSTVWYSDYKVTGLCDATLVDTEITFFSEDGKKESIGKPNTGYRSNYFA
YEKGDKVCKMNYCKHAGVRLPSGVWFEFVDQDVYAAAKLPECPVGATISAPTQTS
VDVSLILDVERILDYSLCQETWSKIRSKQPVSPVDLSYLAPKNPGTGPAFTIINGTLKY
FETRYIRIDIDNPIISKMVGKISGSQTERELWTEWFPYEGVEIGPNGILKTPTGYKFPLF
MIGHGMLDSDLHKTSQAEVFEHPHLAEAPKQLPEEETLFFGDTGISKNPVELIEGWFS
SWKSTVVTFFFAIGVFILLYVVARIVIAVRYRYQGSNNKRIYNDIEMSRFRK (SEQ ID
NO: 99).

Activated T cells express LDL-R. Therefore, artificially synthesized, biosafe lentiviral vectors or retroviral vectors typically use wild-type VSV-G to construct their envelope glycoproteins (VSV-G lentiviral vectors or retroviral vectors) to transduce activated T cells.

In some embodiments of the present invention, the glycoprotein is an envelope glycoprotein of a vesicular stomatitis virus Indiana strain or Cocal strain or a variant thereof, and the glycoprotein receptor is a low density lipoprotein receptor LDL-R;
the extracellular domain of the glycoprotein comprises the amino acid sequence as set forth in SEQ ID NO: 1 or SEQ ID NO: 2, or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 1 or SEQ ID NO: 2.

However, LDL-R is widely expressed on the surface of various cells, such as activated T cells, hepatocytes, cardiomyocytes and endothelial cells. Therefore, VSV-G lentiviral vectors or retroviral vectors can also infect other cells through binding to LDL-R, and the targeting of T cell infection is low.

By weakening the ability of VSV-G to bind to LDL-R, while enabling the envelope thereof to comprise T cell activation primary and secondary signaling molecules such as an anti-CD3 antibody and an anti-CD28 antibody, the ability of VSV-G lentiviral vectors or retroviral vectors to targetedly activate and transduce T cells can be effectively improved.

In some embodiments of the present invention, the first mutation comprises the amino acid sequence comprising a mutation of at least one of the following amino acids:
(a) in SEQ ID NO: 1 or SEQ ID NO: 2, substitution or deletion of the amino acid at site 8, substitution or deletion of the amino acid at site 9, substitution or deletion of the amino acid at site 10, substitution or deletion of the amino acid at site 47, substitution or deletion of the amino acid at site 50, substitution or deletion of the amino acid at site 51, substitution or deletion of the amino acid at site 183, substitution or deletion of the amino acid at site 179, substitution or deletion of the amino acid at site 180, substitution or deletion of the amino acid at site 182, substitution or deletion of the amino acid at site 184, substitution or deletion of the amino acid at site 209, substitution or deletion of the amino acid at site 347, substitution or deletion of the amino acid at site 350, substitution or deletion of the amino acid at site 352, substitution or deletion of the amino acid at site 353, substitution of the amino acid at site 354, deletion of amino acids at sites 1-18, deletion of amino acids at sites 19-36, deletion of amino acids at sites 37-51, deletion of amino acids at sites 314-384, deletion of amino acids at sites 321-374, deletion of amino acids at sites 331-364, deletion of amino acids at sites 344-354, and deletion of amino acids at sites 345-353; and
(b) after optimal global alignment with SEQ ID NO: 1 or SEQ ID NO: 2, substitution or deletion of the amino acid at a site corresponding to site 8, substitution or deletion of the amino acid corresponding to site 9, substitution or deletion of the amino acid corresponding to site 10, substitution or deletion of the amino acid corresponding to site 47, substitution or deletion of the amino acid corresponding to site 50, substitution or deletion of the amino acid corresponding to site 51, substitution or deletion of the amino acid corresponding to site 183, substitution or deletion of the amino acid corresponding to site 179, substitution or deletion of the amino acid corresponding to site 180, substitution or deletion of the amino acid corresponding to site 182, substitution or deletion of the amino acid corresponding to site 184, substitution or deletion of the amino acid corresponding to site 209, substitution or deletion of the amino acid corresponding to site 347, substitution or deletion of the amino acid corresponding to site 350, substitution or deletion of the amino acid corresponding to site 352, substitution or deletion of the amino acid at site 353, substitution of the amino acid corresponding to site 354, deletion of amino acids corresponding to sites 1-18, deletion of amino acids corresponding to sites 19-36, deletion of amino acids corresponding to sites 37-51, deletion of amino acids corresponding to sites 314-384, deletion of amino acids corresponding to sites 321-374, deletion of amino acids corresponding to sites 331-364, deletion of amino acids corresponding to sites 344-354, and deletion of amino acids corresponding to sites 345-353 of SEQ ID NO: 1 or SEQ ID NO: 2.

In some embodiments of the present invention, the first mutation comprises the amino acid sequence comprising a mutation of at least one of the following amino acids:
(a) in SEQ ID NO: 1 or SEQ ID NO: 2, deletion of amino acids at sites 331 to 364, deletion of amino acids at sites 344-354, substitution of K47, deletion of K47, and substitution of R354; and
(b) after optimal global alignment with SEQ ID NO: 1 or SEQ ID NO: 2, deletion of amino acids at sites corresponding to sites 331 to 364, deletion of amino acids corresponding to sites 344-354, substitution corresponding to K47, deletion corresponding to K47, and substitution corresponding to R354 of SEQ ID NO: 1 or SEQ ID NO: 2; and
preferably, the first mutation comprises the amino acid sequence comprising a mutation of at least one of the following amino acids:
(a) in SEQ ID NO: 1 or SEQ ID NO: 2, deletion of amino acids at sites 331 to 364, deletion of amino acids at sites 344-354, K47Q, R354Q, and deletion of K47; and
(b) after optimal global alignment with SEQ ID NO: 1 or SEQ ID NO: 2, deletion of amino acids at sites corresponding to sites 331 to 364, deletion of amino acids corresponding to sites 344-354, Q substitution corresponding to K47, Q substitution corresponding to R354, and deletion corresponding to K47 of SEQ ID NO: 1 or SEQ ID NO: 2.

In some embodiments of the present invention, the first mutation comprises the amino acid sequence comprising the following amino acid mutation:
(a) deletion of K47 located in SEQ ID NO: 1 or SEQ ID NO: 2; and
(b) after optimal global alignment with SEQ ID NO: 1 or SEQ ID NO: 2, deletion corresponding to K47 of SEQ ID NO: 1 or SEQ ID NO: 2.

In some embodiments of the present invention, the first mutation comprises the amino acid sequence comprising a mutation of at least one of the following amino acids:
(a) substitution of K47, deletion of K47, substitution of I182, substitution of R354, and substitution of Y209 in SEQ ID NO: 1;
(b) after optimal global alignment with SEQ ID NO: 1, substitution corresponding to K47, deletion corresponding to K47, substitution corresponding to I182, substitution corresponding to R354, and substitution corresponding to Y209 of SEQ ID NO: 1;
(c) substitution of K47, deletion of K47, substitution of V182, substitution of R354, and substitution of Y209 in SEQ ID NO: 2; and
(d) after optimal global alignment with SEQ ID NO: 2, substitution corresponding to K47, deletion corresponding to K47, substitution corresponding to V182, substitution corresponding to R354, and substitution corresponding to Y209 of SEQ ID NO: 2; and
preferably, the first mutation comprises the amino acid sequence comprising a mutation of at least one of the following amino acids:
(a) K47Q or K47A, deletion of K47, I182E or I182D, R354Q or R354A, and Y209Q in SEQ ID NO: 1;
(b) after optimal global alignment with SEQ ID NO: 1, Q or A substitution corresponding to K47, deletion corresponding to K47, E or D substitution corresponding to I182, Q or A substitution corresponding to R354, and Q substitution corresponding to Y209 of SEQ ID NO: 1;
(c) K47Q or K47A, deletion of K47, V182E or V182D, R354Q or R354A, and Y209Q in SEQ ID NO: 2; and
(d) after optimal global alignment with SEQ ID NO: 2, Q or A substitution corresponding to K47, deletion corresponding to K47, E or D substitution corresponding to V182, Q or A substitution corresponding to R354, and Q substitution corresponding to Y209 of SEQ ID NO: 2.

In some embodiments of the present invention, the extracellular domain of the glycoprotein comprises the amino acid sequence as set forth in SEQ ID NO: 3, SEQ ID NO: 4, SEQ ID NO: 23 or SEQ ID NO: 24.

In some embodiments of the present invention, relative to SEQ ID NO: 1, SEQ ID NO: 3 comprises deletion of K47.

In some embodiments of the present invention, relative to SEQ ID NO: 1, SEQ ID NO: 4 comprises R354Q.

In some embodiments of the present invention, relative to SEQ ID NO: 2, SEQ ID NO: 23 comprises deletion of K47.

In some embodiments of the present invention, relative to SEQ ID NO: 2, SEQ ID NO: 24 comprises R354Q.

In some embodiments of the present invention, the extracellular domain of the glycoprotein comprises the amino acid sequence as set forth in SEQ ID NO: 3, SEQ ID NO: 4, SEQ ID NO: 23 or SEQ ID NO: 24.

In some embodiments of the present invention, the extracellular domain of the glycoprotein comprises the amino acid sequence as set forth in SEQ ID NO: 3, SEQ ID NO: 4, SEQ ID NO: 23 or SEQ ID NO: 24, or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 3, SEQ ID NO: 4, SEQ ID NO: 23 or SEQ ID NO: 24.

In some embodiments of the present invention, any of the above glycoproteins subjected to the first mutation retains the ability to mediate membrane fusion and endosomal/lysosomal escape.

In some embodiments of the present invention, the glycoprotein is further subjected to a second mutation, so that the ability of the glycoprotein to antagonize complement-mediated inactivation is enhanced with respect to the ability before the second mutation, or the glycoprotein is not subjected to complement-mediated inactivation.

In some embodiments of the present invention, the glycoprotein subjected to the second mutation is any of the above glycoproteins.

The complement system consists of a series of proteins and is part of the innate immune system. Complement (C) exists in the serum, tissue fluid and cell membrane surface of normal humans and animals, and has enzymatic activity after activation, and can undergo complex cascade reactions. The complement system is initiated by a series of enzymes cutting each other, and finally forms a pore-like membrane attack complex on target microorganisms, causing the microorganisms to rupture and die. Complement components can be activated by antigen-antibody complexes or antibodies, and clear immune complexes through cytolysis, opsonization, phagocytosis and mediation of inflammatory reactions, exhibiting corresponding biological functions. Complement is widely involved in the defense response of the body against microbial infections and immune regulation, and also mediates immunopathological damage reactions, and is an effector system and an effector method system having important biological functions in the body.

The complement components that play a regulatory role exist in a soluble or membrane-bound form, and mainly include properdin (P factor), C1 inhibitor (C1INH), factor I, factor H, C4 binding protein (C4BP), S protein, SP40/40, membrane cofactor protein (MCP), decay accelerating factor (DAF), homologous restriction factor (HRF), and membrane inhibitor of reactive lysis (MIRL) and the like.

VSV-G lentiviral vectors or retroviral vectors entering serum can be recognized and inactivated by complement, making it difficult to efficiently reach target cells to exert effects. Therefore, when applied to the preparation of CAR-T cells *in vivo*, the efficiency of transduction of non-activated T cells by VSV-G lentiviral vectors or retroviral vectors is low.

By subjecting VSV-G or Cocal-G to the second mutation, the ability of VSV-G or Cocal-G to antagonize complement-mediated inactivation is improved, thereby making the mutant VSV-G lentiviral vectors or retroviral vectors more suitable for application to the preparation of CAR-T cells *in vivo*.

In some embodiments of the present invention, the glycoprotein subjected to the second mutation is an envelope glycoprotein of a vesicular stomatitis virus Indiana strain or Cocal strain or a variant thereof, and the extracellular domain of the glycoprotein comprises the amino acid sequence as set forth in SEQ ID NO: 1 or SEQ ID NO: 2, or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 1 or SEQ ID NO: 2.

In some embodiments of the present invention, the second mutation comprises the amino acid sequence comprising a mutation of at least one of the following amino acids:
(a) the amino acid at site 214 of SEQ ID NO: 1 or SEQ ID NO: 2;
(b) after optimal global alignment with SEQ ID NO: 1 or SEQ ID NO: 2, the amino acid at a site corresponding to site 214 of SEQ ID NO: 1 or SEQ ID NO: 2;
(c) the amino acid at site 352 of SEQ ID NO: 1 or SEQ ID NO: 2;
(d) after optimal global alignment with SEQ ID NO: 1 or SEQ ID NO: 2, the amino acid at a site corresponding to site 352 of SEQ ID NO: 1 or SEQ ID NO: 2;
(e) the amino acid at site 50 of SEQ ID NO: 1 or SEQ ID NO: 2;
(f) after optimal global alignment with SEQ ID NO: 1 or SEQ ID NO: 2, the amino acid at a site corresponding to site 50 of SEQ ID NO: 1 or SEQ ID NO: 2;
(g) the amino acid at site 146 of SEQ ID NO: 1 or SEQ ID NO: 2; and
(h) after optimal global alignment with SEQ ID NO: 1 or SEQ ID NO: 2, the amino acid at a site corresponding to site 146 of SEQ ID NO: 1 or SEQ ID NO: 2;

preferably, the amino acid mutation is at least one selected from the group consisting of deletion, insertion and substitution of the amino acid; and
more preferably, the second mutation comprises the amino acid sequence comprising a substitution of at least one of the following amino acids:
   (a) the amino acid at site 214 of SEQ ID NO: 1 or SEQ ID NO: 2;
   (b) after optimal global alignment with SEQ ID NO: 1 or SEQ ID NO: 2, the amino acid at a site corresponding to site 214 of SEQ ID NO: 1 or SEQ ID NO: 2;
   (c) the amino acid at site 352 of SEQ ID NO: 1 or SEQ ID NO: 2;
   (d) after optimal global alignment with SEQ ID NO: 1 or SEQ ID NO: 2, the amino acid at a site corresponding to site 352 of SEQ ID NO: 1 or SEQ ID NO: 2;
   (e) the amino acid at site 50 of SEQ ID NO: 1 or SEQ ID NO: 2;
   (f) after optimal global alignment with SEQ ID NO: 1 or SEQ ID NO: 2, the amino acid at a site corresponding to site 50 of SEQ ID NO: 1 or SEQ ID NO: 2;
   (g) the amino acid at site 146 of SEQ ID NO: 1 or SEQ ID NO: 2; and
   (h) after optimal global alignment with SEQ ID NO: 1 or SEQ ID NO: 2, the amino acid at a site corresponding to site 146 of SEQ ID NO: 1 or SEQ ID NO: 2.

In some embodiments of the present invention, the second mutation comprises the amino acid sequence as set forth in SEQ ID NO: 1 or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 1 comprising at least one of the following site mutations:
(a) substitution of T214, substitution of T352, substitution of K50, and substitution of S146 in SEQ ID NO: 1; and
(b) after optimal global alignment with SEQ ID NO: 1, substitution corresponding to T214, substitution corresponding to T352, substitution corresponding to K50, and substitution corresponding to S146 of SEQ ID NO: 1; and
preferably, the second mutation comprises the amino acid sequence comprising at least one of the following site mutations:
(a) T214N, T352A, K50T and S146T in SEQ ID NO: 1; and
(b) after optimal global alignment with SEQ ID NO: 1, N substitution corresponding to T214, A substitution corresponding to T352, T substitution corresponding to K50 and T substitution corresponding to S146 of SEQ ID NO: 1.

In some embodiments of the present invention, the second mutation comprises the amino acid sequence comprising any one of the combinations of following site mutations:
(a) (1) substitution of T214 and T352 in SEQ ID NO: 1; or (2) substitution of T214, T352, K50 and S146 in SEQ ID NO: 1; and
(b) after optimal global alignment with SEQ ID NO: 1, (1) substitution at sites corresponding to T214 and T352 of SEQ ID NO: 1; or (2) substitution at sites corresponding to T214, T352, K50 and S146 of SEQ ID NO: 1; and
preferably, the second mutation comprises the amino acid sequence comprising any one of the combinations of following site mutations:
(a) (1) T214N and T352A in SEQ ID NO: 1; or (2) T214N, T352A, K50T and S146T in SEQ ID NO: 1; and
(b) after optimal global alignment with SEQ ID NO: 1, (1) N substitution at a site corresponding to T214 and A substitution at a site corresponding to T352 of SEQ ID NO: 1; or (2) N substitution at a site corresponding to T214, A substitution at a site corresponding to T352, T substitution at a site corresponding to K50 and T substitution at a site corresponding to S146 of SEQ ID NO: 1.

In some embodiments of the present invention, the second mutation comprises the amino acid sequence as set forth in SEQ ID NO: 2 or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 2 comprising at least one of the following site mutations:
(a) substitution of K214, substitution of T352, substitution of K50, and substitution of S146 in SEQ ID NO: 2; and
(b) after optimal global alignment with SEQ ID NO: 2, substitution corresponding to K214, substitution corresponding to T352, substitution corresponding to K50, and substitution corresponding to S146 of SEQ ID NO: 2; and
preferably, the second mutation comprises the amino acid sequence comprising at least one of the following site mutations:
(a) K214N, T352A, K50T and S146T in SEQ ID NO: 2; and
(b) after optimal global alignment with SEQ ID NO: 2, N substitution at a site corresponding to K214, A substitution at a site corresponding to T352, T substitution at a site corresponding to K50 and T substitution at a site corresponding to S146 of SEQ ID NO: 2.

In some embodiments of the present invention, the second mutation comprises the amino acid sequence comprising any one of the combinations of following site mutations:
(a) (1) substitution of K214 and T352 in SEQ ID NO: 2; or (2) substitution of K214, T352, K50 and S146 in SEQ ID NO: 2; and
(b) after optimal global alignment with SEQ ID NO: 2, (1) substitution at sites corresponding to K214 and T352 of SEQ ID NO: 2; or (2) substitution at sites corresponding to K214, T352, K50 and S146 of SEQ ID NO: 2; and
preferably, the second mutation comprises the amino acid sequence comprising any one of the combinations of following site mutations:
(a) (1) K214N and T352A in SEQ ID NO: 2; or (2) K214N, T352A, K50T and S146T in SEQ ID NO: 2;
(b) after optimal global alignment with SEQ ID NO: 2, (1) N substitution at a site corresponding to K214, and A substitution at a site corresponding to T352 of SEQ ID NO: 2; or (2) N substitution at a site corresponding to K214, A substitution at a site corresponding to T352, T substitution at a site corresponding to K50 and T substitution at a site corresponding to S146 of SEQ ID NO: 2.

In some embodiments of the present invention, the extracellular domain of the glycoprotein comprises the amino acid sequence as set forth in SEQ ID NO: 5, SEQ ID NO: 6, SEQ ID NO: 7, SEQ ID NO: 21, SEQ ID NO: 25, SEQ ID NO: 26, SEQ ID NO: 27 or SEQ ID NO: 28.

In some embodiments of the present invention, relative to SEQ ID NO: 1, SEQ ID NO: 5 comprises deletion of K47, T214N, T352A, K50T and S146T.

In some embodiments of the present invention, relative to SEQ ID NO: 1, SEQ ID NO: 6 comprises deletion of K47, T214N and T352A.

In some embodiments of the present invention, relative to SEQ ID NO: 1, SEQ ID NO: 7 comprises R354Q, T214N and T352A.

In some embodiments of the present invention, relative to SEQ ID NO: 1, SEQ ID NO: 21 comprises R354Q, T214N, T352A, K50T and S146T.

In some embodiments of the present invention, relative to SEQ ID NO: 2, SEQ ID NO: 25 comprises deletion of K47, K214N, T352A, K50T and S146T.

In some embodiments of the present invention, relative to SEQ ID NO: 2, SEQ ID NO: 26 comprises deletion of K47, K214N and T352A.

In some embodiments of the present invention, relative to SEQ ID NO: 2, SEQ ID NO: 27 comprises R354Q, K214N, T352A, K50T and S146T.

In some embodiments of the present invention, relative to SEQ ID NO: 2, SEQ ID NO: 28 comprises R354Q, K214N and T352A.

In some embodiments of the present invention, any of the above glycoproteins subjected to the second mutation retains the ability to mediate membrane fusion and endosomal/lysosomal escape.

In some embodiments of the present invention, the glycoprotein is an envelope glycoprotein of a vesicular stomatitis virus Indiana strain or a variant thereof;
the extracellular domain of the glycoprotein comprises the amino acid sequence as set forth in SEQ ID NO: 1 or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 1; and
the glycoprotein is subjected to any of the above first mutations, so that the ability of the glycoprotein to bind LDL-R is weakened or lost with respect to the ability before the first mutation; and the glycoprotein can also be subjected to any of the above second mutations, so that the ability of the glycoprotein to antagonize complement-mediated inactivation is enhanced with respect to the ability before the second mutation, or the glycoprotein is not subjected to complement-mediated inactivation.

In some embodiments of the present invention, the glycoprotein is an envelope glycoprotein of a vesicular stomatitis virus Cocal strain or a variant thereof;
the extracellular domain of the glycoprotein comprises the amino acid sequence as set forth in SEQ ID NO: 2 or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 2; and
the glycoprotein is subjected to any of the above first mutations, so that the ability of the glycoprotein to bind LDL-R is weakened or lost with respect to the ability before the first mutation; and the glycoprotein can also be subjected to any of the above second mutations, so that the ability of the glycoprotein to antagonize complement-mediated inactivation is enhanced with respect to the ability before the second mutation, or the glycoprotein is not subjected to complement-mediated inactivation.

In some embodiments of the present invention, any of the above glycoproteins subjected to the first mutation and the second mutation retains the ability to mediate membrane fusion and endosomal/lysosomal escape.

In some embodiments of the present invention, the extracellular domain of the glycoprotein comprises the amino acid sequence as set forth in SEQ ID NO: 5, SEQ ID NO: 6, SEQ ID NO: 7, SEQ ID NO: 21, SEQ ID NO: 25, SEQ ID NO: 26, SEQ ID NO: 27 or SEQ ID NO: 28, or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 5, SEQ ID NO: 6, SEQ ID NO: 7, SEQ ID NO: 21, SEQ ID NO: 25, SEQ ID NO: 26, SEQ ID NO: 27 or SEQ ID NO: 28.

In some embodiments of the present invention, any of the above viral particles comprise an exogenous payload gene.

In some embodiments of the present invention, the exogenous payload gene encodes therapeutic proteins or polypeptides; grouped according to the molecular type thereof, the therapeutic protein or polypeptide is at least one selected from the group consisting of antibody-based drugs, chimeric antigen receptors, T cell receptors, cytokine receptors, cytokines, Fc fusion proteins, anticoagulants, blood factors, bone morphogenetic proteins, engineered protein scaffolds, enzymes, growth factors, hormones, interferons, interleukins and thrombolytic agents.

In some embodiments of the present invention, the exogenous payload gene encodes a chimeric antigen receptor (CAR).

In some embodiments of the present invention, the chimeric antigen receptor comprises an extracellular antigen-binding domain, a transmembrane domain and an intracellular signaling domain.

In some embodiments of the present invention, the extracellular antigen-binding domain of the CAR binds to a disease-related antigen.

In some embodiments of the present invention, the antigen is at least one selected from the group consisting of:
TSHR, CD2, CD3, CD4, CD5, CD7, CD8, CD14, CD15, CD19, CD20, CD21, CD23, CD24, CD25, CD28, CD37, CD38, CD40, CD40L, CD44, CD46, CD47, CD52, CD54, CD56, CD70, CD73, CD80, CD97, CD123, CD22, CD126, CD138, DR4, DR5, TAC, TEM1/CD248, VEGF, GUCY2C, EGP40, EGP-2, EGP-4, CDL33, IFNAR1, DLL3, kappa light chain, TIM3, tEGFR, IL-22Ra, IL-2, ErbB3, ErbB4, MUC16, MAGE-A3, MAGE-A6, NKG2DL, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin (MSLN), IL-1Ra, PSCA, PRSS21, VEGFR2, Lewis-Y, CD24, PDGFR-β, SSEA-4, CD20, AFP, Folate receptor α, Her2/neu/ERBB2, MUC1, EGFR, CS1, CD138, NCAM, Claudin18.2, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6/E7, MAGE-A4, MART-1, WT-1, ETV6-AML, sperm protein 17, XAGE1, Tie2, MAD-CT-1, MAD-CT-2, Fos-related antigen 1, p53, p53 mutant, prostate-specific protein, survivin and telomerase, PCTA-1/Galectin 8, MelanA/MARTI, Ras mutant, hTERT, sarcoma translocation breakpoint, ML-IAP, TMPRSS2 ETS fusion gene/ERG, NA17, PAX3, androgen receptor, CyclinB1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL and NKG2D.

In some embodiments of the present invention, the antigen is GC-C (guanylate cyclase C).

In some embodiments of the present invention, the antigen is CD19.

In some embodiments of the present invention, the antigen is one or more selected from the group consisting of CD19, CD20, BCMA, CD33, HER2 and CEA.

In some embodiments of the present invention, the extracellular antigen-binding domain of the CAR comprises an antibody or antigen-binding fragment thereof and/or a ligand or receptor-binding fragment thereof, wherein the antibody or antigen-binding fragment thereof is at least one selected from the group consisting of an immunoglobulin (full-length antibody), a half-antibody, Fab, Fab', F(ab')₂, an Fv fragment, a single-chain fragment variable (scFv), a disulfide-stabilized antibody (dsFv), a heavy chain variable region (VH) or a light chain variable region (VL) of an antibody, an Fd fragment consisting of a VH and a CH1 domain, a linear antibody, and a single-domain antibody (nanobody).

In some embodiments of the present invention, the extracellular antigen-binding domain of the CAR comprises an scFv.

In some embodiments of the present invention, the extracellular antigen-binding domain of the CAR is monospecific, bi-specific or multi-specific.

In some embodiments of the present invention, the extracellular antigen-binding domain of the CAR is an antibody or antigen-binding fragment thereof and/or a ligand or receptor-binding fragment thereof derived from mouse, rat, monkey, or human or being humanized.

In some embodiments of the present invention, the transmembrane domain of the CAR is derived from the transmembrane domain of at least one of the following proteins:

CD2, CD3, TCR, CD4, CD5, CD7, CD8, CD8α, CD8β, CD9, CD16, CD22, CD27, CD28, CD28H, CD30, CD33, CD37, CD40, CD45, CD64, CD80, CD84, CD154, CD166, CD226, CD244, 4-1BB, OX40, ICOS, ICAM-1, CTLA-4, PD-1, LAG-3, GITR, HVEM, DAP10, DAP12, TIM-1, LIGHT, ICOS, OX40, 2B4, BTLA, DNAM-1, DR3, FcERIγ, IL7, IL12, IL15, SLAM, KIR2DL4, KIR2DS1, KIR2DS2, NKG2C, NKG2D and CS1.

In some embodiments of the present invention, the transmembrane domain of the CAR is derived from the transmembrane domain of CD8α.

In some embodiments of the present invention, the transmembrane domain of the CAR is derived from the transmembrane domain of human CD8α.

In some embodiments of the present invention, the amino acid sequence of the transmembrane domain of human CD8α has at least about 85%, 86%, 87%, 88%, 89%, 90%, 91%, 92%, 93%, 94%, 95%, 96%, 97%, 98%, 99% or 100% sequence identity to SEQ ID NO: 11.

In some embodiments of the present invention, the intracellular signaling domain of the CAR is derived from the intracellular signaling domain of at least one of the following proteins:
CD3ε, CD3γ, CD3δ, CD3ζ, CD79a, CD79b, FcεRIγ, FcεRβ, FcγRIIa, bovine leukemia virus gp30, Epstein-Barr virus (EBV) LMP2A, simian immunodeficiency virus PBj14Nef, DAP10, DAP12 and the intracellular signaling domain of other proteins comprising at least one ITAM in the intracellular signaling domain.

In some embodiments of the present invention, the intracellular signaling domain of the CAR is derived from the intracellular signaling domain of CD3ζ.

In some embodiments of the present invention, the intracellular signaling domain of the CAR is derived from the intracellular signaling domain of human CD3ζ.

In some embodiments of the present invention, the amino acid sequence of the intracellular signaling domain of human CD3ζ has at least about 85%, 86%, 87%, 88%, 89%, 90%, 91%, 92%, 93%, 94%, 95%, 96%, 97%, 98%, 99% or 100% sequence identity to SEQ ID NO: 18.

In some embodiments of the present invention, any of the above chimeric antigen receptors further comprise a hinge region; and the hinge region sequentially links the extracellular antigen-binding domain and the transmembrane domain.

In some embodiments of the present invention, the hinge region of the CAR is derived from the hinge region of at least one of the following proteins:

CD28, CD8, CD8α, CD8β, CD3, CD45, Ig4, CD4, CD5, CD8, CD9, CD16, CD22, CD33, CD37, CD134, CD137, ICOS and CD154.

In some embodiments of the present invention, the hinge region of the CAR is derived from the hinge region of CD8α.

In some embodiments of the present invention, the hinge region of the CAR is derived from the hinge region of human CD8α.

In some embodiments of the present invention, the amino acid sequence of the hinge region of human CD8α has at least about 85%, 86%, 87%, 88%, 89%, 90%, 91%, 92%, 93%, 94%, 95%, 96%, 97%, 98%, 99% or 100% sequence identity to SEQ ID NO: 10.

In some embodiments of the present invention, any of the above chimeric antigen receptors further comprise a costimulatory signaling domain.

In some embodiments of the present invention, the costimulatory signaling domain of the CAR is derived from the costimulatory signaling domain of at least one of the following proteins:
CD28, 4-1BB, CD27, CD2, CD7, CD8, CD8α, CD8β, OX40, CD226, DR3, SLAM, CDS, ICAM-1, NKG2D, NKG2C, B7-H3, 2B4, FcαR1γ, BTLA, GITR, HVEM, DAP10, DAP12, CD30, CD40, CD40L, TIM1, PD-1, LFA-1, LIGHT, JAML, CD244, CD100, ICOS, CD40 and MyD88.

In some embodiments of the present invention, the costimulatory signaling domain of the CAR is derived from the costimulatory signaling domain of 4-1BB.

In some embodiments of the present invention, the costimulatory signaling domain of the CAR is derived from the costimulatory signaling domain of human 4-1BB.

In some embodiments of the present invention, the costimulatory signaling domain of the CAR comprises the costimulatory signaling domain of human 4-1BB and the costimulatory signaling domain of human CD28.

In some embodiments of the present invention, the amino acid sequence of the costimulatory signaling domain of human 4-1BB has at least about 85%, 86%, 87%, 88%, 89%, 90%, 91%, 92%, 93%, 94%, 95%, 96%, 97%, 98%, 99% or 100% sequence identity to SEQ ID NO: 17.

In some embodiments of the present invention, any of the above CARs further comprise a signal peptide; and there is no particular limitation on the signal peptide as long as it can mediate the membrane surface expression of the CAR.

In some embodiments of the present invention, the signal peptide of the CAR is selected from a CD8α signal peptide, a CD28 signal peptide, and an IgG1 signal peptide.

In some embodiments of the present invention, the signal peptide of the CAR is a human CD8α signal peptide.

In some embodiments of the present invention, the amino acid sequence of the signal peptide of human CD8α has at least about 85%, 86%, 87%, 88%, 89%, 90%, 91%, 92%, 93%, 94%, 95%, 96%, 97%, 98%, 99% or 100% sequence identity to SEQ ID NO: 8.

In some embodiments of the present invention, the efficiency of transduction of non-activated T cells by the viral particle is improved relative to a control vector whose surface does not comprise a T cell activation primary signaling molecule and a T cell activation secondary signaling molecule.

In some embodiments of the present invention, after the viral particle contacts a non-activated T cell, the efficiency of expression of the exogenous payload gene in the T cell is improved relative to a control vector whose surface does not comprise a T cell activation primary signaling molecule and a T cell activation secondary signaling molecule.

In some embodiments of the present invention, after the viral particle contacts a non-activated T cell, the efficiency of membrane surface expression of the chimeric antigen receptor in the T cell is improved relative to a control vector whose surface does not comprise a T cell activation primary signaling molecule and a T cell activation secondary signaling molecule.

In some embodiments of the present invention, after the viral particle contacts a non-activated T cell, the killing efficiency of the prepared CAR-T cells is enhanced relative to a control vector whose surface does not comprise a T cell activation primary signaling molecule and a T cell activation secondary signaling molecule.

In some embodiments of the present invention, the control vector comprises at least one T cell targeting molecule on the surface thereof, the T cell targeting molecule binding to a T cell endocytic receptor.

In some embodiments of the present invention, the T cell endocytic receptor is at least one selected from the group consisting of CD5 and CD7.

In some embodiments of the present invention, the T cell targeting molecule binds to CD7.

In some embodiments of the present invention, the T cell targeting molecule comprises at least one selected from the group consisting of an anti-CD7 antibody or antigen-binding fragment thereof and a CD7 ligand or receptor-binding fragment thereof;
optionally, the anti-CD7 antibody or antigen-binding fragment thereof is an scFv derived from monoclonal antibody TH-69 (TH69-scFv); and the amino acid sequence of the TH69-scFv is as set forth in SEQ ID NO: 37; and the amino acid sequences of the HCDR1-3 regions of the TH69-scFv are as set forth in SEQ ID NOs: 38-40, respectively, and the amino acid sequences of the LCDR1-3 regions of the TH69-scFv are as set forth in SEQ ID NOs: 41-43, respectively.

A "control vector" includes, but is not limited to, a control vector having the same structure as the viral particle except that the surface thereof does not comprise the T cell activation primary signaling molecule and the T cell activation secondary signaling molecule.

In another aspect, the present invention provides an engineered T cell, the engineered T cell expressing a chimeric antigen receptor, and the engineered T cell being prepared by contacting a T cell with any of the above viral particles provided by the present invention carrying a polynucleotide encoding a chimeric antigen receptor.

In some embodiments of the present invention, the chimeric antigen receptor includes, but is not limited to, any of the above chimeric antigen receptors.

In some embodiments of the present invention, the T cells include non-activated T cells and activated T cells.

In some embodiments of the present invention, the contact occurs *in vivo* and/or *in vitro* in a subject; and the subject is an individual to which the engineered T cell and/or any of the above viral particles carrying a polynucleotide encoding a chimeric antigen receptor is administered.

In some embodiments of the present invention, the administration is at least one selected from the group consisting of oral, nasal, intravenous, intraperitoneal, intracerebral (intraparenchymal), intraventricular, intramuscular, intraocular, intra-arterial, portal vein, intralesional, sustained release system and implant device administration.

In some embodiments of the present invention, the T cells are non-activated T cells, and the efficiency of expression of the CAR by the engineered T cell is improved; and
the improvement in the expression efficiency is relative to an engineered T cell prepared by contacting a non-activated T cell with a control vector whose surface does not comprise the T cell activation primary signaling molecule and the T cell activation secondary signaling molecule.

In some embodiments of the present invention, the T cells are non-activated T cells, and the killing efficiency of the engineered T cell is improved; and
the improvement in the killing efficiency is relative to an engineered T cell prepared by contacting a non-activated T cell with a control vector whose surface does not comprise the T cell activation primary signaling molecule and the T cell activation secondary signaling molecule.

In some embodiments of the present invention, the control vector is any of the above control vectors.

In another aspect, the present invention provides a composition, the composition comprising a pharmaceutically acceptable excipient or carrier and any one of the following components: any of the above viral particles and engineered T cells provided by the present invention.

In another aspect, the present invention provides the use of any of the above viral particles, engineered T cells or composition in the preparation of a medicament for the prevention and/or treatment of a disease.

In some embodiments of the present invention, the disease is an autoimmune disease or cancer, the cancer including solid cancer and blood cancer.

In some embodiments of the present invention, the cancer is blood cancer.

In some embodiments of the present invention, the cancer is B lymphocyte cancer, B lymphocyte leukemia, Hodgkin lymphoma or multiple myeloma.

In some embodiments of the present invention, the blood cancer is one or more selected from the group consisting of non-Hodgkin lymphoma (NHL), acute B-cell lymphoblastic leukemia (B-ALL), chronic lymphocytic leukemia (CLL), large B-cell lymphoma (LBCL), transplant-ineligible LBCL, diffuse LBCL (DLBCL), high-grade B-cell lymphoma (HGBCL), primary mediastinal B-cell lymphoma (PMBCL), mantle cell lymphoma (MCL), follicular lymphoma (FL), marginal zone lymphoma (MZL), small lymphocytic lymphoma (SLL), precursor B-cell lymphoma/leukemia, Burkitt lymphoma (BL), multiple myeloma (MM), acute myeloid leukemia (AML), primary plasma cell leukemia (pPCL), peripheral T-cell lymphoma (PTCL-NHL), NK/T-cell lymphoma, anaplastic large cell lymphoma (ALCL), intestinal T-cell lymphoma, T-large granular lymphocyte leukemia (T-LGL) and germinal center T-cell lymphoma (FTCL).

In some embodiments of the present invention, the solid cancer is one or more selected from the group consisting of mesothelioma, pancreatic cancer, ovarian cancer, lung cancer, gastric cancer, breast cancer, colorectal cancer, bladder cancer, gastroesophageal junction cancer, biliary tract cancer and gastrointestinal cancer.

In some embodiments of the present invention, the cancer is one or more selected from the group consisting of CD19⁺ cancer, CD20⁺ cancer, BCMA⁺ cancer, CD33⁺ cancer, HER2⁺ cancer and CEA⁺ cancer.

In another aspect, the present invention provides a method for treating cancer in a subject or killing cancer cells in a subject, comprising administering to the subject any of the above viral particles, engineered T cells or composition provided by the present invention; and the viral particles or the composition comprise an exogenous payload gene.

In some embodiments of the present invention, the exogenous payload gene is any of the above exogenous payload genes.

In some embodiments of the present invention, the exogenous payload gene encodes a chimeric antigen receptor.

In some embodiments of the present invention, the chimeric antigen receptor includes, but is not limited to, any of the above chimeric antigen receptors.

In some embodiments of the present invention, the administration is at least one of the above administrations.

In another aspect, the present invention further provides a cell, the cell being configured to produce any of the above viral particles provided by the present invention.

In some embodiments of the present invention, the cell line is at least one selected from the group consisting of NS0 cells, Vero cells, HeLa cells, COS cells, CHO cells, HEK cells, BHK cells and MDCKII cells; and
preferably, the cell is an HEK-293T cell.

In some embodiments of the present invention, the cell is selected from HEK cells.

In some embodiments of the present invention, the cell is an HEK-293T cell.

In some embodiments of the present invention, the subject is a subject in need thereof.

In another aspect, the present invention further provides a method for preparing a viral particle, the method being culturing any of the above cells provided by the present invention to a degree sufficient to produce the viral particle.

In another aspect, the present invention further provides a method for transducing a T cell, the method being contacting a T cell with any of the above viral particles provided by the present invention carrying an exogenous payload gene.

In some embodiments of the present invention, the exogenous payload gene is any of the above exogenous payload genes.

In some embodiments of the present invention, the exogenous payload gene encodes a chimeric antigen receptor.

In some embodiments of the present invention, the chimeric antigen receptor includes, but is not limited to, any of the above chimeric antigen receptors.

In some embodiments of the present invention, the T cells include activated T cells and non-activated T cells.

In some embodiments of the present invention, the contact occurs *in vivo* and/or *in vitro* in a subject; and the subject is an individual to which the T cells transduced by the transducing method and/or any of the above viral particles carrying an exogenous payload gene is administered.

In some embodiments of the present invention, the contact occurs *in vivo* in a subject, and the subject is an individual to which any of the above viral particles carrying an exogenous payload gene is administered.

In some embodiments of the present invention, the viral particle is any of the above viral particles carrying a CAR gene.

In some embodiments of the present invention, the administration is at least one of the above administrations.

The present invention includes the following beneficial effects.

Any of the above viral particles provided by the present invention comprise a T cell activation primary signaling molecule such as an anti-CD3 antibody and a T cell activation secondary signaling molecule such as an anti-CD28 antibody on the surface thereof, and can effectively targetedly activate a non-activated T cell; and the glycoprotein on the surface of the viral particle is subjected to a first mutation, so that the ability of the glycoprotein to bind a glycoprotein receptor is weakened or lost, further significantly improving the ability of the viral particle to targetedly activate and transduce a non-activated T cell; the glycoprotein of the viral particle can also be subjected to a second mutation, so that the ability of the glycoprotein to antagonize complement-mediated inactivation is enhanced, making it more suitable for application to the preparation of CAR-T cells *in vivo*; and compared with a control vector whose surface does not comprise a T cell activation primary signaling molecule and a T cell activation secondary signaling molecule, after the viral particle delivers a CAR gene to the genome of a T cell, the efficiency of membrane surface expression of the CAR molecule in the T cell is higher, and the killing efficiency of the prepared CAR-T cells is better.

As used herein:
"T cell": a T cell is one of several important types of white blood cells in the human immune system and plays an important role in the adaptive immune response. One of the main functions of T cells is immune-mediated cell death, which is mainly performed by two T cell subtypes: CD8⁺ T cells (cytotoxic T cell) and CD4⁺ T cells (helper T cell).

In some embodiments of the present invention, T cells are CD4⁺/CD8⁻, CD4⁻/CD8⁺, CD4⁺/CD8⁺, CD4⁻/CD8⁻ T cells or combinations thereof. In some embodiments of the present invention, CD4⁺ T cells produce IL-2, TFN, TNF or combinations thereof after expressing the CAR and binding to a target cell such as a CD19⁺ tumor cell. In some embodiments of the present invention, CD8⁺ T cells lyse antigen-specific target cells after expressing the CAR and binding to a target cell.

Non-activated T cells refer to T cells that do not proliferate, do not differentiate, are in a resting state, have not recognized antigens, and have not been activated or stimulated by a T cell activation primary signaling molecule such as an anti-CD3 antibody and the like (TCR-CD3 complex-binding molecule), and T cell activation secondary signaling molecule/co-stimulatory molecule such as an anti-CD28 antibody, CD80 and CD86 and the like, a, for example, T cells in the G0 phase of the cell cycle, resting/quiescent T cells or naive T cells. Resting T cells are also referred to as quiescent T cells or natural T cells, and refer to T cells that are not mitotically active or have not been exposed to a cognate antigen presented on an antigen-presenting cell (such as a macrophage or dendritic cell).

An "antibody" refers to a polypeptide or a combination of polypeptides comprising sufficient sequences from an immunoglobulin heavy chain variable region and/or sufficient sequences from an immunoglobulin light chain variable region to specifically bind to an antigen. As used herein, "antibody" encompasses various forms and structures as long as they exhibit the desired antigen-binding activity.

As used herein, "antibody" includes a typical "four-chain antibody", which belongs to an immunoglobulin consisting of two heavy chains (HC) and two light chains (LC); the heavy chain refers to a polypeptide chain composed, in the direction from the N-terminus to the C-terminus, of a heavy chain variable region (VH), a heavy chain constant region CH1 domain, a hinge region (HR), a heavy chain constant region CH2 domain, and a heavy chain constant region CH3 domain; and, when the full-length antibody is of the IgE isotype, optionally further comprising a heavy chain constant region CH4 domain; the light chain refers to a polypeptide chain composed, in the direction from the N-terminus to the C-terminus, of a light chain variable region (VL) and a light chain constant region (CL); and the heavy chains and the heavy and light chains are linked by disulfide bonds to form a "Y"-shaped structure.

In the context of antibodies, the term "variable region" or "variable domain" refers to a domain of an antibody heavy chain or light chain that is involved in binding of the antibody to an antigen. The variable regions of the heavy and light chains (VH and VL regions, respectively) of natural antibodies typically have similar structures, each domain comprising four conserved framework regions (FRs) and three complementarity determining regions (CDRs). (See, for example, Kindt et al., Kuby Immunology, 6th edition, W. H. Freeman and Co., page 91 (2007)). A single VH or VL region can be sufficient to confer antigen-binding specificity. Furthermore, the antibody binding to a particular antigen can be separated using the VH or VL region of the antibody that binds the particular antigen to respectively screen a library of complementary VL or VH regions. See, for example, Portolano et al., J. Immunol. 150:880-887 (1993); Clarkson et al., Nature 352:624-628 (1991).

The terms "complementarity determining region" and "CDR", synonymous with "hypervariable region" and "HVR", are known in the art to refer to non-contiguous sequences of amino acids within antibody variable regions that confer antigen specificity and/or binding affinity. Generally, there are three CDRs in each heavy chain variable region (HCDR1, HCDR2, HCDR3) and three CDRs in each light chain variable region (LCDR1, LCDR2, LCDR3).

In some embodiments of the present invention, the determination of CDR regions is according to the IMGT numbering scheme, the Kabat numbering scheme, the Martin numbering scheme, the AbM numbering scheme, the Chothia numbering scheme or the Contact numbering scheme.

In some embodiments of the present invention, the determination of CDR regions is according to the Kabat numbering scheme.

As used herein, "antibody" also includes a single-chain fragment variable ("scFv").

As used herein, "antibody" also includes an antibody not comprising a light chain, for example, heavy-chain antibodies (HCAbs) produced by *camelus dromedarius*, *camelus bactrianus*, *lama glama*, *lama guanicoe* and *vicugna pacos* and the like, and Ig new antigen receptors (IgNAR) found in cartilaginous fish such as sharks.

As used herein, the terms "VHH domain" and "single domain antibody" (sdAb) have the same meaning and are used interchangeably, and refer to cloning the variable region of a heavy-chain antibody and constructing a single-domain antibody consisting of only a heavy chain variable region, which is the smallest antigen-binding fragment with complete functions. Generally, a natural heavy-chain antibody lacking a light chain and a heavy chain constant region 1 (CH1) is first obtained, and then the variable region of the antibody heavy chain is cloned to construct a single-domain antibody consisting of only a heavy chain variable region.

As used herein, "antibody" also includes a monoclonal antibody or antigen-binding portion thereof. The monoclonal antibody or antigen-binding portion thereof can be non-human, chimeric, humanized or human, preferably humanized or human. Immunoglobulin structure and function are reviewed, for example, in Harlow et al., eds., Antibodies: A Laboratory Manual, Chapter 14 (Cold Spring Harbor Laboratory, Cold Spring Harbor, 1988).

As used herein, "antibody" can be derived from any animal, including, but not limited to, humans and non-human animals, and the non-human animals can be selected from primates, mammals, rodents and vertebrates, such as camelids, llamas, guanacos, monkeys (such as cynomolgus monkeys and rhesus monkeys), alpacas, sheep, rabbits, mice, rats or cartilaginous fish (such as sharks).

As used herein, "antigen-binding fragment": refers to a fragment that does not have the complete structure of an intact antibody and comprises only a part or a variant part of the intact antibody, the part or variant part having the ability to bind an antigen.

Exemplarily, as used herein, "antibody or antigen-binding fragment thereof" includes, but is not limited to: an immunoglobulin (full-length antibody), a half-antibody, Fab, Fab', F(ab')₂, an Fv fragment, a single-chain fragment variable (scFv), a disulfide-stabilized antibody (dsFv), a heavy chain variable region (VH) or a light chain variable region (VL) of an antibody, an Fd fragment consisting of a VH and a CH1 domain, a linear antibody, a heavy-chain antibody and a nanobody (VHH).

In some embodiments of the present invention, there is no particular limitation on the order in which the scFv comprises a VH region or a VL region from the N-terminus to the C-terminus, for example, comprising VH-Linker-VL or VL-Linker-VH from the N-terminus to the C-terminus; and the linker peptide can be selected from a flexible linker peptide.

A "ligand": in receptor-ligand binding, a ligand is typically a molecule that binds to a site on a receptor to produce a signal, the binding typically resulting in a conformational change of a complex structure, thereby inducing related physiological activity.

A "receptor-binding fragment": refers to a fragment that does not have the complete structure of an intact ligand and comprises only a part or a variant part of the intact ligand, the part or variant part having the ability to bind a receptor. Exemplarily, as used herein, a "ligand-binding fragment" includes, but is not limited to, the extracellular domain, functional fragment, epitope, binding region and variable region of the ligand.

"Endocytosis" refers to a process by which substances enter a cell, in which a region of the plasma membrane surrounds the substance to be taken in, and then the plasma membrane performs budding in the cell to form a vesicle containing the taken-in substance. Endocytosis can be divided into four categories: receptor-mediated endocytosis (also known as clathrin-mediated endocytosis), caveolae, pinocytosis and phagocytosis (Marsh M, Endocytosis. Oxford University Press. p. vii., 2001).

A "chimeric antigen receptor" (CAR) refers to an artificial cell surface receptor that is engineered to be expressed on immune effector cells such as lymphocytes and specifically binds to an antigen, comprising at least (1) an extracellular antigen-binding region, such as an scFv or VHH; (2) a transmembrane domain anchoring the CAR molecule into an immune effector cell; and (3) an intracellular signaling domain; and the extracellular structure of the CAR can further comprise a hinge region, and the intracellular structure can further comprise a costimulatory signaling domain. CAR molecules can use the extracellular antigen-binding region to redirect T cells and other immune effector cells to selected targets, such as cancer cells, in a non-MHC-restricted manner. In some embodiments of the present invention, the various polypeptide structures comprised in the CAR are of human origin.

"Chimeric": the term "chimeric" refers to any nucleic acid molecule or protein that is non-endogenous and comprises a combination of sequences that are joined or linked together and are not naturally joined or linked together in nature. For example, a chimeric nucleic acid molecule can comprise nucleic acids encoding various domains from a plurality of different genes. In another example, a chimeric nucleic acid molecule can comprise regulatory sequences and coding sequences from different sources, or regulatory sequences and coding sequences from the same source but arranged in a manner different from that in nature.

"Antigen": the terms "antigen" and "Ag" refer to a molecule capable of inducing an immune response. The induced immune response can include antibody production and/or activation of specific immunocompetent cells. Macromolecules including proteins, glycoproteins and glycolipids can serve as antigens. Antigens can be derived from recombinant or genomic DNA. As contemplated herein, an antigen does not need to (i) be encoded solely by the full-length nucleotide sequence of a gene or (ii) be encoded entirely by a gene. An antigen can be generated or synthesized, or an antigen can be derived from a biological sample. Such biological samples can include, but are not limited to, tissue samples, tumor samples, cells or biological fluids.

"Weakened": when referring to the ability of the glycoprotein variant to specifically bind to the receptor being "weakened", the term "weakened" includes completely eliminating the ability of the glycoprotein variant to specifically bind to the receptor, as well as significantly weakening the binding ability. In specific embodiments, "significantly weakened" refers to: relative to a wild-type viral glycoprotein, "weakened" is selected from being weakened by at least 95%, at least 90%, at least 85%, at least 80%, at least 75%, at least 70%, at least 65%, at least 60%, at least 55%, at least 50%, at least 45%, at least 40%, at least 35%, at least 30%, at least 25%, at least 20%, at least 15%, at least 10%, at least 5%, at least 4%, at least 3%, at least 2% and at least 1%.

"Nucleic acid": refers to any compound and/or substance including a polymer comprising nucleotides, such as a polynucleotide. As used herein, "nucleic acid", "polynucleotide" and "gene" are used synonymously. Each nucleotide consists of a base, in particular a purine or pyrimidine base (i.e., cytosine (C), guanine (G), adenine (A), thymine (T) or uracil (U)), a sugar (i.e., deoxyribose or ribose) and a phosphate group. Generally, a nucleic acid molecule is described by the sequence of bases, whereby the bases represent the primary structure (linear structure) of the nucleic acid molecule. The sequence of bases is typically expressed as 5' to 3'. As used herein, the term "nucleic acid" encompasses deoxyribonucleic acid (DNA), including, for example, complementary DNA (cDNA) and genomic DNA, ribonucleic acid (RNA), in particular messenger RNA (mRNA), synthetic forms of DNA or RNA, and mixed polymers comprising two or more of these molecules. "Nucleic acid" can be linear or circular. Furthermore, "nucleic acid" includes both sense (coding) and antisense (template) strands, and is in single-stranded or double-stranded form. Moreover, the "nucleic acid" described herein can contain naturally occurring or non-naturally occurring nucleotides. Examples of non-naturally occurring nucleotides include nucleotide bases modified with derived sugars, phosphate backbone linkages or chemically modified residues.

A "nucleic acid vector" refers to a vector carrying, accommodating or expressing any nucleic acid. The nucleic acid vector can have a specific function such as expression, packaging, pseudotyping or transduction. If the nucleic acid vector is suitable for use as a cloning vector or a shuttle vector, it can also have a manipulation function. The structure of the vector can include any desired form making it feasible and suitable for a particular use. Such forms include, for example, circular forms such as plasmids and phagemids, linear or branched forms. Nucleic acid vectors can be composed of, for example, DNA or RNA, and contain nucleotide derivatives, analogues and mimetics in whole or in part. Such nucleic acid vectors can be obtained from natural sources, recombinantly produced or chemically synthesized.

A "transgene": also known as a payload gene, as used herein, the term "transgene" refers to a gene or polynucleotide encoding a protein of interest (e.g., a CAR or engineered TCR, etc.), the expression of which in a host cell/target cell is desired and which has been transferred into the cell by genetic engineering techniques. A transgene can encode a protein having therapeutic significance as well as a protein serving as a reporter, tag, marker, suicide protein and the like. A transgene can be from a natural source, a modification of a natural gene, or a recombinant or synthetic molecule. In certain embodiments, the transgene is a component of a vector, such as a particle (including NIL).

An "expression cassette": as used herein, the term "expression cassette" refers to a unique component of a vector nucleic acid comprising at least one transgene and a regulatory sequence (e.g., a promoter, a 3'UTR) controlling the expression thereof in a host cell. A tandem expression cassette refers to a component of a vector nucleic acid comprising at least two transgenes under the control of a set of identical regulatory sequences for tandem expression of the at least two transgenes. In certain embodiments, the tandem expression cassette comprises at least two transgenes under the control of the same promoter. In certain embodiments, the first transgene and the second transgene are separated by an internal ribosome entry site (IRES), a furin cleavage site or a self-cleaving viral 2A peptide to allow co-expression of two proteins from a single mRNA.

As used herein, the terms "peptide", "polypeptide" and "protein" are used interchangeably and refer to a compound composed of amino acid residues covalently linked by peptide bonds.

"Encoding" refers to the inherent property of a specific polynucleotide sequence (such as a DNA, cDNA and mRNA sequence) to serve as a template for the synthesis of other polymers and macromolecules in a biological process, the template having a defined nucleotide sequence (i.e., rRNA, tRNA and mRNA) or a defined amino acid sequence and the resulting biological properties. Therefore, if the transcription and translation of an mRNA corresponding to a polynucleotide in a cell or other biological system produces a protein, the polynucleotide encodes the protein. Both the coding strand and the non-coding strand can be referred to as encoding the protein or other product of the polynucleotide. Unless otherwise specified, "a nucleotide sequence encoding an amino acid sequence" includes all nucleotide sequences that are degenerate to each other and encode the same amino acid sequence.

An "autoproteolytic peptide" or "self-cleaving peptide" or "2A peptide": refers to a self-cleaving peptide configured to generate two or more proteins from a single open reading frame, including an FT2A peptide, an F2A peptide, an E2A peptide, a T2A peptide and a P2A peptide and the like. A 2A peptide is an 18 to 22 residue-long viral oligopeptide that mediates "cleavage" of a polypeptide during translation in eukaryotic cells. A "2A peptide" can refer to peptides having different amino acid sequences. In the present disclosure, it should be understood that, in the case where the particle (including NIL) comprises two or more 2A peptides, the 2A peptides can be identical to or different from each other. Detailed methods for designing and using 2A peptides are provided by Szymczak-Workman et al. (2012) Cold Spring Harb. Protoc. 2012: 199-204.

"Exogenous": refers to any molecule derived from outside a living organism, including nucleic acids, proteins, polypeptides or small molecule compounds and the like. In contrast, the term "endogenous" refers to any molecule derived from inside a living organism (i.e., naturally produced by the living organism).

A "promoter": as used herein, the term "promoter" is defined as a DNA sequence recognized by cellular synthetic machinery or introduced synthetic machinery as being required for the initiation of transcription of a specific polynucleotide sequence. As used herein, the term "promoter/regulatory sequence" means a nucleic acid sequence required for the expression of a gene product operably linked to the promoter/regulatory sequence. In some cases, the sequence can be a core promoter sequence, and in other cases, the sequence can comprise an enhancer sequence and other regulatory elements required for the expression of the gene product. The promoter/regulatory sequence can be, for example, a sequence that expresses the gene product in a tissue-specific manner.

A "constitutive" promoter is a nucleotide sequence that, when operably linked to a polynucleotide encoding or specifying a gene product, causes the gene product to be produced in the cell in most or all physiological conditions of the cell.

An "inducible" promoter is a nucleotide sequence that, when operably linked to a polynucleotide encoding or specifying a gene product, substantially causes the gene product to be produced in the cell only when a corresponding inducer of the promoter is present in the cell.

A "tissue-specific" promoter is a nucleotide sequence that, when operably linked to a polynucleotide encoding or specified by a gene, substantially causes the gene product to be produced in the cell only when the cell is a cell of a tissue type corresponding to the promoter.

A "viral envelope": refers to the outermost layer of a variety of viruses (HURLBERT, RONALD E., Fundamentals of Microbiology, 102. Chapter #11: Viruses. Archived from the original on 2008-11-10.).

When a virus shuttles in a host cell, the viral envelope protects the genetic material during the life cycle thereof. Not all viruses have a viral envelope. A variety of human pathogenic viruses are wrapped in a lipid bilayer, and they infect target cells by fusing the viral envelope with the cell membrane.

"Retrovirus" and "retroviral vector": retrovirus is a virus that can integrate a DNA copy of its RNA genome into the DNA of a host cell it infects, thereby altering the genome of the host cell. An overview of available packaging systems is provided in *Cold Spring Harbour Laboratory Press*, 1997, page 447, written by JM Coffin, SM Hughes *et al.*, which is incorporated herein by reference in its entirety.

A "lentivirus": a lentivirus is a complex retrovirus that, in addition to the common retroviral genes Gag, Pol and env, also contains other genes having regulatory or structural functions. The higher complexity enables the virus to regulate its life cycle, as in latent infection. Lentiviruses belong to a genus of retroviruses that can infect dividing cells and non-dividing cells. Examples of lentiviruses include, but are not limited to, HIV (human immunodeficiency virus, including HIV type I and HIV type II), equine infectious anemia virus, feline immunodeficiency virus (FIV), bovine immunodeficiency virus (BIV) and simian immunodeficiency virus (SIV).

A "lentiviral vector" is a vector derived from a lentivirus and comprises one or more lentiviral packaging proteins and/or lentiviral proteins required for the expression of one or more genes carried by the vector. It is produced by multiple attenuations of virulence genes of lentiviruses such as HIV through gene editing, genetic engineering and other technical means, for example, by deleting genes env, vif, vpr, vpu and nef, so that the lentiviral vector has biological safety.

As used herein, the term "lentiviral vector" is intended to mean a lentiviral particle comprising a viral envelope, having at least one characteristic of a lentivirus, being capable of invading a target cell, and being incapable of self-replication.

A lentiviral vector or a retroviral vector is generally packaged in a packaging cell by a lentiviral vector packaging system or a retroviral vector packaging system. For example, the process and method of packaging a lentiviral vector are described in Merten OW, et al., Production of lentiviral vectors. Mol Ther Methods Clin Dev. (2016), 3, 16017, which is incorporated herein by reference in its entirety.

A commonly used pseudotyped lentiviral vector includes a so-called third-generation lentiviral vector packaging system. The third-generation lentiviral vector packaging system includes four plasmids, and generally includes a transfer plasmid and three packaging plasmids: a transfer plasmid encoding a gene of interest ("GOI"), such as a transgene, a GagPol plasmid, a Rev plasmid and an envelope plasmid (comprising a viral glycoprotein gene such as VSV-G or a variant thereof or Cocal-G or a variant thereof).

A "transfer plasmid (transfer vector)" comprises a lentiviral vector backbone genome and a transgene. The transfer plasmid typically has one or more transgenes flanked by long terminal repeat (LTR) sequences, which facilitate the integration of the transgene comprised in the transfer plasmid into the host genome. The LTRs are responsible for the reverse transcription and integration processes of the viral genome. Through these sequences, the lentivirus can integrate the transgene into the genome of a host cell. For safety reasons, the transfer plasmid is typically designed such that the resulting viral vector cannot self-replicate. For example, the transfer plasmid lacks the genetic elements required to produce infectious lentiviral particles in a host cell. In addition, the transfer plasmid can be designed to be deficient in the 3' LTR, thereby making the virus "self-inactivating". Compared with a traditional second-generation pseudotyped lentiviral vector packaging system (typically comprising a single packaging plasmid of nucleic acid encoding Gag, Pol, Rev and Tat and a separate envelope plasmid), the TAT gene is eliminated from the third-generation pseudotyped lentiviral vector packaging system by adding a chimeric 5' LTR fused to a heterologous promoter (e.g., a CMV or RSV promoter) on the transfer plasmid. The transfer plasmid typically comprises a ψ sequence (Psi sequence, also known as a ψ packaging signal) located downstream of the 5' LTR, with the ψ sequence being responsible for packaging the transgene RNA into a viral particle. The ψ sequence ensures that only the RNA comprising the transgene is packaged into the viral particle. The transfer plasmid can optionally comprise an internal ribosome entry site ("IRES"), to allow simultaneous translation of two or more open reading frames (ORFs) on a single mRNA, thereby achieving multi-gene expression. Some transfer plasmids, such as the lentiviral master plasmid/transfer plasmid used in some embodiments of the present invention, can further comprise a selectable marker gene, such as an antibiotic resistance gene (e.g., PuroR, encoding puromycin resistance) or a fluorescent protein gene (e.g., GFP), for screening or tracking transduced cells.

For the transfer plasmid in a lentiviral vector packaging system, reference can be made to Du Ll, et al., J. Virol. 72:8463-71 (1998); Miyoshi, et al., J. Virol. 72:8150-57 (1998).

The third-generation lentiviral vector system typically also includes three packaging plasmids: a GagPol plasmid, a Rev plasmid and an envelope plasmid. The envelope plasmid typically carries a viral glycoprotein gene, and wild-type VSV-G or Cocal-G is a commonly used viral glycoprotein; and the viral glycoprotein gene is operably linked to a promoter, the promoter typically being a CMV promoter, to initiate transcription of the viral glycoprotein gene. The third-generation lentiviral vector system further includes two packaging plasmids, one comprising a gene encoding a Gag protein and a Pol protein (GagPol packaging plasmid), and the other comprising a gene encoding a Rev protein (Rev plasmid) as a further safety feature, which is an improvement over the so-called second-generation packaging system with a single packaging plasmid. The Gag gene encodes a Gag polyprotein precursor comprising lentiviral structural proteins, including matrix, capsid and nucleocapsid; the Pol gene encodes a Pol polyprotein precursor providing lentiviral enzyme functions required for replication, including protease, reverse transcriptase and integrase; the Rev gene encodes a Rev protein that binds to a Rev response element (RRE) to allow nuclear export of unspliced and singly spliced HIV RNA during viral replication. The Gag and Pol polyprotein precursors are cleaved during viral particle preparation. The Rev protein binds to a Rev response element (RRE) sequence on viral RNA, and promotes the transport of incompletely spliced viral RNA from the nucleus to the cytoplasm by interacting with the nuclear export machinery of the host cell. These unspliced RNAs can be translated into viral structural proteins and enzymes in the cytoplasm, or assembled into new viral particles.

Exemplarily, the packaging plasmids include, but are not limited to, pMD2.G, pRSV-rev, pMDLG-pRRE and pRRL-GOI.

Lentiviral vectors and lentiviral vector backbone genomes are known in the art, see Naldini, et al., (1996) Science 272: 263-7; Zufferey, et al., (1998) J. Virol. 72: 9873-9880; Du Ll, et al., (1998) J. Virol. 72: 8463-8471, U.S. Patent No. 6,013,516 and U.S. Patent No. 5,994,136, each of which is incorporated herein by reference in its entirety.

Compared with a pseudotyped lentiviral vector packaging system, a pseudotyped retroviral vector packaging system typically does not comprise a Rev plasmid, because the genomic RNA of a retrovirus derived from Moloney murine leukemia virus ("MMLV") and the like can naturally be transported from the nucleus to the cytoplasm for translation and assembly, and therefore does not depend on a specific nuclear export mechanism such as the Rev protein. A pseudotyped retroviral vector packaging system typically comprises a transfer plasmid and two packaging plasmids: an envelope plasmid and a GagPol packaging plasmid. The transgene sequence comprised in the transfer plasmid is flanked by long terminal repeat (LTR) sequences, which facilitate the integration of the transfer plasmid sequence into the host genome. Typically, during viral transduction, the sequence between the LTRs, including the LTRs, will be integrated into the host genome. A backbone genome of MMLV or murine stem cell virus ("MSCV") comprising the respective LTRs thereof is typically used to construct a transfer plasmid in a pseudotyped retroviral vector packaging system. The GagPol packaging plasmid comprises a Gag gene and a Pol gene; and the envelope plasmid typically comprises a polynucleotide encoding a viral glycoprotein, such as VSV-G or Cocal-G. In some embodiments of the present invention, the envelope plasmid can further comprise a nucleic acid encoding the T cell activation primary signaling molecule and/or the T cell activation secondary signaling molecule.

In some embodiments, a producer cell is transfected with the transfer plasmid, GagPol plasmid, envelope plasmid and Rev plasmid at a defined ratio. In some embodiments, the ratio of each plasmid is determined by mass, which is not particularly limited as long as biologically active non-integrating lentiviral vectors can be packaged. In some embodiments, the mass of each of the transfer plasmid and the GagPol plasmid is higher than the mass of each of the envelope plasmid and the Rev plasmid. In some embodiments, the defined ratio of the transfer plasmid, GagPol plasmid, envelope plasmid and Rev plasmid is about 1:1:1:1 to about 9:4:2:2. In some embodiments of the present invention, the envelope plasmid can comprise a nucleic acid encoding the T cell activation primary signaling molecule and/or the T cell activation secondary signaling molecule.

In some embodiments, the envelope plasmid comprises a tandem expression cassette encoding VSV-G or a variant thereof or Cocal-G or a variant thereof and the T cell activation primary signaling molecule and/or the T cell activation secondary signaling molecule as disclosed herein. In specific embodiments, the tandem expression cassette comprised in the envelope plasmid comprises a polynucleotide encoding a first signal peptide, a polynucleotide encoding the T cell activation primary signaling molecule and/or the T cell activation secondary signaling molecule, a polynucleotide encoding one of an internal ribosome entry site (IRES), a furin cleavage site or a viral 2A peptide, a polynucleotide encoding a second signal peptide, and a polynucleotide encoding VSV-G or a variant thereof or Cocal-G or a variant thereof. In certain embodiments, the polynucleotide encoding VSV-G or a variant thereof or Cocal-G or a variant thereof is located at the 5' end of the polynucleotide encoding the T cell activation primary signaling molecule and/or the T cell activation secondary signaling molecule. In other embodiments, the polynucleotide encoding VSV-G or a variant thereof or Cocal-G or a variant thereof is located at the 3' end of the polynucleotide encoding the T cell activation primary signaling molecule and/or the T cell activation secondary signaling molecule. The polynucleotide encoding the T cell activation primary signaling molecule and/or the T cell activation secondary signaling molecule and the polynucleotide encoding VSV-G or a variant thereof or Cocal-G or a variant thereof are separated in the tandem cassette by a polynucleotide encoding an IRES, a furin cleavage site or a viral 2A peptide, which allows co-expression of two proteins from a single mRNA. In certain embodiments, the viral 2A peptide is porcine teschovirus-1 (P2A), *thosea asigna* virus (T2A), equine rhinitis A virus (E2A), foot-and-mouth disease virus (F2A) or a variant thereof.

The use of a lentiviral/retroviral vector or particle packaging system relies on a "packaging cell line". Generally, a packaging cell line is a cell line, in which the cell, when a transfer plasmid and one or more packaging plasmids are introduced into the cell, can produce lentiviral vectors or retroviral vectors that are incapable of self-replication and can infect/transduce target cells. An overview of available packaging systems is provided in Cold Spring Harbour Laboratory Press, 1997, page 447, written by JM Coffin, SM Hughes *et al.*, which is incorporated herein by reference in its entirety.

Exemplarily, transfection methods including chemical-mediated transfection methods, physical-mediated transfection methods or biological-mediated transfection methods can be used to introduce various plasmids into a packaging cell line. For example, chemical-mediated transfection methods include transfection using chemical reagents such as calcium phosphate, DEAE-dextran or PEI (Polyethylenimine), and physical-mediated transfection methods include transfection methods such as electroporation.

Producer/host/packaging cells that can be used to prepare the particles disclosed in the present invention include human embryonic kidney (HEK) 293 cells and derivatives thereof. The producer cells can be adherent cell lines such as HEK293T producer cells, or suspension cell lines such as HEK293T/17SF producer cells.

Exemplarily, the packaging cell/host cell is selected from CHO cells, BHK cells, MDCK cells, C3H-10T1/2 cells, FLY cells, Psi-2 cells, BOSC 23 cells, PA317 cells, WEHI cells, COS cells, BSC-1 cells, BSC-40 cells, BMT-10 cells, VERO cells, W138 cells, MRC5 cells, A549 cells, HT1080 cells, HEK-293 cells, B-50 cells, 3T3 cells, NIH3T3 cells, HepG2 cells, Saos-2 cells, Huh7 cells, HeLa cells, W163 cells and 211 cells;
preferably, the packaging cell/host cell is an HEK-293T cell.

"Retrovirus" and "retroviral vector": "retrovirus" refers to an RNA virus having a single-stranded positive-sense RNA molecule. A retrovirus comprises reverse transcriptase and integrase. After entering a target cell, the retrovirus uses the reverse transcriptase to transcribe the RNA molecule into a DNA molecule. Subsequently, the integrase is used to integrate the DNA molecule into the host cell genome. After integration into the host cell genome, the sequence from the retrovirus is referred to as a provirus (e.g., a proviral sequence or sequence of provirus). A retroviral vector typically refers to a pseudotyped retroviral vector derived from a retrovirus, exemplarily derived from a γ-retrovirus. Unlike lentiviral vectors that can transduce dividing and non-dividing cells, retroviral vectors can only transduce dividing cells, and the exogenous transgene they can carry is generally relatively small. For a comparison and discussion of lentiviral vectors and retroviral vectors, see: Stripecke, R., Kasahara, N. (2007). Lentiviral and Retroviral Vector Systems. In: Hunt, K. K., Vorburger, S. A., Swisher, S. G. (eds) Gene Therapy for Cancer. Cancer Drug Discovery and Development. Humana Press.

Lentiviral vectors and retroviral vectors can stably integrate shuttle genes and other exogenous payload genes into the chromosomes of target cells, thereby allowing the target cells to express the delivered shuttle gene for a long time, providing a great advantage for gene therapy. In addition, they do not transfer viral genes, thereby avoiding the problem of producing transduced cells that can be destroyed by cytotoxic T cells. Moreover, they have a stronger cloning capacity, sufficient to meet most expected clinical applications.

An "envelope glycoprotein": refers to a glycoprotein coated on the outer layer of a virus, which plays an important role in the adsorption and penetration of the virus into a host cell, pathogenicity, down-regulation of the expression of host surface proteins, and increasing viral packaging and budding.

A "variant": a variant refers to a mutant having at least about 50% identity to a non-mutant (wild-type) amino acid sequence, in which "at least about 50% identity" refers to about 50%, about 60%, about 70%, about 80%, about 90%, about 91%, about 92%, about 93%, about 94%, about 95%, about 96%, about 97%, about 98% or about 99% identity to a non-mutant (wild-type) amino acid sequence; or a variant refers to a mutant in which the nucleic acid sequence encoding the variant has at least 50% identity to the nucleic acid sequence encoding a non-mutant (wild-type), in which "at least 50% identity" means that the nucleic acid sequence encoding the variant has at least 50%, at least 60%, at least 70%, at least 80%, at least 90%, at least 91%, at least 92%, at least 93%, at least 94%, at least 95%, at least 96%, at least 97%, at least 98% or at least 99% identity to the nucleic acid sequence encoding a non-mutant (wild-type). In some embodiments of the present invention, the variant comprises a mutant comprising a conservative substitution relative to a non-mutant. "Conservative substitution" is known in the art as the substitution of one amino acid with another amino acid having similar properties. Exemplarily, conservative substitution is well known in the art (see, for example, WO 97/09433, page 10, published on March 13, 1997; Lehninger, Biochemistry, Second Edition; Worth Publishers, Inc. NY: NY (1975), pages 71-77; Lewin, Genes IV, Oxford University Press, NY *and* Cell Press, Cambridge, MA (1990), page 8).

A "pharmaceutically acceptable excipient or carrier": a pharmaceutically acceptable excipient or carrier includes, but is not limited to, diluents, solubilizers, emulsifiers, preservation solutions, preservatives, and/or adjuvants. The excipient is preferably non-toxic or substantially non-toxic to the recipient at the dose and concentration employed. Such excipients include, but are not limited to: saline, buffer, glucose, water, glycerol, ethanol, and combinations thereof. In certain embodiments, the pharmaceutical composition can contain a substance for improving, maintaining or retaining, for example, the pH, permeability, viscosity, clarity, color, isotonicity, odor, sterility, stability, dissolution or release rate, absorption or permeation of the composition. The optimal pharmaceutical composition can be determined depending on the intended route of administration, delivery method and desired dose.

A "subject": as used herein, "subject", "patient" and "individual" are used synonymously, and include, but are not limited to, mammals, such as humans or non-human mammals, for example, livestock, agricultural animals or wild animals, and birds and aquatic animals. As used herein, "subject" includes a subject suffering from a disease, disorder or condition, or at risk of developing a disease, disorder or condition, or otherwise in need of any of the viral particles, CAR-T cells, compositions or treatment methods provided herein.

A "disease" is a health condition of a subject in which the subject cannot maintain homeostasis and in which the health of the subject continues to deteriorate if the disease is not improved. In contrast, a "disorder" or "adverse condition" of a subject is a health condition in which the subject can maintain homeostasis, but in which the health condition of the subject is less favorable than in the absence of the disorder or adverse condition. Without treatment, a disorder or adverse condition does not necessarily lead to a further deterioration of the health condition of the subject.

"Cancer": as used herein, the term "cancer" is defined as a disease characterized by the rapid and uncontrolled growth of abnormal cells. Abnormal cells can form a solid tumor or constitute a hematological malignancy. Cancer cells can spread locally or to other parts of the body through the bloodstream and lymphatic system. Various examples of cancer include, but are not limited to, blood cancers, such as B lymphocyte malignancies; and solid cancers, such as breast cancer, prostate cancer, ovarian cancer, cervical cancer, skin cancer, pancreatic cancer, colorectal cancer, kidney cancer, liver cancer, brain cancer, lung cancer and the like.

"Treatment": refers to the application of a treatment method described herein to a subject to achieve at least one positive therapeutic effect (for example, a reduction in the number of cancer cells, a reduction in tumor volume, a reduction in the rate of infiltration of cancer cells into peripheral organs, or a reduction in the rate of tumor metastasis or tumor growth). An effective method for treating a patient can vary depending on various factors (such as the disease state of the patient, age, weight, and the ability of the therapy to elicit an anti-cancer response in the subject).

As used herein, "treatment" includes any beneficial or desired effect related to treatment. "Treatment" does not necessarily indicate the complete eradication or cure of a disease or disorder, or the symptoms associated therewith.

The therapeutically effective amount will depend on, for example, the extent and target of treatment. Those skilled in the art will appreciate that the appropriate dosage level for treating will depend in part on the delivered drug, molecule, cell, indication, route of administration, and patient situation (weight, body surface or organ size) and/or condition (age and general health condition). In certain embodiments, a clinician can titrate the dose and alter the route of administration to achieve the optimal therapeutic effect.

The frequency of administration will depend on the pharmacokinetic parameters of the engineered T cells or the viral particles in the formulation used. A clinician typically administers a pharmaceutical composition until a dose that achieves the desired effect is reached. The pharmaceutical composition can thus be administered as a single dose, or over time as two or more doses (which can contain or not contain the same amount of the desired molecule), or by continuous infusion through an implant device or catheter.

"Administration": the route of administration of a pharmaceutical composition is conventional in the art, such as injecting via oral, nasal, intravenous, intraperitoneal, intracerebral (intraparenchymal), intraventricular, intramuscular, intraocular, intra-arterial, portal vein or intralesional route, and administration can also be performed by a sustained release system or through an implant device.

"And/or": should be understood to mean one or both alternatives.

"About"/"approximately": as used herein, the term "about" refers to the common error range for the corresponding value readily known to those skilled in the art, exemplarily including, but not limited to, referring to a quantity, level, value, number, frequency, percentage, size, magnitude, amount, weight or length that varies by up to 15%, 10%, 9%, 8%, 7%, 6%, 5%, 4%, 3%, 2% or 1% compared to a reference quantity, level, value, number, frequency, percentage, size, magnitude, amount, weight or length. A description of "about" a certain value or parameter includes (and describes) embodiments directed to the value or parameter itself. For example, a description of "about X" includes a description of "X".

"Comprise": as used herein, unless the context requires otherwise, the word "comprise" will be understood to mean inclusion of the stated step, or element, or group of steps or elements, but not exclusion of any other step, or element, or group of steps or elements. In some embodiments of the present invention, the terms "including", "having", "containing" and "comprising" are used synonymously.

"Embodiment": references throughout this specification to "some embodiments", "some examples", "embodiment", "specific embodiment", "related embodiment", "certain embodiment", "another embodiment" or "other embodiment" or combinations thereof refer to a particular feature, structure or characteristic described in connection with the embodiment being included in at least one embodiment of the present invention. Therefore, the appearances of the foregoing phrases in various places throughout this specification are not necessarily all referring to the same embodiment. Furthermore, the particular features, structures or characteristics can be combined in any suitable manner in one or more embodiments.

"Prevention": as used herein, "prevention" and similar words, such as "preventing" and the like, indicate a method for preventing, inhibiting or reducing the likelihood of the occurrence or recurrence of a disorder. As used herein, "prevention" and similar words also include reducing the intensity, effect, symptoms and/or burden of a disease or disorder before the onset or recurrence thereof.

"Stable integration": also known as "stable transfection", refers to the integration of an exogenous polynucleotide into the genome of a host cell after the exogenous polynucleotide is introduced into the host cell, and stable gene expression in the host cell for a long time, which is in contrast to transient transfection and transient expression.

"Specific binding": as used herein, the term "specific binding" refers to binding that occurs between paired molecule species (for example, a receptor and a ligand). When the interaction of two species produces a non-covalently bound complex, the binding is typically the result of electrostatic, hydrogen bonding or lipophilic interactions. In various embodiments, specific binding between one or more species is direct. In some embodiments of the present invention, the affinity of specific binding is about 2 times, about 5 times, about 10 times, about 20 times, about 50 times, about 100 times, or about 1000 times or more times higher than background binding (non-specific binding).

"Sequence identity": generally, "sequence identity" or "sequence homology" respectively refers to the precise correspondence between nucleotides or amino acids of two polynucleotide or polypeptide sequences. Typically, techniques for determining sequence identity include determining the nucleotide sequence of a polynucleotide and/or determining the amino acid sequence encoded thereby, and comparing these sequences with a second nucleotide or amino acid sequence. Two or more sequences (polynucleotide or amino acid) can be compared by determining the "identity percent" thereof. Whether a nucleic acid or amino acid sequence, the identity percent of two sequences is the number of exact matches between the two aligned sequences divided by the length of the shorter sequence, and then multiplied by 100. For example, sequence information can also be compared using the advanced BLAST computer program available from the National Institutes of Health to determine the identity percent. The BLAST program is based on the alignment method of Karlin and Altschul, Proc. Natl. Acad. Sci. USA 87: 2264-2268 (1990) and is discussed in Altschul et al., J. Mol. Biol. 215: 403-410 (1990); Karlin and Altschul, Proc. Natl. Acad. Sci. USA 90: 5873-5877 (1993); and Altschul et al., Nucleic Acids Res. 25: 3389-3402 (1997). Briefly, the BLAST program defines identity as the number of aligned identical symbols (typically nucleotides or amino acids) divided by the total number of symbols in the shorter of the two sequences. The program can be used to determine the identity percent over the entire length of the compared protein.

A "signal peptide": sometimes also referred to as a signal sequence, targeting signal, localization signal, localization sequence, transit peptide or leader peptide, is a short peptide (typically comprising 16-30 amino acids) (*Kapp, Katja; Schrempf, Sabrina; Lemberg, Marius K.; Dobberstein, Bernhard* (2013-01-01).), and present at the N-terminus of most newly synthesized proteins destined for the secretory pathway (occasionally non-classically present at the C-terminus or internally) (Owji, et al., A comprehensive review of signal peptides: Structure, roles, and applications, European Journal of Cell Biology. 97 (6): 422-441. (2018)) (Blobel G, Dobberstein B, et al., Transfer of proteins across membranes. I. Presence of proteolytically processed and unprocessed nascent immunoglobulin light chains on membrane-bound ribosomes of murine myeloma, The Journal of Cell Biology, 67 (3): 835-51. (1975)). A signal peptide is a short peptide present at the N-terminus of a newly synthesized protein, and is dedicated to the plasma membrane or the secretory pathway. Signal sequences typically comprise a short, hydrophilic and positively charged amino acid sequence at the N-terminus, a central hydrophobic domain having 5-15 residues, and a C-terminal region having a signal sequence cleavage site. In eukaryotes, signal sequences facilitate the translocation of newly synthesized proteins to the endoplasmic reticulum, where the protein is cleaved by a signal peptidase to produce a mature protein, which then enters its appropriate destination. The diversity of signal sequence length and amino acid composition makes it difficult to precisely predict cleavage sites. For the polypeptide sequences disclosed herein, when referring to signal sequences, polypeptide sequences without a signal sequence or having a partial signal sequence are also contemplated.

The function of a signal peptide is to facilitate the transfer of a protein by a cell, typically to the cell membrane. In prokaryotes, a signal peptide guides a newly synthesized protein to the SecYEG protein conduction channel present in the plasma membrane. A homologous system exists in eukaryotes, in which the signal peptide guides a newly synthesized protein to the Sec61 channel, which has structural and sequence homology to SecYEG but is present in the endoplasmic reticulum (Rapoport TA, Protein translocation across the eukaryotic endoplasmic reticulum and bacterial plasma membranes, Nature. 450 (7170): 663-9 (2007).). The SecYEG and Sec61 channels are commonly referred to as translocons, and transport through the channel is referred to as translocation. As a secreted protein passes through the channel, a transmembrane domain can diffuse through a lateral gate in the translocon to partition into the surrounding membrane.

"MOI": i.e., "multiplicity of infection", refers to the number of viral particles added to each cell during infection. For example, when one million viral particles are added to one million cells, MOI = 1.

"Operably": when a polynucleotide is in a functional relationship with another polynucleotide, the nucleic acid is "operably linked". For example, if the DNA of a presequence or secretory leader sequence is expressed as a preprotein involved in the secretion of a polypeptide, then this DNA is operably linked to the DNA of the polypeptide; if a promoter or enhancer affects the transcription of a coding sequence, the promoter or enhancer is operably linked to the sequence; or if a ribosome binding site is positioned to facilitate translation, the ribosome binding site is operably linked to the coding sequence. Generally, "operably linked" means that the linked polynucleotides are contiguous, and, in the case of a secretory leader sequence, are contiguous and in reading frame. However, enhancers need not be contiguous. Linking is accomplished by ligation at suitable restriction sites. If such sites do not exist, synthetic oligonucleotide adapters or linkers are used in accordance with conventional practice.

"Transduction": as used herein, the terms "transfection", "transformation" and "transduction" are used synonymously, and refer to the process of transferring or introducing an exogenous nucleic acid into a host cell or packaging cell. A "transfected", "transformed" or "transduced" cell is a cell that has been transfected, transformed or transduced with an exogenous nucleic acid. The cell includes a primary subject cell and progeny thereof.

Methods of introducing a vector such as a viral particle or an isolated nucleic acid into a mammalian cell are known in the art. The described vectors can be transferred into immune effector cells by physical, chemical or biological methods.

Physical methods for introducing a vector or isolated nucleic acid into an immune effector cell include calcium phosphate precipitation, lipofection, particle bombardment, microinjection, electroporation and the like. Methods for producing a cell comprising a vector and/or an exogenous nucleic acid are well known in the art (see Sambrook, J., Fritsch, E. F. and Maniatis, T. (2001) Molecular Cloning: A Laboratory Manual. Cold Spring Harbor Laboratory Press, Cold Spring Harb*or.*). In some embodiments of the present invention, a vector is introduced into a cell by electroporation. In some embodiments of the present invention, a vector is introduced into a cell by a PEI (Polyethylenimine) transfection reagent.

Biological methods for introducing a vector or isolated nucleic acid into an immune effector cell include the use of DNA and RNA vectors. Viral vectors have become the most widely used method of inserting genes into mammal cells (e.g., human cells).

Chemical methods for introducing a vector or isolated nucleic acid into an immune effector cell include colloidal dispersion systems, such as including macromolecular complexes, nanocapsules, microspheres, beads and lipid-based systems, such as including oil-in-water emulsions, micelles, mixed micelles and liposomes. An exemplary colloidal system for use as an *in vitro* delivery vehicle is a liposome.

All publications, documents, and patents mentioned in this text are hereby incorporated by reference in their entirety, as if each individual publication, document, or patent that has not been specifically and individually indicated to be incorporated by reference in its entirety were incorporated herein. In the case of conflict, the present invention (including any definitions herein) shall control. However, any references, articles, publications, patents, patent publications, and patent applications cited herein do not, and shall not be deemed to, constitute an admission or any form of suggestion that they constitute valid prior art or form part of the common general knowledge in any country of the world.

The section headings used herein are for organizational purposes only and are not to be construed as limiting the subject matter described.

### BRIEF DESCRIPTION OF THE DRAWINGS

FIG. 1: shows flow cytometry detection results of the transduction efficiency of the LVV-V5-GFP and the wild-type LVV-GFP respectively transducing LDL-R⁺CD3⁺Jurkat cells, LDL-R⁺CD3⁻Nalm-6 cells and LDL-R⁻CD3⁺human non-activated PBMCs in Example 1;
FIG. 2: shows flow cytometry detection results of the expression efficiency of CAR-19 in CD3⁺ T cells on Day 5 after the LVV-V5-CAR19 and the LVV-V7-CAR19 respectively transduce human non-activated PBMCs in Example 2;
FIG. 3: shows flow cytometry detection results of the expression of CD19 in each group of mixed cells on Day 4 after CD19-CAR-T cells prepared by the LVV-V5-CAR19 and the LVV-V7-CAR19 respectively transducing human non-activated PBMCs kill CD19⁺Nalm-6 cells in Example 3;
FIG. 4: shows flow cytometry detection results of the expression of CD19 in each group of mixed cells on Day 7 after CD19-CAR-T cells prepared by the LVV-V5-CAR19 and the LVV-V7-CAR19 respectively transducing human non-activated PBMCs kill CD19⁺Nalm-6 cells in Example 4;
FIG. 5: shows a bar comparison chart of the expression efficiency of CAR-19 in each group of cells on Day 2 after the LVV-V5-CAR19, LVV-V6-CAR19, LVV-V7-CAR19 and LVV-V8-CAR19 respectively transduce human non-activated PBMCs in Example 5;
FIG 6: shows a bar comparison chart of the killing efficiency of CD19-CAR-T cells prepared by the LVV-V5-CAR19, LVV-V6-CAR19, LVV-V7-CAR19 and LVV-V8-CAR19 respectively transducing human non-activated PBMCs killing CD19⁺Nalm-6 cells on Day 5 in Example 5;
FIG. 7: shows a bar comparison chart of the expression efficiency of CAR-19 in each group of cells on Day 2 after the LVV-V1-CAR19, LVV-V2-CAR19, LVV-V3-CAR19 and LVV-V4-CAR19 respectively transduce human non-activated PBMCs in Example 6;
FIG. 8: shows a bar comparison chart of the killing efficiency of CD19-CAR-T cells prepared by the LVV-V1-CAR19, LVV-V2-CAR19, LVV-V3-CAR19 and LVV-V4-CAR19 respectively transducing human non-activated PBMCs killing CD19⁺Nalm-6 cells on Day 5 in Example 6;
FIG. 9: shows a bar comparison chart of the expression efficiency of CAR-20 in each group of cells on Day 2 after the LVV-V9-CAR20, LVV-V10-CAR20 and LVV-V11-CAR20 respectively transduce human non-activated PBMCs in Example 7;
FIG. 10: shows flow cytometry detection results of the expression of CD20 in each group of mixed cells on Day 5 after CD20-CAR-T cells prepared by the LVV-V9-CAR20, LVV-V10-CAR20 and LVV-V11-CAR20 respectively transducing human non-activated PBMCs kill CD20⁺Dakiki cells in Example 7;
FIG. 11: shows a bar comparison chart of the expression efficiency of CAR-HER2 in each group of cells on Day 2 after the LVV-V13-CARHER2, LVV-V14-CARHER2 and LVV-V15-CARHER2 respectively transduce human non-activated PBMCs in Example 8;
FIG. 12: shows a bar comparison chart of the killing efficiency of HER2-CAR-T cells prepared by the LVV-V13-CARHER2, LVV-V14-CARHER2 and LVV-V15-CARHER2 respectively transducing human non-activated PBMCs killing HER-2⁺OVCAR-3 cells on Day 2 in Example 8;
FIG. 13: shows flow cytometry detection results of the expression efficiency of CAR-CEA in CD3⁺ T cells on Day 2 after the LVV-V16-CARCEA, LVV-V17-CARCEA and LVV-V18-CARCEA respectively transduce human non-activated PBMCs in Example 9;
FIG. 14: shows a bar comparison chart of the killing efficiency of CEA-CAR-T cells prepared by the LVV-V16-CARCEA, LVV-V17-CARCEA and LVV-V18-CARCEA respectively transducing human non-activated PBMCs killing CEA⁺T-84 cells on Day 2 in Example 9;
FIG. 15: shows flow cytometry detection results of the expression efficiency of CAR-33 in CD3⁺ T cells on Day 2 after the LVV-V19-CAR33, LVV-V20-CAR33 and LVV-V21-CAR33 respectively transduce human non-activated PBMCs in Example 10;
FIG. 16: shows a bar comparison chart of the killing efficiency of CD33-CAR-T cells prepared by the LVV-V19-CAR33, LVV-V20-CAR33 and LVV-V21-CAR33 respectively transducing human non-activated PBMCs killing CD33⁺MOLM-13 cells on Day 5 in Example 10;
FIG. 17: shows flow cytometry detection results of the expression efficiency of CAR-BCMA in CD3⁺ T cells on Day 2 after the LVV-V22-CARBCMA, LVV-V23-CARBCMA and LVV-V24-CARBCMA respectively transduce human non-activated PBMCs in Example 11; and
FIG. 18: shows a bar comparison chart of the killing efficiency of BCMA-CAR-T cells prepared by the LVV-V22-CARBCMA, LVV-V23-CARBCMA and LVV-V24-CARBCMA respectively transducing human non-activated PBMCs killing BCMA⁺U266 cells on Day 5 in Example 11.

### DETAILED DESCRIPTION OF THE EMBODIMENTS

The concept of the present invention and the technical effects produced thereby are clearly and completely described below in conjunction with the examples, so as to fully understand the objectives, features, and effects of the present invention. Obviously, the described examples are only some, rather than all, of the examples of the present invention. Based on the examples of the present invention, other examples obtained by those skilled in the art without any inventive effort shall all fall within the protection scope of the present invention.

In the following examples, experimental methods in which specific conditions are not indicated are carried out according to conventional methods and conditions well known in the art, or according to the product instruction manual. Reagents and raw materials of which specific components are not indicated in the present invention are all commercially available.

### Example 1

Construction of a Targeted Lentiviral Vector V5-GFP (LVV-V5-GFP) Capable of Targetedly Activating and Transducing Non-Activated T Cells.

### 1. Design of a Polynucleotide Encoding a Membrane-Expressed Anti-CD3 Antibody × Anti-CD28 Antibody Structure

In this example, the polynucleotide encoding the membrane-expressed anti-CD3 antibody × anti-CD28 antibody (membrane-expressed CD3×CD28 bispecific antibody) sequentially comprised, from the 5' end to the 3' end: a polynucleotide encoding a human CD8α signal peptide, a polynucleotide encoding an anti-CD3 antibody (UCHT1-scFv), a polynucleotide encoding a human CD8α hinge region, a polynucleotide encoding a human CD8α transmembrane domain, a polynucleotide encoding an FT2A peptide, a polynucleotide encoding a human CD8α signal peptide, a polynucleotide encoding an anti-CD28 antibody (15E8-scFv), a polynucleotide encoding a human CD8α hinge region, and a polynucleotide encoding a human CD8α transmembrane domain;
(1) the amino acid sequence of the human CD8α signal peptide is as set forth in SEQ ID NO: 8;
(2) the amino acid sequence of the UCHT1-scFv is as set forth in SEQ ID NO: 9;
(3) the amino acid sequence of the human CD8α hinge region is as set forth in SEQ ID NO: 10;
(4) the amino acid sequence of the human CD8α transmembrane domain is as set forth in SEQ ID NO: 11;
(5) the amino acid sequence of the FT2A peptide is as set forth in SEQ ID NO: 12; and
(6) the amino acid sequence of the 15E8-scFv is as set forth in SEQ ID NO: 13.

### 2. Packaging of LVV-V5-GFP Capable of Targetedly Activating Non-Activated T Cells

A. The following 4 plasmids were prepared: an envelope plasmid (envelope plasmid 1) carrying a polynucleotide encoding a mutant VSV-G1 and a polynucleotide encoding the membrane-expressed CD3×CD28 bispecific antibody, a pMDLg/pRRE packaging plasmid, a pRSV-REV packaging plasmid, and a lentiviral-GFP master plasmid (pGClenti-GFP plasmid) carrying a GFP (Green Fluorescent Protein) gene; and the envelope plasmid 1 was synthesized by conventional molecular cloning methods; and
the extracellular domain of the mutant VSV-G1 comprised the amino acid sequence as set forth in SEQ ID NO: 3; relative to SEQ ID NO: 1, SEQ ID NO: 3 comprises deletion of K47; the amino acid sequence as set forth in SEQ ID NO: 1 was the amino acid sequence comprised in the wild-type VSV-G extracellular domain; the full-length protein of the wild-type VSV-G (including the VSV-G signal peptide) had the amino acid sequence as set forth in SEQ ID NO: 22; and the full-length protein of the mutant VSV-G1 (including the VSV-G signal peptide) had the amino acid sequence as set forth in SEQ ID NO: 29.

The mutant VSV-G1 has the ability to specifically bind to LDL-R weakened or lost due to the deletion of the lysine at site 47 of the extracellular domain thereof, but still retains the ability to mediate membrane fusion and endosomal/lysosomal escape, thereby enabling a lentiviral vector comprising the mutant VSV-G1 in the viral envelope, such as the LVV-V5-GFP, to have improved specificity for targeted activation and transduction of non-activated T cells.

### B: Packaging and preparation of the LVV-V5-GFP:

(1) the 4 plasmids were mixed, and the 4 plasmids were transfected into a packaging cell line (HEK-293T cell line) through a PEI reagent, and the specific steps were:
   preparation of the HEK-293T cell culture system: 56 mL of FBS was filtered into 500 mL of DMEM/high glucose (10% FBS), and 4 mL of P/S (double antibody, penicillin × streptomycin) was added, and the resultant was shaken well, and placed in a carbon dioxide incubator to preheat for use during transfection.

At Day 0, 4.5×10⁶ HEK-293T cells were inoculated using a 10 cm culture dish, and after about 48 h of inoculation, when the cell confluence was 80%-90%, the 4 plasmids were transfected into the packaging cells (HEK-293T cells) through the PEI reagent, including:
9 µg of the master plasmid, 4 µg of the pMDLg/pRRE packaging plasmid, 2 µg of the pRSV-REV packaging plasmid and 2 µg of the envelope plasmid 1 were added to 1 mL of an Opti-MEM culture medium and shaken well; and then 64 µL of the PEI reagent was added, pipetted uniformly, allowed to stand for 10 minutes, and then added to the culture medium of the HEK-293T cells; and the culture medium was updated after 6 hours, the culture medium supernatant was collected 48 hours after transfection, filtered using a 0.45 µm filter membrane, and centrifuged at 50000g for 2.5 h, the supernatant was discarded, and the LVV-V5-GFP was resuspended using 200 µL of F12 culture medium and stored frozen at -80 °C.
Opti-MEM culture medium: Opti-MEM alpha reduced serum culture medium, brand: GIBCO; catalog number: #SP0272;
HEK-293T cell culture medium: DMEM + 10% FBS; DMEM: brand: GIBCO; catalog number: #C12430500BT; FBS: brand: EXCELL; catalog number: #FSP500;
F12 culture medium: brand: GIBCO; catalog number: #C11330500BT; and
syringe filter: brand: SORFA; catalog number: #622120.

### (2) Transduction of the LDL-R⁺CD3⁺ Jurkat cells, the LDL-R⁺CD3⁻ Nalm-6 cells and the human non-activated PBMCs (MOI = 5):

at Day 0, 1×10⁵ of LDL-R⁺CD3⁺ Jurkat cells, LDL-R⁺CD3⁻ Nalm-6 cells and LDL-R⁻CD3⁺ human non-activated PBMCs (thawing frozen non-activated PBMCs of healthy human Donor 1, in which the thawing method is well known to those skilled in the art) were respectively resuspended in 200 µL of culture media; the culture medium for resuspending the Jurkat cells and the Nalm-6 cells comprised 1640 (brand: ELGBIO, catalog number: #EH80809) culture medium and 10% FBS (brand: EXCELL, catalog number: #FSP500); the culture medium for resuspending the human non-activated PBMCs (PBMCs culture medium) comprised an XVT culture medium (trade name: PRIME-XV T cell CDM, brand: IRVINE (FUJIFILM), catalog number: #91154), IL-7 at a final concentration of 20 ng/mL (trade name: IL-7 Protein, Human, Recombinant, brand: Sino Biological, catalog number: #11821-HNAE) and IL-15 at a final concentration of 20 ng/mL (trade name: IL-15 Protein, Human, Recombinant (His Tag), brand: Sino Biological, catalog number: #10360-H07E).

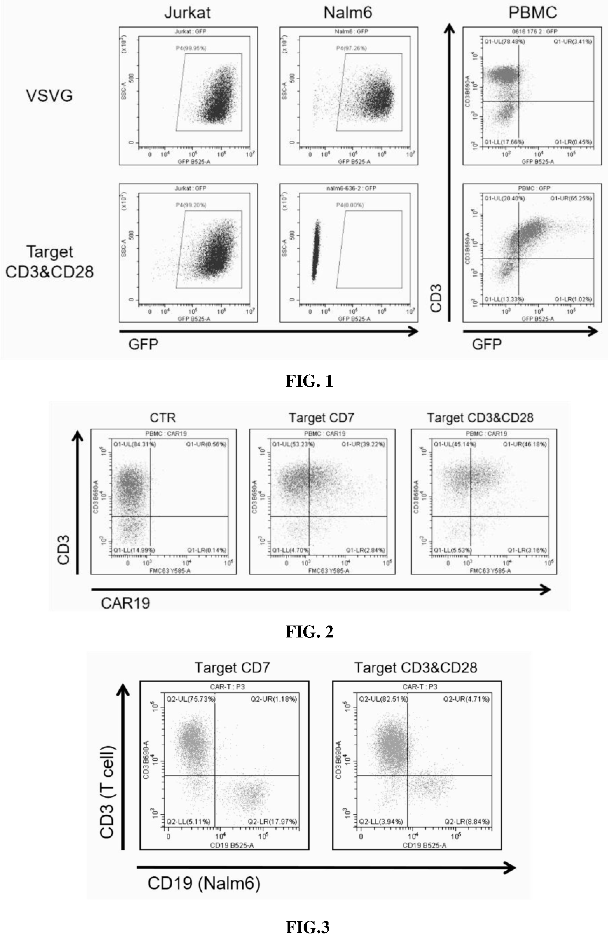

The LVV-V5-GFP was added at MOI = 5 to the cell culture systems of the Jurkat cells, the Nalm-6 cells and the human non-activated PBMCs respectively, mixed well, and cultured in a 5% CO₂ and 37 °C incubator;
at Day 5, the expression of GFP in the Jurkat cells, the Nalm-6 cells and the PBMCs was detected, and the results are as shown in FIG. 1 (lower panel of FIG. 1, "Target CD3&CD28").

### 3. Packaging of Control Wild-Type VSV-G Lentiviral Vector

(1) With reference to the above method for packaging the LVV-V5-GFP, a wild-type VSV-G lentiviral vector (wild-type LVV-GFP) was packaged, and the specific packaging steps were:
   the following 4 plasmids were prepared: a pMD2.G envelope plasmid (comprising a wild-type VSV-G gene), a pMDLg/pRRE packaging plasmid, a pRSV-REV packaging plasmid and the lentiviral-GFP master plasmid; the 4 plasmids were mixed, and the 4 plasmids were transfected into a packaging cell line (HEK-293T cell line) through a PEI reagent to package and prepare the wild-type LVV-GFP; and
   the extracellular domain of the wild-type VSV-G comprises the amino acid sequence as set forth in SEQ ID NO: 1; the viral envelope of the wild-type LVV-GFP did not comprise the anti-CD3 antibody or the anti-CD28 antibody but still had the ability to specifically bind to LDL-R.
(2) Transduction of the CD3⁺ Jurkat cells, the CD3⁻ Nalm-6 cells and the human non-activated PBMCs (MOI = 5)

At Day 0, with reference to the above method for the LVV-V5-GFP transducing the LDL-R⁺CD3⁺ Jurkat cells, the LDL-R⁺CD3⁻ Nalm-6 cells and the LDL-R⁺CD3⁺ human non-activated PBMCs, the wild-type LVV-GFP was used to respectively transduce the cells of each group, and the expression of GFP in the cells of each group was detected on Day 5, and the results are as shown in FIG. 1 (upper panel of FIG. 1, "VSVG").

### 4. Transduction Results

It can be seen from FIG. 1 that: the LVV-V5-GFP cannot effectively transduce the LDL-R⁺CD3⁻ Nalm-6 cells, but can effectively transduce the LDL-R⁺CD3⁺ Jurkat cells and the LDL-R⁻CD3⁺ human non-activated PBMCs; while the wild-type LVV-GFP can effectively transduce the LDL-R⁺CD3⁺ Jurkat cells and the LDL-R⁺CD3⁻ Nalm-6 cells, but is difficult to transduce the LDL-R⁻CD3⁺ human non-activated PBMCs.

Because the VSV-G is subjected to the first mutation, that is, the lysine at site 47 of the extracellular domain of the VSV-G is deleted (K47 deletion), the ability of the mutant VSV-G1 to specifically bind to LDL-R is weakened or even lost, and the LVV-V5-GFP is difficult to transduce the LDL-R⁺CD3⁻ Nalm-6 cells;
while the LVV-V5-GFP comprises, on the surface of the viral envelope, the T cell activation primary and secondary signaling molecules, that is, the membrane-expressed anti-CD3 antibody and anti-CD28 antibody, and can effectively activate non-activated T cells by binding to the endocytic receptor CD3 on the surface of the non-activated T cells in the Jurkat cells and the human non-activated PBMCs, and can also enter and effectively transduce the CD3⁺ non-activated T cells in the CD3⁺ Jurkat cells and the human non-activated PBMCs through endocytosis;
while the wild-type LVV-GFP can enter and transduce the Jurkat cells and the Nalm-6 cells by binding to the LDL-R on the surfaces of the Jurkat cells and the Nalm-6 cells, and the specificity of targeted transduction of T cells is significantly lower than that of the LVV-V5-GFP; and the wild-type LVV-GFP is difficult to effectively transduce non-activated T cells that do not express or express low levels of LDL-R in the human non-activated PBMCs.

Compared with the wild-type LVV-GFP, the ability of the LVV-V5-GFP to transduce LDL-R⁺ cells is inhibited, the specificity of targeted transduction and activation of CD3⁺ non-activated T cells is significantly improved, and the LVV-V5-GFP is more suitable for application in scenarios requiring targeted activation and transduction of non-activated T cells, such as *in vivo* CAR-T cell therapy.

### Example 2

Comparison of the expression efficiency of a lentiviral vector comprising, on a Viral Envelope, T Cell Activation Primary and Secondary Signaling Molecules, or a Membrane-Expressed Anti-CD7 Antibody, in transducing Non-Activated T Cells and delivering a CAR Gene Targeting CD19.

### 1. Design of Chimeric Antigen Receptor CAR-19

In this example, a chimeric antigen receptor targeting human CD19 (CAR-19) was designed (human CD19: Uniprot ID: P15391); and CD19 is an effective target for treating B-cell malignancies, particularly B-cell lymphoma and acute lymphoblastic leukemia.

The structure of the CAR-19 from the N-terminus to the C-terminus successively comprises: an antigen binding region targeting human CD19, the human CD8α hinge region, the human CD8α transmembrane domain, a human 4-1BB costimulatory signaling domain and a human CD3ζ intracellular signaling domain; and
the antigen binding region targeting human CD19 was an scFv (FMC63-scFv) derived from monoclonal antibody FMC-63 capable of specifically binding to human CD19, and the heavy chain variable region (VH) of the FMC63-scFv was linked through a (G₄S)₃ linker peptide to the light chain variable region (VL) of the scFv.

The 5' end of the polynucleotide encoding the CAR-19 was operably linked to the 3' end of the polynucleotide encoding the human CD8α signal peptide, and the human CD8α signal peptide was located at the N-terminus of the CAR-19;
(1) the amino acid sequence of the human CD8α signal peptide is as set forth in SEQ ID NO: 8;
(2) the amino acid sequence of the VH region of the FMC63-scFv is as set forth in SEQ ID NO: 14, and the amino acid sequence of the VL region of the FMC63-scFv is as set forth in SEQ ID NO: 15; the VH region and the VL region were linked through a (G₄S)₃ linker peptide, and the amino acid sequence of the (G₄S)₃ linker peptide is as set forth in SEQ ID NO: 16; the amino acid sequence of the FMC63-scFv is as set forth in SEQ ID NO: 30; and the amino acid sequences of the HCDR1-3 regions of the FMC63-scFv are respectively as set forth in SEQ ID NOs: 31-33, and the amino acid sequences of the LCDR1-3 regions of the FMC63-scFv are respectively as set forth in SEQ ID NOs: 34-36;
(3) the amino acid sequence of the hinge region of the human CD8α is as set forth in SEQ ID NO: 10;
(4) the amino acid sequence of the transmembrane domain of the human CD8α is as set forth in SEQ ID NO: 11;
(5) the amino acid sequence of the human 4-1BB costimulatory domain is as set forth in SEQ ID NO: 17; and
(6) the amino acid sequence of the intracellular signaling domain of the human CD3ζ is as set forth in SEQ ID NO: 18.

### 2. Packaging of LVV-V5-CAR19

The following 4 plasmids were prepared: the envelope plasmid 1, the pMDLg/pRRE packaging plasmid, the pRSV-REV packaging plasmid and a lentiviral master plasmid (CAR-19 master plasmid) carrying a polynucleotide encoding the CAR-19 (CAR-19 gene); and the CAR-19 master plasmid was synthesized by a conventional molecular cloning method;
with reference to the above packaging method of the LVV-V5-GFP, the lentiviral vector LVV-V5-CAR19 carrying the CAR-19 gene was packaged.

### 3. Packaging of LVV-V7-CAR19

The following 4 plasmids were prepared: an envelope plasmid 2 carrying a polynucleotide encoding the mutant VSV-G1 and a polynucleotide encoding a membrane-expressed anti-CD7 antibody, the pMDLg/pRRE packaging plasmid, the pRSV-REV packaging plasmid and the CAR-19 master plasmid; and the envelope plasmid 2 was synthesized by a conventional molecular cloning method; and
the membrane-expressed anti-CD7 antibody structurally comprised, from the N-terminus to the C-terminus: an scFv (TH69-scFv) derived from monoclonal antibody TH-69 capable of specifically binding to human CD7, the human CD8α hinge region, and the human CD8α transmembrane domain (human CD7: Uniprot ID: P15391).

The amino acid sequence of the VH region of the TH69-scFv is as set forth in SEQ ID NO: 19; the amino acid sequence of the VH region of the TH69-scFv is as set forth in SEQ ID NO: 19; the amino acid sequence of the TH69-scFv is as set forth in SEQ ID NO: 37; and the amino acid sequences of the HCDR1-3 regions of the TH69-scFv are respectively as set forth in SEQ ID NOs: 38-40, and the amino acid sequences of the LCDR1-3 regions of the TH69-scFv are respectively as set forth in SEQ ID NOs: 41-43;
the 5' end of the polynucleotide encoding the membrane-expressed anti-CD7 antibody (membrane-expressed TH69-scFv) was operably linked to the 3' end of the polynucleotide encoding the human CD8α signal peptide, and the CD8α signal peptide was located at the N-terminus of the membrane-expressed anti-CD7 antibody;
with reference to the packaging method of the LVV-V5-GFP, the LVV-V7-CAR19 carrying the CAR-19 gene was packaged.

### 4. Comparison of Transduction Efficiencies of the LVV-V5-CAR19 and the LVV-V7-CAR19 in Transducing Non-Activated T Cells

With reference to the transduction method of the LVV-V5-GFP transducing the human non-activated PBMCs, the LVV-V5-CAR19 and the LVV-V7-CAR19 were respectively used to transduce the human non-activated PBMCs at MOI = 5, and on Day 5, the efficiency of expression of CAR-19 in the CD3⁺ T cells in the PBMCs of each group was detected by flow cytometry, and the results are as shown in FIG. 2.

It can be seen from the middle panel of FIG. 2 (i.e., "Target CD7") that: after the LVV-V7-CAR19 transduces the human non-activated PBMCs, the efficiency of expression of the CAR-19 molecule in the CD3⁺ T cells is about 39.22%;
and it can be seen from the rightmost panel of FIG. 2 (i.e., "Target CD3&CD28") that: after the LVV-V5-CAR19 targetedly activates and transduces the non-activated T cells, the efficiency of expression of the CAR-19 molecule in the CD3⁺ T cells is about 46.18%; and
it can be seen that the transduction efficiency of the LVV-V5-CAR19, the viral envelope of which comprises the T cell activation primary and secondary signaling molecules, that is, the membrane-expressed anti-CD3 antibody and the anti-CD28 antibody, in transducing the non-activated T cells is significantly superior to that of the LVV-V7-CAR19, the viral envelope of which does not comprise the T cell activation primary and secondary signaling molecules and only comprises the targeting-molecule membrane-expressed anti-CD7 antibody.

### Flow cytometry antibodies used for detection:

Flow cytometry antibody for detecting CD3: trade name: FITC Mouse Anti-Human CD3; brand: BIOLEGEND, catalog number: #555339.
Flow cytometry antibody for detecting CAR-19 molecule: trade name: PE-Labeled Monoclonal Anti-FMC63 Antibody, Mouse IgG1 (Y45) (Site-specific conjugation) (0.03% Proclin) DMF Filed, brand: Acro, catalog number: #FM3-PY54A2-200 tests.

### Example 3

Detection of *In Vitro* Killing Efficiency of CD19-CAR-T Cells.

At Day 0, 1×10⁷ human non-activated PBMCs were respectively transduced using the LVV-V5-CAR19 and the LVV-V7-CAR19 at MOI = 5 to prepare two groups of CD19-targeted CAR-T cells (CD19-CAR-T cells);
at Day 2, the two groups of CD19-CAR-T cells were counted using a cell counter (brand: COUNTERSTAR, model: Rigel S2), and 1×10⁶ CD19⁺ target cells (Nalm-6 cells) (human B-lymphocytic leukemia cells) were respectively added to the two groups of CD19-CAR-T cells at an effector-to-target ratio (E:T) = 1:1; and
at Day 4, the killing efficiency of the two groups of CD19-CAR-T cells killing the Nalm-6 cells was respectively detected by flow cytometry, and the results are respectively as shown in FIG. 3.

It can be seen from FIG. 3 that: on Day 4, the expression of CD19 in the mixed cells added with the LVV-V5-CAR19 is only 8.84%; while the expression of CD19 in the mixed cells added with the LVV-V7-CAR19 is still 17.97%. Thid indicates that on Day 4, the killing efficiency of the activated CD19-CAR-T cells prepared by the LVV-V5-CAR19 transducing the CD3⁺ T cells in the human non-activated PBMCs is significantly superior to that of the non-activated or relatively insufficiently activated CD19-CAR-T cells prepared by the LVV-V7-CAR19 transducing the human non-activated PBMCs.

### Example 4

With reference to the methods in Example 2 for packaging the LVV-V5-CAR19 and the LVV-V7-CAR19, the LVV-V5-CAR19 and the LVV-V7-CAR19 were packaged in the same batch.

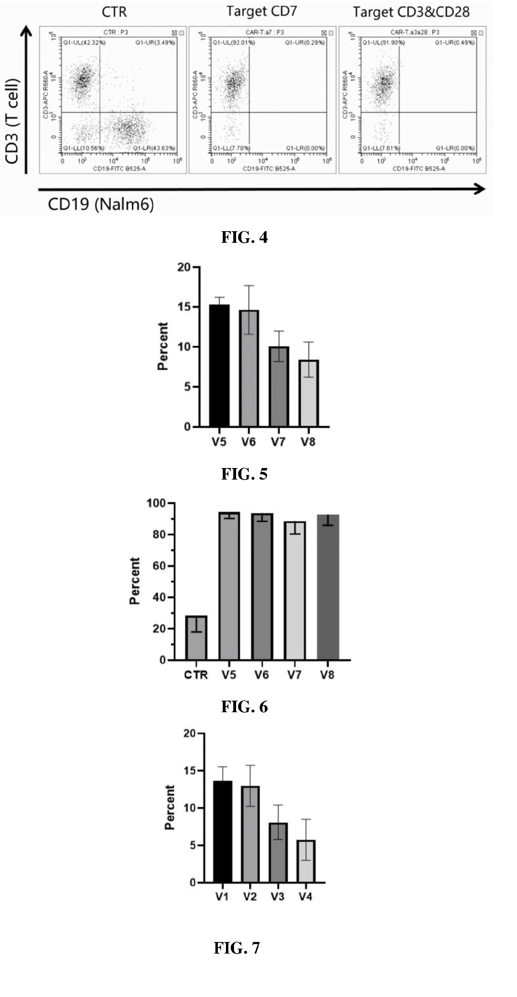

At Day 0, 1×10⁷ human non-activated PBMCs were respectively transduced using the LVV-V5-CAR19 and the LVV-V7-CAR19 at MOI = 5 to prepare two groups of CD19-CAR-T cells;
at Day 2, the two groups of CD19-CAR-T cells were counted using a cell counter, and at an effector-to-target ratio (E:T)= 1:1, 1×10⁶ CD19⁺ target cells (Nalm-6 cells) were respectively added to the two groups of CD19-CAR-T cells; and
at Day 7, the killing efficiency of the two groups of CD19-CAR-T cells killing the Nalm-6 cells was respectively detected by flow cytometry, and the results are as shown in FIG. 4.

It can be seen from FIG. 4 that: on Day 7, the sustained killing efficiencies of the two groups of CD19-CAR-T cells are comparable.

### Example 5

### 1. Design of Polynucleotide Encoding Membrane-Expressed Anti-CD3 Antibody × Membrane-Expressed CD86 Structure

A membrane-expressed CD86 was constructed, and the membrane-expressed CD86 sequentially comprised, from the N-terminus to the C-terminus: a human CD86 extracellular domain and a human CD86 transmembrane domain (CD86-ECD+TM);
the 5' end of the polynucleotide encoding the human CD86-ECD+TM was operably linked to the 3' end of the polynucleotide encoding the human CD86 signal peptide;
the amino acid sequence of the human CD86-ECD+TM is as set forth in SEQ ID NO: 79; and the amino acid sequence of the human CD86-ECD+TM comprising the human CD86 signal peptide is as set forth in SEQ ID NO: 80:
   Human CD86-ECD+TM: 
APLKIQAYFNETADLPCQFANSQNQSLSELVVFWQDQENLVLNEVYLGKEKFDSVHS
KYMGRTSFDSDSWTLRLHNLQIKDKGLYQCIIHHKKPTGMIRIHQMNSELSVLANFS
QPEIVPISNITENVYINLTCSSIHGYPEPKKMSVLLRTKNSTIEYDGVMQKSQDNVTEL
YDVSISLSVSFPDVTSNMTIFCILETDKTRLLSSPFSIELEDPQPPPDHIPWITAVLPTVIIC
VMVFCLILW (SEQ ID NO: 79);
   Human CD86 signal peptide + human CD86-ECD+TM:

MDPQCTMGLSNILFVMAFLLSGAAPLKIQAYFNETADLPCQFANSQNQSLSELVVFW
QDQENLVLNEVYLGKEKFDSVHSKYMGRTSFDSDSWTLRLHNLQIKDKGLYQCIIHH
KKPTGMIRIHQMNSELSVLANFSQPEIVPISNITENVYINLTCSSIHGYPEPKKMSVLLR
TKNSTIEYDGVMQKSQDNVTELYDVSISLSVSFPDVTSNMTIFCILETDKTRLLSSPFSI
ELEDPQPPPDHIPWITAVLPTVIICVMVFCLILW (SEQ ID NO: 80).

The sites 1-23 (underlined) of SEQ ID NO: 80 are the amino acid sequence of the human CD86 signal peptide: MDPQCTMGLSNILFVMAFLLSGA (SEQ ID NO: 96).

In this example, the polynucleotide encoding the membrane-expressed anti-CD3 antibody (the membrane-expressed UCHT1-scFv) × membrane-expressed CD86 sequentially comprised, from the 5' end to the 3' end: a polynucleotide encoding the human CD8α signal peptide, a polynucleotide encoding the UCHT1-scFv, a polynucleotide encoding the human CD8α hinge region, a polynucleotide encoding the human CD8α transmembrane domain, a polynucleotide encoding the FT2A peptide, a polynucleotide encoding the human CD86 signal peptide, and a polynucleotide encoding the human CD86-ECD+TM.

When transfected into packaging cells, the membrane-expressed UCHT1-scFv and the membrane-expressed CD86-ECD+TM can be separately and membrane-expressed on the cell membrane of the packaging cells under the action of the self-cleaving peptide FT2A peptide.

### 2. Packaging of Multiple Groups of Lentiviral Vectors

With reference to the method in Example 2 for packaging the LVV-V5-CAR19, multiple groups of lentiviral vectors were packaged in the same batch: the LVV-V5-CAR19, the LVV-V6-CAR19, the LVV-V7-CAR19 and the LVV-V8-CAR19:
the LVV-V6-CAR19: (a) the viral envelope comprised (i) a T cell activation primary signaling molecule, that is, the membrane-expressed UCHT1-scFv, and a T cell activation secondary signaling molecule, that is, the membrane-expressed CD86-ECD+TM; and (ii) the mutant VSV-G1; and (b) comprised the CAR-19 gene; and
the LVV-V8-CAR19: (a) the viral envelope comprises (i) a T cell activation primary signaling molecule, i.e., the membrane-expressed UCHT1-scFv, and (ii) the mutant VSV-G1; and (b) comprised the CAR-19 gene.

### 3. Transduction of Human Non-Activated PBMCs, and Detection of Expression Efficiency of CAR-19

With reference to the method in Example 2 for using the LVV-V5-CAR19 to transduce the human non-activated PBMCs, the LVV-V5-CAR19, the LVV-V6-CAR19, the LVV-V7-CAR19 and the LVV-V8-CAR19 were respectively used to transduce the non-activated PBMCs of Donor 1 at MOI = 1; and on Day 2, the efficiency of expression of CAR-19 in the PBMCs of each group was detected by flow cytometry, and the results are as shown in FIG. 5.

It can be seen from FIG. 5 that: the efficiencies of expression of CAR-19 in the human non-activated PBMCs transduced by the LVV-V5-CAR19 and the LVV-V6-CAR19 are comparable; but both are significantly superior to the transduction efficiencies of the LVV-V7-CAR19 and the LVV-V8-CAR19. This may be because the anti-CD7 antibody does not have the ability to activate and stimulate non-activated T cells, so the efficiency of expression of CAR-19 in the non-activated T cells is lower; and in the absence of the T cell activation secondary signaling molecule, the T cell activation primary signaling molecule alone also cannot sufficiently activate and stimulate the non-activated T cells, resulting in a lower efficiency of expression of CAR-19.

In summary, constructing the T cell activation primary signaling molecule and the T cell activation secondary signaling molecule on the surface of a lentiviral vector comprising the mutant VSV-G1 with weakened or lost ability to specifically bind to LDL-R can not only improve the specificity of targeted transduction of T cells by the lentiviral vector to improve the transduction efficiency, but also can sufficiently activate and stimulate the non-activated T cells to further improve the transduction efficiency.

### 4. Detection of Killing Efficiency of CAR-T Cells Prepared by Transduction of PBMCs by Each Group of Lentiviral Vectors

At Day 0, 5 groups of 1×10⁶ human non-activated PBMCs of Donor 1 were respectively mixed with Nalm-6 cells at an effector-to-target ratio E:T= 1:1 and added to a culture medium (1640 + 10% FBS); the LVV-V5-CAR19, the LVV-V6-CAR19, the LVV-V7-CAR19 and the LVV-V8-CAR19 were respectively added to 4 groups of mixed cells at MOI = 1; and the control group was a mixed cell group without addition of any lentiviral vector (CTR group).

At Day 5, the expression of CD19 in the mixed cells of each group was detected by flow cytometry to detect the killing efficiency of the CD19-CAR-T cells prepared by transduction of the human non-activated PBMCs by each group of lentiviral vectors, and the specific method was: using a blank group of Nalm-6 cells without added PBMCs and lentiviral vectors as a background value, calculating the proportion of remaining Nalm-6 cells in the mixed cells of each group, and then calculating the killing proportion. The results are as shown in FIG. 6.

It can be seen from FIG. 6 that: on Day 5, the CD19-CAR-T cells prepared by the LVV-V5-CAR19, the LVV-V6-CAR19, the LVV-V7-CAR19 and the LVV-V8-CAR19 transducing the human non-activated PBMCs can all effectively kill the Nalm-6 cells.

### Example 6

### 1. Packaging of Each Group of Lentiviral Vectors Comprising Mutant VSV-G2

With reference to the method in Example 2 for packaging the LVV-V5-CAR19, multiple groups of lentiviral vectors comprising, on viral envelopes, the mutant VSV-G2 were packaged in the same batch: LVV-V1-CAR19, LVV-V2-CAR19, LVV-V3-CAR19 and LVV-V4-CAR19; and
the extracellular domain of the mutant VSV-G2 comprised the amino acid sequence as set forth in SEQ ID NO: 4; relative to SEQ ID NO: 1, SEQ ID NO: 4 comprises R354Q, such that the ability of the mutant VSV-G2 to specifically bind to LDL-R is weakened or lost, but the ability to mediate membrane fusion and endosomal/lysosomal escape is retained.

The LVV-V1-CAR19: (a) the viral envelope comprised (i) a T cell activation primary signaling molecule, that is, the membrane-expressed UCHT1-scFv, and a T cell activation secondary signaling molecule, that is, the membrane-expressed 15E8-scFv; and (ii) the mutant VSV-G2; and (b) comprised the CAR-19 gene;
the LVV-V2-CAR19: (a) the viral envelope comprised (i) a T cell activation primary signaling molecule, that is, the membrane-expressed UCHT1-scFv, and a T cell activation secondary signaling molecule, that is, the membrane-expressed CD86-ECD+TM; and (ii) the mutant VSV-G2; and (b) comprised the CAR-19 gene;
the LVV-V3-CAR19: (a) the viral envelope comprised (i) a membrane-expressed anti-CD7 antibody, that is, the membrane-expressed TH69-scFv, and (ii) the mutant VSV-G2; and (b) comprised the CAR-19 gene; and
the LVV-V4-CAR19: (a) the viral envelope comprised (i) a T cell activation primary signaling molecule, i.e., anti-CD3 antibody, that is, the membrane-expressed UCHT1-scFv, and (ii) the mutant VSV-G2; and (b) comprised the CAR-19 gene.

### 2. Transduction of Human Non-Activated PBMCs, and Detection of Expression Efficiency of CAR-19 (MOI = 1)

With reference to the method in Example 2 for using the LVV-V5-CAR19 to transduce the human non-activated PBMCs, the LVV-V1-CAR19, the LVV-V2-CAR19, the LVV-V3-CAR19 and the LVV-V4-CAR19 were respectively used to transduce the non-activated PBMCs of Donor 1 at MOI = 1; and on Day 2, the efficiency of expression of CAR-19 in the PBMCs of each group was detected by flow cytometry, and the results are as shown in FIG. 7.

It can be seen from FIG. 7 that: the efficiencies of expression of CAR-19 in the human non-activated PBMCs transduced by the LVV-V1-CAR19 and the LVV-V2-CAR19 are comparable; but both are significantly superior to the transduction efficiencies of the LVV-V3-CAR19 and the LVV-V4-CAR19.

### 3. Detection of Killing Efficiency of CAR-T Cells Prepared by Transduction of PBMCs by Each Group of Lentiviral Vectors

At Day 0, with reference to the method in Example 4 for detecting the killing efficiency of the CD19-CAR-T cells prepared by each group of lentiviral vectors transducing the human non-activated PBMCs, the LVV-V1-CAR19, the LVV-V2-CAR19, the LVV-V3-CAR19 and the LVV-V4-CAR19 were respectively added to 4 groups of mixed cell culture media; and the control group was a mixed cell group without addition of any lentiviral vector (CTR group).

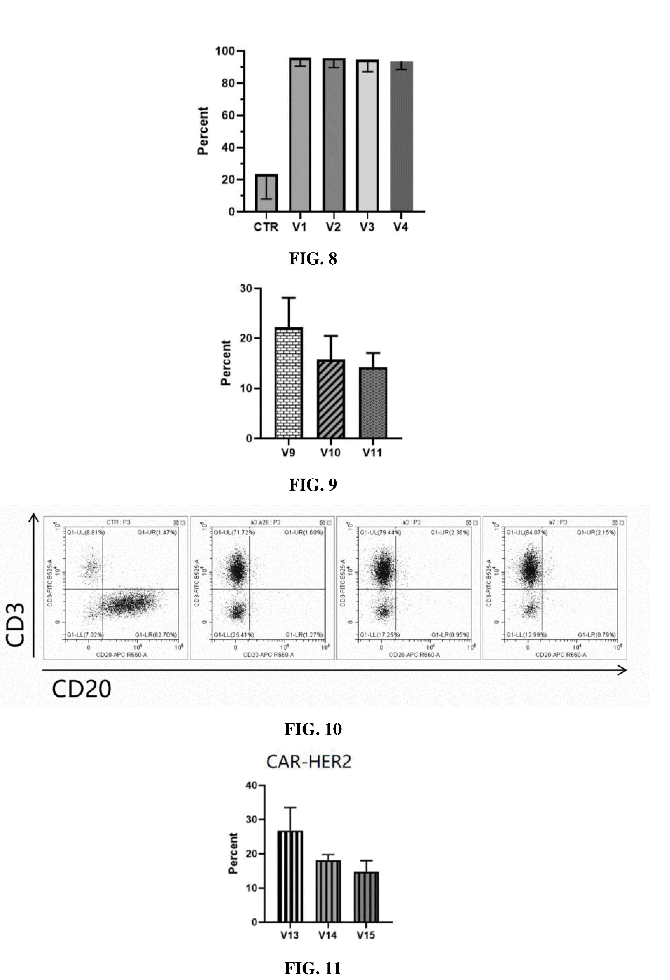

At Day 5, the expression of CD19 in the mixed cells of each group was detected by flow cytometry to calculate the killing efficiency of each group of CD19-CAR-T cells; and the results are as shown in FIG. 8.

It can be seen from FIG. 8 that: on Day 5, the CD19-CAR-T cells prepared by the LVV-V1-CAR19, the LVV-V2-CAR19, the LVV-V3-CAR19 and the LVV-V4-CAR19 transducing the human non-activated PBMCs can all effectively kill the Nalm-6 cells.

### Example 7

### 1. Design of CAR Targeting CD20

A CAR targeting human CD20 (CAR-20) was designed; and the polynucleotide encoding the CAR-20 (CAR-20 gene) sequentially comprised, from the 5' end to the 3' end:
a polynucleotide encoding the human CD8α signal peptide, a polynucleotide encoding an antigen binding region targeting human CD20, a polynucleotide encoding the human CD8α hinge region, a polynucleotide encoding the human CD8α transmembrane domain, a polynucleotide encoding the human 4-1BB costimulatory signaling domain, and a polynucleotide encoding the human CD3 ζ intracellular signaling domain; and
human CD20: Uniprot ID: P11836. CD20 is an effective target for treating B-cell malignancies, such as non-Hodgkin lymphoma and chronic lymphocytic leukemia.

The antigen binding region targeting human CD20 was an scFv (2f2-scFv) derived from monoclonal antibody 2f2, the amino acid sequence of the 2f2-scFv is as set forth in SEQ ID NO: 44; the amino acid sequences of the HCDR1-3 regions of the 2f2-scFv are respectively as set forth in SEQ ID NOs: 45-47; and the amino acid sequences of the LCDR1-3 regions of the 2f2-scFv are respectively as set forth in SEQ ID NOs: 48-50.

### 2. Packaging of Multiple Groups of Lentiviral Vectors Comprising the CAR-20 Gene

With reference to the method for packaging the LVV-V5-CAR19, multiple groups of lentiviral vectors LVV-V9-CAR20, LVV-V10-CAR20 and LVV-V11-CAR20 were packaged in the same batch:
the LVV-V9-CAR20: (a) the viral envelope comprised (i) a T cell activation primary signaling molecule, that is, the membrane-expressed UCHT1-scFv, and a T cell activation secondary signaling molecule, that is, the membrane-expressed 15E8-scFv; and (ii) the mutant VSV-G1; and (b) comprised the CAR-20 gene;
the LVV-V10-CAR20: (a) the viral envelope comprised (i) a membrane-expressed anti-CD7 antibody, that is, the membrane-expressed TH69-scFv, and (ii) the mutant VSV-G1; and (b) comprised the CAR-20 gene; and
the LVV-V11-CAR20: (a) the viral envelope comprised (i) a T cell activation primary signaling molecule, that is, the membrane-expressed UCHT1-scFv, and (ii) the mutant VSV-G1; and (b) comprised the CAR-20 gene.

### 3. Transduction of Human Non-Activated PBMCs, and Detection of Expression Efficiency of CAR-20 (MOI = 1)

At Day 0, the LVV-V9-CAR20, the LVV-V10-CAR20 and the LVV-V11-CAR20 were respectively added to the culture systems of the non-activated PBMCs of Donor 1 in each group at MOI = 1; and on Day 2, the efficiency of expression of CAR-20 in the PBMCs of each group was detected by flow cytometry, and the results are as shown in FIG. 9.

It can be seen from FIG. 9 that: the transduction efficiency of the LVV-V9-CAR20 transducing the human non-activated PBMCs and delivering the CAR-20 gene is significantly superior to those of the LVV-V10-CAR20 and the LVV-V11-CAR20.

Flow cytometry detection antibody: Anti-G4Slinker, brand: HYS-BIO, catalog number: #GS-ARPE100.

### 4. Detection of Killing Efficiency of CD20-CAR-T Cells Prepared by Transduction of PBMCs by Each Group of Lentiviral Vectors

At Day 0, 4 groups of 1×10⁶ human non-activated PBMCs of Donor 1 were respectively mixed with CD20⁺Dakiki cells (human B lymphocytes) at an effector-to-target ratio E:T = 1:1 and added to a culture medium (1640 + 10% FBS); the LVV-V9-CAR20, the LVV-V10-CAR20 and the LVV-V11-CAR20 were respectively added to 3 groups of mixed cell culture media at MOI = 1; and the control group (CTR group) was a mixed cell group not comprising any lentiviral vector.

At Day 5, the number of mixed cells of each group was counted using a cell counter, and the expression of CD20 in the mixed cells of each group was detected by flow cytometry, and the results are as shown in FIG. 10.

It can be seen from FIG. 10 that: on Day 5, the expression of CD20 in the mixed cells of each group is significantly reduced relative to the control group, and the CD20-CAR-T cells prepared by the LVV-V9-CAR20, the LVV-V10-CAR20 and the LVV-V11-CAR20 transducing the human non-activated PBMCs can all effectively kill the CD20⁺Dakiki cells.

### Example 8

### 1. Design of CAR Targeting HER-2

A CAR targeting human HER-2 (CAR-HER2) was designed, and the polynucleotide encoding the CAR-HER2 (CAR-HER2 gene) sequentially comprised, from the 5' end to the 3' end:
a polynucleotide encoding the human CD8α signal peptide, a polynucleotide encoding an antigen binding region targeting human HER-2, a polynucleotide encoding the human CD8α hinge region, a polynucleotide encoding the human CD8α transmembrane domain, a polynucleotide encoding the human 4-1BB costimulatory signaling domain, and a polynucleotide encoding the human CD3 ζ intracellular signaling domain; and
human HER-2: Uniprot ID: P04626. HER-2 is an effective target for treating HER-2⁺ solid cancers such as breast cancer and gastric cancer.

The antigen binding region targeting human HER-2 was an scFv (Pertuzumab-scFv) derived from monoclonal antibody Pertuzumab, the amino acid sequence of the Pertuzumab-scFv is as set forth in SEQ ID NO: 51; the amino acid sequences of the HCDR1-3 regions of the Pertuzumab-scFv are respectively as set forth in SEQ ID NOs: 52-54; and the amino acid sequences of the LCDR1-3 regions of the Pertuzumab-scFv are respectively as set forth in SEQ ID NOs: 55-57.

### 2. Packaging of Multiple Groups of Lentiviral Vectors Comprising the CAR-HER2 Gene

With reference to the method for packaging the LVV-V5-CAR19, multiple groups of lentiviral vectors LVV-V13-CARHER2, LVV-V14-CARHER2 and LVV-V15-CARHER2 were packaged in the same batch:
the LVV-V13-CARHER2: (a) the viral envelope comprised (i) a T cell activation primary signaling molecule, that is, the membrane-expressed UCHT1-scFv, and a T cell activation secondary signaling molecule, that is, the membrane-expressed 15E8-scFv; and (ii) the mutant VSV-G1; and (b) comprised the CAR-HER2 gene;
the LVV-V14-CARHER2: (a) the viral envelope comprises (i) a membrane-expressed anti-CD7 antibody, that is, the membrane-expressed TH69-scFv, and (ii) the mutant VSV-G1; and (b) comprised the CAR-HER2 gene; and
the LVV-V15-CARHER2: (a) the viral envelope comprises (i) a T cell activation primary signaling molecule, that is, the membrane-expressed UCHT1-scFv, and (ii) the mutant VSV-G1; and (b) comprised the CAR-HER2 gene.

### 3. Transduction of Human Non-Activated PBMCs, and Detection of Expression Efficiency of CAR-HER2 (MOI = 1)

At Day 0, the LVV-V13-CARHER2, the LVV-V14-CARHER2 and the LVV-V15-CARHER2 were respectively added to the culture systems of the non-activated PBMCs of Donor 1 in each group at MOI = 1; and at Day 2, the efficiency of expression of CAR-HER2 in the PBMCs of each group was detected by flow cytometry, and the results are as shown in FIG. 11.

It can be seen from FIG. 11 that: the transduction efficiency of the LVV-V13-CARHER2 transducing the human non-activated PBMCs and delivering the CAR-HER2 gene is about 28%, which is significantly superior to those of the LVV-V14-CARHER2 (about 18%) and the LVV-V15-CARHER2 (about 15%).

Flow cytometry detection antibody: Anti-G4Slinker, brand: HYS-BIO, catalog number: #GS-ARPE100.

### 4. Detection of Killing Efficiency of HER2-CAR-T Cells Prepared by Transduction of PBMCs by Each Group of Lentiviral Vectors

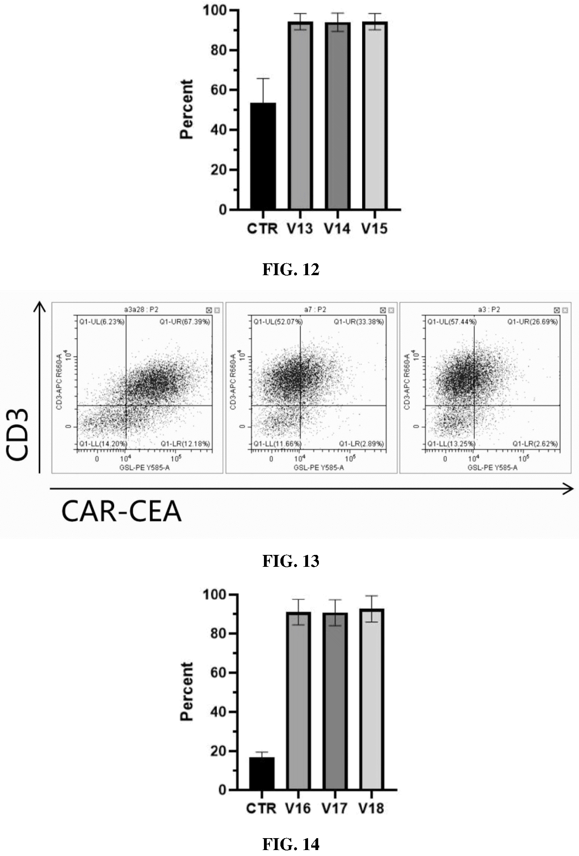

At Day 0, 4 groups of 1×10⁶ human non-activated PBMCs of Donor 1 were respectively mixed with HER-2⁺OVCAR-3 cells (human ovarian cancer cells) transduced with a luciferase gene at an effector-to-target ratio E:T = 1:1 and added to a culture medium (1640 + 10% FBS); the LVV-V13-CARHER2, the LVV-V14-CARHER2 and the LVV-V15-CARHER2 were respectively added to 3 groups of mixed cell culture media at MOI = 1; and the control group did not comprise any lentiviral vector, and only comprised the mixed cells (CTR group); and
at Day 2, a microplate reader was used to detect and calculate the killing efficiency of each group of HER2-CAR-T cells, and the specific method was: using the fluorescence value of a blank group without added PBMCs and LVV and only comprising target cells OVCAR-3 cells as a background value, which was the total fluorescence value of the original tumor cell number, wherein the tumor fluorescence value remaining after the HER2-CAR-T cells of each group killed the OVCAR-3 cells was a remaining fluorescence value, and the killing efficiency calculation formula was as follows: killing efficiency (%) = (total fluorescence value - remaining fluorescence value) / total fluorescence value × 100%; and the calculation results are as shown in FIG. 12.

It can be seen from FIG. 12 that: on Day 2, the HER2-CAR-T cells prepared by the LVV-V13-CARHER2, the LVV-V14-CARHER2 and the LVV-V15-CARHER2 transducing the human non-activated PBMCs can all effectively kill the HER-2⁺OVCAR-3 cells.

### Example 9

### 1. Design of CAR Targeting CEA

A CAR targeting human CEA (CAR-CEA) was designed, and the polynucleotide encoding the CAR-CEA (CAR-CEA gene) sequentially comprised, from the 5' end to the 3' end:
a polynucleotide encoding the human CD8α signal peptide, a polynucleotide encoding an antigen binding region targeting human CEA, a polynucleotide encoding the human CD8α hinge region, a polynucleotide encoding the human CD8α transmembrane domain, a polynucleotide encoding the human 4-1BB costimulatory signaling domain, and a polynucleotide encoding the human CD3ζ intracellular signaling domain; and
human CEA: Uniprot ID: P06731. CEA is an effective target for treating CEA⁺ solid cancers such as colorectal cancer, gastric cancer, pancreatic cancer and lung cancer.

The antigen binding region targeting human CEA was an scFv (anti-CEA-scFv) of anti-human CEA, the amino acid sequence of the anti-CEA-scFv is as set forth in SEQ ID NO: 58; the amino acid sequences of the HCDR1-3 regions of the anti-CEA-scFv are respectively as set forth in SEQ ID NOs: 59-61; and the amino acid sequences of the LCDR1-3 regions of the anti-CEA-scFv are respectively as set forth in SEQ ID NOs: 62-64.

### 2. Packaging of Multiple Groups of Lentiviral Vectors Comprising the CAR-CEA Gene

With reference to the method for packaging the LVV-V5-CAR19, multiple groups of lentiviral vectors LVV-V16-CARCEA, LVV-V17-CARCEA and LVV-V18-CARCEA were packaged in the same batch:
the LVV-V16-CARCEA: (a) the viral envelope comprised (i) a T cell activation primary signaling molecule, that is, the membrane-expressed UCHT1-scFv, and a T cell activation secondary signaling molecule, that is, the membrane-expressed 15E8-scFv; and (ii) the mutant VSV-G1; and (b) comprised the CAR-CEA gene;
the LVV-V17-CARCEA: (a) the viral envelope comprises (i) a membrane-expressed anti-CD7 antibody, that is, the membrane-expressed TH69-scFv, and (ii) the mutant VSV-G1; and (b) comprised the CAR-CEA gene; and
the LVV-V18-CARCEA: (a) the viral envelope comprises (i) a T cell activation primary signaling molecule, that is, the membrane-expressed UCHT1-scFv, and (ii) the mutant VSV-G1; and (b) comprises the CAR-CEA gene.

### 3. Transduction of Human Non-Activated PBMCs, and Detection of Expression Efficiency of CAR-CEA (MOI = 1)

At Day 0, the LVV-V16-CARCEA, the LVV-V17-CARCEA and the LVV-V18-CARCEA were respectively added to the culture systems of the non-activated PBMCs of Donor 1 in each group at MOI = 1; and on Day 2, the efficiency of expression of CAR-CEA in the CD3⁺ T cells in the PBMCs of each group was detected by flow cytometry, and the results are as shown in FIG. 13.

It can be seen from FIG. 13 that: the transduction efficiency of the LVV-V16-CARCEA transducing the CD3⁺ T cells in the human non-activated PBMCs and delivering the CAR-CEA gene is about 67.39%, which is significantly superior to those of the LVV-V17-CARCEA (about 33.38%) and the LVV-V18-CARCEA (about 26.69%).

Flow cytometry detection antibody: Anti-G4Slinker, brand: HYS-BIO, catalog number: #GS-ARPE100.

### 4. Detection of Killing Efficiency of CEA-CAR-T Cells Prepared by Transduction of PBMCs by Each Group of Lentiviral Vectors

At Day 0, 4 groups of 1×10⁶ human non-activated PBMCs of Donor 1 were respectively mixed with CEA⁺T-84 cells (human colonic adenocarcinoma cells) transduced with a luciferase gene at an effector-to-target ratio E:T = 1:1 and added to a culture medium (1640 + 10% FBS); the LVV-V16-CARCEA, the LVV-V17-CARCEA and the LVV-V18-CARCEA were respectively added to 3 groups of mixed cell culture media at MOI = 1; and the control group (CTR) was the mixed cells not comprising any lentiviral vector.

At Day 2, a microplate reader was used to detect and calculate the killing efficiency of each group of CEA-CAR-T cells, and the calculation results are as shown in FIG. 14.

It can be seen from FIG. 14 that: on Day 2, the CEA-CAR-T cells prepared by the LVV-V16-CARCEA, the LVV-V17-CARCEA and the LVV-V18-CARCEA transducing the human non-activated PBMCs can all effectively kill the CEA⁺T-84 cells.

### Example 10

### 1. Design of CAR Targeting CD33

A CAR targeting human CD33 (CAR-33) was designed, and the polynucleotide encoding the CAR-33 (CAR-33 gene) sequentially comprised, from the 5' end to the 3' end:
a polynucleotide encoding the human CD8α signal peptide, a polynucleotide encoding an antigen binding region targeting human CD33, a polynucleotide encoding the human CD8α hinge region, a polynucleotide encoding the human CD8α transmembrane domain, a polynucleotide encoding the human 4-1BB costimulatory signaling domain, and a polynucleotide encoding the human CD3ζ intracellular signaling domain;
human CD33: Uniprot ID: P20138. CD33 is an effective target for treating blood cancers such as acute myeloid leukemia.

The antigen binding region targeting human CD33 was an scFv (Gemtuzumab-scFv) derived from monoclonal antibody Gemtuzumab, the amino acid sequence of the Gemtuzumab-scFv is as set forth in SEQ ID NO: 65; the amino acid sequences of the HCDR1-3 regions of the Gemtuzumab-scFv are respectively as set forth in SEQ ID NOs: 66-68; and the amino acid sequences of the LCDR1-3 regions of the Gemtuzumab-scFv are respectively as set forth in SEQ ID NOs: 69-71.

### 2. Packaging of Multiple Groups of Lentiviral Vectors Comprising the CAR-33 Gene

With reference to the method for packaging the LVV-V5-CAR19, multiple groups of lentiviral vectors LVV-V19-CAR33, LVV-V20-CAR33 and LVV-V21-CAR33 were packaged in the same batch:
the LVV-V19-CAR33: (a) the viral envelope comprised (i) a T cell activation primary signaling molecule, that is, the membrane-expressed UCHT1-scFv, and a T cell activation secondary signaling molecule, that is, the membrane-expressed 15E8-scFv; and (ii) the mutant VSV-G1; and (b) comprised the CAR-33 gene;
the LVV-V20-CAR33: (a) the viral envelope comprised (i) a membrane-expressed anti-CD7 antibody, that is, the membrane-expressed TH69-scFv, and (ii) the mutant VSV-G1; and (b) comprised the CAR-33 gene; and
the LVV-V21-CAR33: (a) the viral envelope comprised (i) a T cell activation primary signaling molecule, that is, the membrane-expressed UCHT1-scFv, and (ii) the mutant VSV-G1; and (b) comprised the CAR-33 gene.

### 3. Transduction of Human Non-Activated PBMCs, and Detection of Expression Efficiency of CAR-33 (MOI = 1)

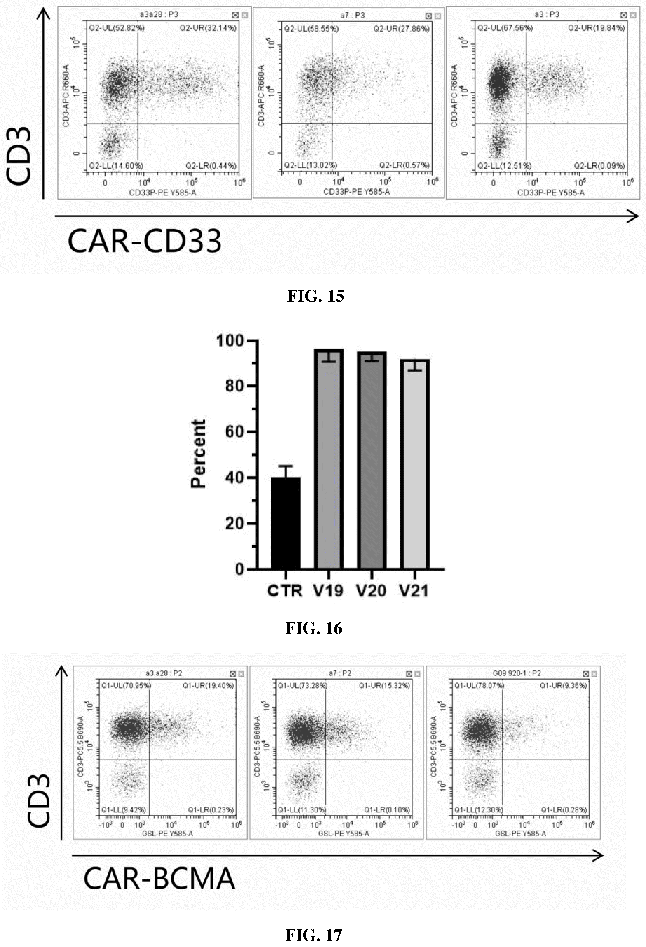

At Day 0, the LVV-V19-CAR33, the LVV-V20-CAR33 and the LVV-V21-CAR33 were respectively added to the culture systems of the non-activated PBMCs of Donor 1 in each group at MOI = 1; and at Day 2, the efficiency of expression of CAR-33 in the CD3⁺ T cells in the PBMCs of each group was detected by flow cytometry, and the results are as shown in FIG. 15.

It can be seen from FIG. 15 that: the transduction efficiency of the LVV-V19-CAR33 transducing the CD3⁺ T cells in the human non-activated PBMCs and delivering the CAR-33 gene (about 32.14%) is significantly superior to those of the LVV-V20-CAR33 (about 27.86%) and the LVV-V21-CAR33 (about 19.84%).

Flow cytometry detection antibody: PE-Labeled Human Siglec-3 / CD33 Protein, brand: Acro, catalog number: #CD3-HP2E3.

### 4. Detection of Killing Efficiency of CD33-CAR-T Cells Prepared by Transduction of PBMCs by Each Group of Lentiviral Vectors

At Day 0, 4 groups of 1×10⁶ human non-activated PBMCs of Donor 1 were respectively mixed with CD33⁺MOLM-13 cells (human acute myeloid leukemia cells) at an effector-to-target ratio E:T = 1:1 and added to a culture medium (1640 + 10% FBS); and the LVV-V19-CAR33, the LVV-V20-CAR33 and the LVV-V21-CAR33 were respectively added to 3 groups of mixed cell culture media at MOI = 1; and the control group (CTR) did not comprise any lentiviral vector, and only comprised the mixed cells.

At Day 5, the number of mixed cells of each group was counted using a cell counter, and the expression of CD33 in the mixed cells of each group was detected by flow cytometry to calculate the killing efficiency, and the results are as shown in FIG. 16.

It can be seen from FIG. 16 that: on Day 5, compared with the control group, the CD33-CAR-T cells prepared by the LVV-V19-CAR33, the LVV-V20-CAR33 and the LVV-V21-CAR33 transducing the human non-activated PBMCs can all effectively kill the CD33⁺MOLM-13 cells.

### Example 11

### 1. Design of CAR Targeting BCMA

A CAR targeting human BCMA (CAR-BCMA) was designed, and the polynucleotide encoding the CAR-BCMA (CAR-BCMA gene) sequentially comprised, from the 5' end to the 3' end:
a polynucleotide encoding the human CD8α signal peptide, a polynucleotide encoding an antigen binding region targeting human BCMA, a polynucleotide encoding the human CD8α hinge region, a polynucleotide encoding the human CD8α transmembrane domain, a polynucleotide encoding the human 4-1BB costimulatory signaling domain, and a polynucleotide encoding the human CD3 ζ intracellular signaling domain;
human BCMA: Uniprot ID: Q02223. BCMA is an effective target for treating multiple myeloma.

The antigen binding region targeting human BCMA was an scFv (anti-BCMA-scFv) of anti-human BCMA, the amino acid sequence of the anti-BCMA-scFv is as set forth in SEQ ID NO: 72; the amino acid sequences of the HCDR1-3 regions of the anti-BCMA-scFv are respectively as set forth in SEQ ID NOs: 73-75; and the amino acid sequences of the LCDR1-3 regions of the anti-BCMA-scFv are respectively as set forth in SEQ ID NOs: 76-78.

### 2. Packaging of Multiple Groups of Lentiviral Vectors Comprising the CAR-BCMA Gene

With reference to the method for packaging the LVV-V5-CAR19, multiple groups of lentiviral vectors LVV-V22-CARBCMA, LVV-V23-CARBCMA and LVV-V24-CARBCMA were packaged in the same batch:
the LVV-V22-CARBCMA: (a) the viral envelope comprised (i) a T cell activation primary signaling molecule, that is, the membrane-expressed UCHT1-scFv, and a T cell activation secondary signaling molecule, that is, the membrane-expressed 15E8-scFv; and (ii) the mutant VSV-G1; and (b) comprised the CAR-BCMA gene;
the LVV-V23-CARBCMA: (a) the viral envelope comprised (i) a membrane-expressed anti-CD7 antibody, that is, the membrane-expressed TH69-scFv, and (ii) the mutant VSV-G1; and (b) comprised the CAR-BCMA gene; and
the LVV-V24-CARBCMA: (a) the viral envelope comprised (i) a T cell activation primary signaling molecule, that is, the membrane-expressed UCHT1-scFv, and (ii) the mutant VSV-G1; and (b) comprised the CAR-BCMA gene.

### 3. Transduction of Human Non-Activated PBMCs, and Detection of Expression Efficiency of CAR-BCMA (MOI = 1)

At Day 0, the LVV-V22-CARBCMA, the LVV-V23-CARBCMA and the LVV-V24-CARBCMA were respectively added to the culture systems of the non-activated PBMCs of Donor 1 in each group at MOI = 1; and at Day 2, the efficiency of expression of CAR-BCMA in the CD3⁺ T cells in the PBMCs of each group was detected by flow cytometry, and the results are as shown in FIG. 17.

It can be seen from FIG. 17 that: the transduction efficiency of the LVV-V22-CARBCMA transducing the CD3⁺ T cells in the human non-activated PBMCs and delivering the CAR-BCMA gene (about 19.4%) is significantly superior to those of the LVV-V23-CARBCMA (about 15.32%) and the LVV-V24-CARBCMA (about 9.36%).

Flow cytometry detection antibody: Anti-G4Slinker, brand: HYS-BIO, catalog number: #GS-ARPE100.

### 4. Detection of Killing Efficiency of BCMA-CAR-T Cells Prepared by Transduction of PBMCs by Each Group of Lentiviral Vectors

At Day 0, 4 groups of 1×10⁶ human non-activated PBMCs of Donor 1 were respectively mixed with BCMA⁺U266 cells (human multiple myeloma cells) at an effector-to-target ratio E:T = 1:1 and added to a culture medium (1640 + 10% FBS); the LVV-V22-CARBCMA, the LVV-V23-CARBCMA and the LVV-V24-CARBCMA were respectively added to 3 groups of mixed cell culture media at MOI = 1; and the control group did not comprise any lentiviral vector, and only comprised the mixed cells.

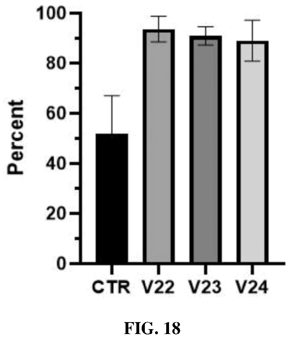

At Day 5, the number of mixed cells of each group was counted using a cell counter, and the expression of BCMA in the mixed cells of each group was detected by flow cytometry to calculate the killing efficiency, and the results are as shown in FIG. 18.

It can be seen from FIG. 18 that: on Day 5, compared with the control group, BCMA-CAR-T cells Prepared by the LVV-V22-CARBCMA, the LVV-V23-CARBCMA and the LVV-V24-CARBCMA transducing the human non-activated PBMCs can all effectively kill the BCMA⁺U266 cells.

## Claims

1. A viral particle, **characterized in that**
(a) a surface of the viral particle comprises a T cell activation primary signaling molecule and a T cell activation secondary signaling molecule; and
(b) the surface of the viral particle comprises a glycoprotein, wherein the glycoprotein is subjected to a first mutation, so that an ability of the glycoprotein to bind a glycoprotein receptor is weakened or lost with respect to an ability before the first mutation.

2. The viral particle according to claim 1, wherein the viral particle targetedly activates and transduces non-activated T cells.

3. The viral particle according to claim 1 or 2, wherein the viral particle is a lentiviral vector (LVV) or a retroviral vector (RVV).

4. The viral particle according to any one of claims 1 to 3, wherein the T cell activation primary signaling molecule binds to a TCR/CD3 complex or a TCR/CD3 complex subunit; and the TCR/CD3 complex subunit is selected from CD3ε, CD3γ, CD3δ, TCRα and TCRβ.

5. The viral particle according to claim 4, wherein the TCR/CD3 complex or TCR/CD3 complex subunit is a human TCR/CD3 complex or TCR/CD3 complex subunit.

6. The viral particle according to claim 4 or 5, wherein the T cell activation primary signaling molecule comprises an anti-CD3 antibody or an antigen-binding fragment thereof.

7. The viral particle according to claim 6, wherein the anti-CD3 antibody is an scFv derived from UCHT1 (UCHT1-scFv), an amino acid sequence of the UCHT1-scFv is as set forth in SEQ ID NO: 9; and amino acid sequences of HCDR1-3 regions of the UCHT1-scFv are as set forth in SEQ ID NOs: 81-83, respectively, and amino acid sequences of LCDR1-3 regions of the UCHT1-scFv are as set forth in SEQ ID NOs: 84-86, respectively.

8. The viral particle according to any one of claims 1 to 7, wherein the T cell activation secondary signaling molecule binds to CD28.

9. The viral particle according to claim 8, wherein the CD28 is human CD28.

10. The viral particle according to claim 8 or 9, wherein the T cell activation secondary signaling molecule is at least one selected from the group consisting of an anti-CD28 antibody or an antigen-binding fragment thereof and a CD28 ligand or a receptor-binding fragment thereof.

11. The viral particle according to claim 10, wherein the CD28 ligand or receptor-binding fragment thereof comprises CD80 or a receptor-binding fragment thereof and CD86 or a receptor-binding fragment thereof.

12. The viral particle according to claim 11, wherein the CD80 receptor-binding fragment is a CD80 extracellular domain; and the CD86 receptor-binding fragment is a CD86 extracellular domain.

13. The viral particle according to claim 8, wherein the T cell activation secondary signaling molecule comprises an anti-CD28 antibody or an antigen-binding fragment thereof.

14. The viral particle according to claim 13, wherein the anti-CD28 antibody or antigen-binding fragment thereof is an scFv derived from 15E8 (15E8-scFv), an amino acid sequence of the 15E8-scFv is as set forth in SEQ ID NO: 13; and amino acid sequences of HCDR1-3 regions of the 15E8-scFv are as set forth in SEQ ID NOs: 87-89, respectively, and amino acid sequences of LCDR1-3 regions of the 15E8-scFv are as set forth in SEQ ID NOs: 90-92, respectively.

15. The viral particle according to any one of claims 8 to 14, wherein the T cell activation secondary signaling molecule further comprises at least one ligand or receptor-binding fragment thereof selected from the group consisting of an ICOS ligand (ICOSL) or a receptor-binding fragment thereof, a 4-1BB ligand (4-1BBL) or a receptor-binding fragment thereof, and an OX40 ligand (OX40L) or a receptor-binding fragment thereof.

16. The viral particle according to any one of claims 1 to 15, wherein the T cell activation primary signaling molecule and/or the T cell activation secondary signaling molecule is directly or indirectly linked to a transmembrane polypeptide and displayed on the surface of the viral particle.

17. The viral particle according to claim 16, wherein the transmembrane polypeptide is selected from a transmembrane domain of following proteins:
CD2, CD3, CD4, CD5, CD7, CD8, CD8α, CD8β, CD9, CD16, CD22, CD27, CD28, CD28H, CD30, CD33, CD37, CD40, CD45, CD64, CD80, CD86, CD84, CD154, CD166, CD226, CD244, 4-1BB, OX40, ICOS, ICAM-1, CTLA-4, PD-1, LAG-3, GITR, HVEM, DAP10, DAP12, TIM-1, LIGHT, ICOS, OX40, 2B4, BTLA, DNAM-1, DR3, FcERIγ, IL7, IL12, IL15, SLAM, KIR2DL4, KIR2DS1, KIR2DS2, NKG2C, NKG2D and CS1; and
preferably, the transmembrane polypeptide is a CD8α transmembrane domain.

18. The viral particle according to claim 16 or 17, wherein the T cell activation primary signaling molecule and/or the T cell activation secondary signaling molecule is indirectly linked to the transmembrane polypeptide through a linker domain and displayed on the surface of the viral particle;
preferably, the linker domain is selected from:
(a) an immunoglobulin hinge region, wherein the immunoglobulin hinge region is selected from a wild-type or modified IgG1, IgG2, IgG3, IgG4, IgA and IgD hinge regions;
(b) a hinge region, wherein the hinge region is selected from a wild-type or modified hinge region of following proteins: CD28, CD7, CD8, CD8α, CD8β, CD3, CD45, CD4, CD5, CD8, CD9, CD16, CD22, CD33, CD37, CD134, CD137, ICOS and CD154;
(c) all or a portion of an Fc domain, wherein the Fc domain is one or more selected from the group consisting of a CH1 domain, a CH2 domain and a CH3 domain;
(d) a stalk region of type II C-lectin, wherein the type II C-lectin is selected from stalk regions of CD23, CD69, CD72, CD94, NKG2A and NKG2D; and
(e) a flexible linker peptide; and
more preferably, the linker domain is a CD8α hinge region.

19. The viral particle according to any one of claims 16 to 18, wherein the T cell activation primary signaling molecule comprises, from N-terminus to C-terminus, the anti-CD3 antibody or antigen-binding fragment thereof, a CD8α hinge region and a CD8α transmembrane domain; and preferably, the anti-CD3 antibody is the UCHT1-scFv.

20. The viral particle according to any one of claims 16 to 19, wherein the T cell activation secondary signaling molecule comprises, from N-terminus to C-terminus, an anti-CD28 antibody or antigen-binding fragment thereof, a CD8α hinge region and a CD8α transmembrane domain; and preferably, the anti-CD28 antibody is the 15E8-scFv.

21. The viral particle according to any one of claims 1 to 19, wherein the T cell activation secondary signaling molecule is at least one selected from the group consisting of human CD80 or a receptor-binding fragment thereof and human CD86 or a receptor-binding fragment thereof;
preferably, an amino acid sequence of the human CD80 has at least about 85%, 86%, 87%, 88%, 89%, 90%, 91%, 92%, 93%, 94%, 95%, 96%, 97%, 98%, 99% or 100% sequence identity to SEQ ID NO: 93; and
preferably, an amino acid sequence of the human CD86 has at least about 85%, 86%, 87%, 88%, 89%, 90%, 91%, 92%, 93%, 94%, 95%, 96%, 97%, 98%, 99% or 100% sequence identity to SEQ ID NO: 95.

22. The viral particle according to claim 21, wherein the receptor-binding fragment of the human CD80 comprises an extracellular domain and a transmembrane domain of the human CD80; and the receptor-binding fragment of the human CD86 comprises an extracellular domain and a transmembrane domain of the human CD86;
preferably, an amino acid sequence of the extracellular domain and the transmembrane domain of the human CD80 has at least about 85%, 86%, 87%, 88%, 89%, 90%, 91%, 92%, 93%, 94%, 95%, 96%, 97%, 98%, 99% or 100% sequence identity to SEQ ID NO: 94; and
preferably, an amino acid sequence of the extracellular domain and the transmembrane domain of the human CD86 has at least about 85%, 86%, 87%, 88%, 89%, 90%, 91%, 92%, 93%, 94%, 95%, 96%, 97%, 98%, 99% or 100% sequence identity to SEQ ID NO: 79.

23. The viral particle according to claim 16, wherein the transmembrane polypeptide is the glycoprotein, and the glycoprotein is directly or indirectly linked to the T cell activation primary signaling molecule and/or the T cell activation secondary signaling molecule.

24. The viral particle according to claim 23, wherein the glycoprotein is indirectly linked to the T cell activation primary signaling molecule and/or the T cell activation secondary signaling molecule through a first polypeptide linker.

25. The viral particle according to claim 23 or 24, wherein the T cell activation primary signaling molecule is directly or indirectly linked to the T cell activation secondary signaling molecule.

26. The viral particle according to claim 25, wherein the T cell activation primary signaling molecule is indirectly linked to the T cell activation secondary signaling molecule through a second polypeptide linker.

27. The viral particle according to any one of claims 24 to 26, wherein the polypeptide linker is a flexible linker peptide.

28. The viral particle according to claim 27, wherein the flexible linker peptide is selected from a (G₄S)ₙ linker peptide, linker 1: GSTSGSGKPGSGEGSTKG (SEQ ID NO: 97) and linker 2: GSSGGSGGGGSGGGGSGGGGSSG (SEQ ID NO: 98), wherein n = 1 to 4.

29. The viral particle according to any one of claims 24 to 28, wherein
(a) the glycoprotein is indirectly linked to the anti-CD3 antibody or antigen-binding fragment thereof through a (G₄S)ₙ linker peptide, and the anti-CD3 antibody or antigen-binding fragment thereof is indirectly linked to the anti-CD28 antibody or antigen-binding fragment thereof, the human CD86 extracellular domain or the human CD80 extracellular domain through the (G₄S)ₙ linker peptide; and/or
(b) the glycoprotein is indirectly linked to the anti-CD28 antibody or antigen-binding fragment thereof, the human CD86 extracellular domain or the human CD80 extracellular domain through the (G₄S)ₙ linker peptide, and the anti-CD28 antibody or antigen-binding fragment thereof, the human CD86 extracellular domain or the human CD80 extracellular domain is indirectly linked to the anti-CD3 antibody or antigen-binding fragment thereof through the (G₄S)ₙ linker peptide;
preferably, n = 3;
preferably, the anti-CD3 antibody or antigen-binding fragment thereof is the UCHT1-scFv; and
preferably, the anti-CD28 antibody or antigen-binding fragment thereof is the 15E8-scFv.

30. The viral particle according to any one of claims 1 to 29, wherein the glycoprotein is at least one selected from the group consisting of an envelope glycoprotein of a vesicular stomatitis virus strain and a variant thereof, an envelope glycoprotein of baboon endogenous retrovirus BaEV and a variant thereof, an envelope glycoprotein RD114 of feline endogenous retrovirus and a variant thereof, and an envelope glycoprotein GALV of gibbon ape leukemia virus and a variant thereof.

31. The viral particle according to claim 30, wherein the glycoprotein is at least one selected from the group consisting of the envelope glycoprotein of a vesicular stomatitis virus strain and the variant thereof; and
preferably, the envelope glycoprotein of the vesicular stomatitis virus strain and the variant thereof comprise envelope glycoproteins and variants thereof: an envelope glycoprotein of a vesicular stomatitis virus Indiana strain and a variant thereof, an envelope glycoprotein of a vesicular stomatitis virus Cocal strain and a variant thereof, an envelope glycoprotein of a vesicular stomatitis virus Maraba strain and a variant thereof, an envelope glycoprotein of a vesicular stomatitis virus Morreton strain and a variant thereof, an envelope glycoprotein of a vesicular stomatitis virus Alagoas strain and a variant thereof, an envelope glycoprotein of a vesicular stomatitis virus New Jersey strain and a variant thereof, an envelope glycoprotein of a vesicular stomatitis virus Carajas strain and a variant thereof, an envelope glycoprotein of a vesicular stomatitis virus Chandipura strain and a variant thereof, an envelope glycoprotein of a vesicular stomatitis virus Eptesicus strain and a variant thereof, an envelope glycoprotein of a vesicular stomatitis virus Isfahan strain and a variant thereof, an envelope glycoprotein of a vesicular stomatitis virus Jurona strain and a variant thereof, an envelope glycoprotein of a vesicular stomatitis virus Malpais strain and a variant thereof, an envelope glycoprotein of a vesicular stomatitis virus Perinet strain and a variant thereof, an envelope glycoprotein of a vesicular stomatitis virus Piry strain and a variant thereof, an envelope glycoprotein of a vesicular stomatitis virus Radi strain and a variant thereof, an envelope glycoprotein of a vesicular stomatitis virus Rhinolopus strain and a variant thereof, and an envelope glycoprotein of a vesicular stomatitis virus Yug Bogdanovac strain and a variant thereof.

32. The viral particle according to claim 31, wherein the glycoprotein is the envelope glycoprotein of the vesicular stomatitis virus Indiana strain or Cocal strain or the variant thereof, and the glycoprotein receptor is a low density lipoprotein receptor LDL-R; and the extracellular domain of the glycoprotein comprises an amino acid sequence as set forth in SEQ ID NO: 1 or SEQ ID NO: 2, or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 1 or SEQ ID NO: 2.

33. The viral particle according to claim 32, wherein the first mutation comprises an amino acid sequence comprising a mutation of at least one of following amino acids:
(a) in SEQ ID NO: 1 or SEQ ID NO: 2, substitution or deletion of an amino acid at site 8, substitution or deletion of an amino acid at site 9, substitution or deletion of an amino acid at site 10, substitution or deletion of an amino acid at site 47, substitution or deletion of an amino acid at site 50, substitution or deletion of an amino acid at site 51, substitution or deletion of an amino acid at site 183, substitution or deletion of an amino acid at site 179, substitution or deletion of an amino acid at site 180, substitution or deletion of an amino acid at site 182, substitution or deletion of an amino acid at site 184, substitution or deletion of an amino acid at site 209, substitution or deletion of an amino acid at site 347, substitution or deletion of an amino acid at site 350, substitution or deletion of an amino acid at site 352, substitution or deletion of an amino acid at site 353, substitution of an amino acid at site 354, deletion of amino acids at sites 1-18, deletion of amino acids at sites 19-36, deletion of amino acids at sites 37-51, deletion of amino acids at sites 314-384, deletion of amino acids at sites 321-374, deletion of amino acids at sites 331-364, deletion of amino acids at sites 344-354, and deletion of amino acids at sites 345-353; and
(b) after optimal global alignment with SEQ ID NO: 1 or SEQ ID NO: 2, substitution or deletion of an amino acid at a site corresponding to site 8, substitution or deletion of an amino acid corresponding to site 9, substitution or deletion of an amino acid corresponding to site 10, substitution or deletion of an amino acid corresponding to site 47, substitution or deletion of an amino acid corresponding to site 50, substitution or deletion of an amino acid corresponding to site 51, substitution or deletion of an amino acid corresponding to site 183, substitution or deletion of an amino acid corresponding to site 179, substitution or deletion of an amino acid corresponding to site 180, substitution or deletion of an amino acid corresponding to site 182, substitution or deletion of an amino acid corresponding to site 184, substitution or deletion of an amino acid corresponding to site 209, substitution or deletion of an amino acid corresponding to site 347, substitution or deletion of an amino acid corresponding to site 350, substitution or deletion of an amino acid corresponding to site 352, substitution or deletion of an amino acid at site 353, substitution of an amino acid corresponding to site 354, deletion of amino acids corresponding to sites 1-18, deletion of amino acids corresponding to sites 19-36, deletion of amino acids corresponding to sites 37-51, deletion of amino acids corresponding to sites 314-384, deletion of amino acids corresponding to sites 321-374, deletion of amino acids corresponding to sites 331-364, deletion of amino acids corresponding to sites 344-354, and deletion of amino acids corresponding to sites 345-353 of SEQ ID NO: 1 or SEQ ID NO: 2.

34. The viral particle according to claim 33, wherein the first mutation comprises an amino acid sequence comprising a mutation of at least one of following amino acids:
(a) in SEQ ID NO: 1 or SEQ ID NO: 2, deletion of amino acids at sites 331 to 364, deletion of amino acids at sites 344-354, substitution of K47, deletion of K47, and substitution of R354; and
(b) after optimal global alignment with SEQ ID NO: 1 or SEQ ID NO: 2, deletion of amino acids at sites corresponding to sites 331 to 364, deletion of amino acids corresponding to sites 344-354, substitution corresponding to K47, deletion corresponding to K47, and substitution corresponding to R354 of SEQ ID NO: 1 or SEQ ID NO: 2; and
preferably, the first mutation comprises an amino acid sequence comprising a mutation of at least one of following amino acids:
(a) in SEQ ID NO: 1 or SEQ ID NO: 2, deletion of amino acids at sites 331 to 364, deletion of amino acids at sites 344-354, K47Q, R354Q, and deletion of K47; and
(b) after optimal global alignment with SEQ ID NO: 1 or SEQ ID NO: 2, deletion of amino acids at sites corresponding to sites 331 to 364, deletion of amino acids corresponding to sites 344-354, Q substitution corresponding to K47, Q substitution corresponding to R354, and deletion corresponding to K47 of SEQ ID NO: 1 or SEQ ID NO: 2.

35. The viral particle according to any one of claims 32 to 34, wherein the first mutation comprises an amino acid sequence comprising a following amino acid mutation:
(a) deletion of K47 in SEQ ID NO: 1 or SEQ ID NO: 2; and
(b) after optimal global alignment with SEQ ID NO: 1 or SEQ ID NO: 2, deletion corresponding to K47 of SEQ ID NO: 1 or SEQ ID NO: 2.

36. The viral particle according to claim 32 or 33, wherein the first mutation comprises an amino acid sequence comprising a mutation of at least one of following amino acids:
(a) substitution of K47, deletion of K47, substitution of I182, substitution of R354, and substitution of Y209 in SEQ ID NO: 1;
(b) after optimal global alignment with SEQ ID NO: 1, substitution corresponding to K47, deletion corresponding to K47, substitution corresponding to I182, substitution corresponding to R354, and substitution corresponding to Y209 of SEQ ID NO: 1;
(c) substitution of K47, deletion of K47, substitution of V182, substitution of R354, and substitution of Y209 in SEQ ID NO: 2; and
(d) after optimal global alignment with SEQ ID NO: 2, substitution corresponding to K47, deletion corresponding to K47, substitution corresponding to V182, substitution corresponding to R354, and substitution corresponding to Y209 of SEQ ID NO: 2; and
preferably, the first mutation comprises an amino acid sequence comprising a mutation of at least one of following amino acids:
(a) K47Q or K47A, deletion of K47, I182E or I182D, R354Q or R354A, and Y209Q in SEQ ID NO: 1;
(b) after optimal global alignment with SEQ ID NO: 1, Q or A substitution corresponding to K47, deletion corresponding to K47, E or D substitution corresponding to I182, Q or A substitution corresponding to R354, and Q substitution corresponding to Y209 of SEQ ID NO: 1;
(c) K47Q or K47A, deletion of K47, V182E or V182D, R354Q or R354A, and Y209Q in SEQ ID NO: 2; and
(d) after optimal global alignment with SEQ ID NO: 2, Q or A substitution corresponding to K47, deletion corresponding to K47, E or D substitution corresponding to V182, Q or A substitution corresponding to R354, and Q substitution corresponding to Y209 of SEQ ID NO: 2.

37. The viral particle according to any one of claims 1 to 36, wherein an extracellular domain of the glycoprotein comprises an amino acid sequence as set forth in SEQ ID NO: 3, SEQ ID NO: 4, SEQ ID NO: 23 or SEQ ID NO: 24.

38. The viral particle according to any one of claims 1 to 37, wherein the glycoprotein is further subjected to a second mutation, so that an ability of the glycoprotein to antagonize complement-mediated inactivation is enhanced with respect to an ability before the second mutation, or the glycoprotein is not subjected to complement-mediated inactivation.

39. The viral particle according to claim 38, wherein the glycoprotein is an envelope glycoprotein of a vesicular stomatitis virus Indiana strain or Cocal strain or a variant thereof, and an extracellular domain of the glycoprotein comprises an amino acid sequence as set forth in SEQ ID NO: 1 or SEQ ID NO: 2, or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 1 or SEQ ID NO: 2.

40. The viral particle according to claim 39, wherein the second mutation comprises an amino acid sequence comprising a mutation of at least one of following amino acids:
(a) an amino acid at site 214 of SEQ ID NO: 1 or SEQ ID NO: 2;
(b) after optimal global alignment with SEQ ID NO: 1 or SEQ ID NO: 2, an amino acid at a site corresponding to site 214 of SEQ ID NO: 1 or SEQ ID NO: 2;
(c) an amino acid at site 352 of SEQ ID NO: 1 or SEQ ID NO: 2;
(d) after optimal global alignment with SEQ ID NO: 1 or SEQ ID NO: 2, an amino acid at a site corresponding to site 352 of SEQ ID NO: 1 or SEQ ID NO: 2;
(e) an amino acid at site 50 of SEQ ID NO: 1 or SEQ ID NO: 2;
(f) after optimal global alignment with SEQ ID NO: 1 or SEQ ID NO: 2, an amino acid at a site corresponding to site 50 of SEQ ID NO: 1 or SEQ ID NO: 2;
(g) an amino acid at site 146 of SEQ ID NO: 1 or SEQ ID NO: 2; and
(h) after optimal global alignment with SEQ ID NO: 1 or SEQ ID NO: 2, an amino acid at a site corresponding to site 146 of SEQ ID NO: 1 or SEQ ID NO: 2;
preferably, the amino acid mutation is at least one selected from the group consisting of deletion, insertion and substitution of an amino acid; and
more preferably, the second mutation comprises an amino acid sequence comprising a substitution of at least one of following amino acids:
(a) the amino acid at site 214 of SEQ ID NO: 1 or SEQ ID NO: 2;
(b) after optimal global alignment with SEQ ID NO: 1 or SEQ ID NO: 2, the amino acid at the site corresponding to site 214 of SEQ ID NO: 1 or SEQ ID NO: 2;
(c) the amino acid at site 352 of SEQ ID NO: 1 or SEQ ID NO: 2;
(d) after optimal global alignment with SEQ ID NO: 1 or SEQ ID NO: 2, the amino acid at the site corresponding to site 352 of SEQ ID NO: 1 or SEQ ID NO: 2;
(e) the amino acid at site 50 of SEQ ID NO: 1 or SEQ ID NO: 2;
(f) after optimal global alignment with SEQ ID NO: 1 or SEQ ID NO: 2, the amino acid at the site corresponding to site 50 of SEQ ID NO: 1 or SEQ ID NO: 2;
(g) the amino acid at site 146 of SEQ ID NO: 1 or SEQ ID NO: 2; and
(h) after optimal global alignment with SEQ ID NO: 1 or SEQ ID NO: 2, the amino acid at the site corresponding to site 146 of SEQ ID NO: 1 or SEQ ID NO: 2.

41. The viral particle according to claim 40, wherein the second mutation comprises the amino acid sequence as set forth in SEQ ID NO: 1 or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 1 comprising at least one of following site mutations:
(a) substitution of T214, substitution of T352, substitution of K50, and substitution of S146 in SEQ ID NO: 1; and
(b) after optimal global alignment with SEQ ID NO: 1, substitution corresponding to T214, substitution corresponding to T352, substitution corresponding to K50, and substitution corresponding to S146 of SEQ ID NO: 1; and
preferably, the second mutation comprises an amino acid sequence comprising at least one of following site mutations:
(a) T214N, T352A, K50T and S146T in SEQ ID NO: 1; and
(b) after optimal global alignment with SEQ ID NO: 1, N substitution corresponding to T214, A substitution corresponding to T352, T substitution corresponding to K50 and T substitution corresponding to S146 of SEQ ID NO: 1.

42. The viral particle according to claim 41, wherein the second mutation comprises an amino acid sequence comprising any one of following combinations of site mutations:
(a) (1) substitution of T214 and T352 in SEQ ID NO: 1; or (2) substitution of T214, T352, K50 and S146 in SEQ ID NO: 1; and
(b) after optimal global alignment with SEQ ID NO: 1, (1) substitution at sites corresponding to T214 and T352 of SEQ ID NO: 1; or (2) substitution at sites corresponding to T214, T352, K50 and S146 of SEQ ID NO: 1; and
preferably, the second mutation comprises an amino acid sequence comprising any one of following combinations of site mutations:
(a) (1) T214N and T352A in SEQ ID NO: 1; or (2) T214N, T352A, K50T and S146T in SEQ ID NO:1; and
(b) after optimal global alignment with SEQ ID NO: 1, (1) N substitution at a site corresponding to T214 and A substitution at a site corresponding to T352 of SEQ ID NO:1; or (2) N substitution at a site corresponding to T214, A substitution at a site corresponding to T352, T substitution at a site corresponding to K50 and T substitution at a site corresponding to S146 of SEQ ID NO: 1.

43. The viral particle according to claim 40, wherein the second mutation comprises the amino acid sequence as set forth in SEQ ID NO: 2 or an amino acid sequence having at least about 50%, about 60%, about 70%, about 80%, about 90%, about 91%, about 92%, about 93%, about 94%, about 95%, about 96%, about 97%, about 98% or about 99% sequence identity to the amino acid sequence as set forth in SEQ ID NO: 2 comprising at least one of following site mutations:
(a) substitution of K214, substitution of T352, substitution of K50, and substitution of S146 in SEQ ID NO: 2; and
(b) after optimal global alignment with SEQ ID NO: 2, substitution corresponding to K214, substitution corresponding to T352, substitution corresponding to K50, and substitution corresponding to S146 of SEQ ID NO: 2; and
preferably, the second mutation comprises an amino acid sequence comprising at least one of following site mutations:
(a) K214N, T352A, K50T and S146T in SEQ ID NO: 2; and
(b) after optimal global alignment with SEQ ID NO: 2, N substitution at a site corresponding to K214, A substitution at a site corresponding to T352, T substitution at a site corresponding to K50 and T substitution at a site corresponding to S146 of SEQ ID NO: 2.

44. The viral particle according to claim 43, wherein the second mutation comprises an amino acid sequence comprising any one of following combinations of site mutations:
(a) (1) substitution of K214 and T352 in SEQ ID NO: 2; or (2) substitution of K214, T352, K50 and S146 in SEQ ID NO: 2; and
(b) after optimal global alignment with SEQ ID NO: 2, (1) substitution at sites corresponding to K214 and T352 of SEQ ID NO: 2; or (2) substitution at sites corresponding to K214, T352, K50 and S146 of SEQ ID NO: 2; and
preferably, the second mutation comprises an amino acid sequence comprising any one of following combinations of site mutations:
(a) (1) K214N and T352A in SEQ ID NO: 2; or (2) K214N, T352A, K50T and S146T in SEQ ID NO: 2; and
(b) after optimal global alignment with SEQ ID NO: 2, (1) N substitution at a site corresponding to K214, and A substitution at a site corresponding to T352 of SEQ ID NO: 2; or (2) N substitution at a site corresponding to K214, A substitution at a site corresponding to T352, T substitution at a site corresponding to K50 and T substitution at a site corresponding to S146 of SEQ ID NO: 2.

45. The viral particle according to any one of claims 38 to 44, wherein the extracellular domain of the glycoprotein comprises an amino acid sequence as set forth in SEQ ID NO: 5, SEQ ID NO: 6, SEQ ID NO: 7, SEQ ID NO: 21, SEQ ID NO: 25, SEQ ID NO: 26, SEQ ID NO: 27 or SEQ ID NO: 28.

46. The viral particle according to any one of claims 1 to 45, wherein the viral particle comprises an exogenous payload gene.

47. The viral particle according to claim 46, wherein the exogenous payload gene encodes a therapeutic protein or polypeptide, wherein the therapeutic protein or polypeptide is at least one selected from the group consisting of antibody-based drugs, chimeric antigen receptors, T cell receptors, cytokine receptors, cytokines, Fc fusion proteins, anticoagulants, blood factors, bone morphogenetic proteins, engineered protein scaffolds, enzymes, growth factors, hormones, interferons, interleukins and thrombolytic agents.

48. The viral particle according to claim 47, wherein the exogenous payload gene encodes the chimeric antigen receptors (CAR).

49. The viral particle according to claim 48, wherein the chimeric antigen receptor comprises an extracellular antigen-binding domain, a transmembrane domain and an intracellular signaling domain.

50. The viral particle according to claim 49, wherein the extracellular antigen-binding domain binds to a disease-related antigen.

51. The viral particle according to claim 50, wherein the antigen is at least one selected from the group consisting of:
TSHR, CD2, CD3, CD4, CD5, CD7, CD8, CD14, CD15, CD19, CD20, CD21, CD23, CD24, CD25, CD28, CD37, CD38, CD40, CD40L, CD44, CD46, CD47, CD52, CD54, CD56, CD70, CD73, CD80, CD97, CD123, CD22, CD126, CD138, DR4, DR5, TAC, TEM1/CD248, VEGF, GUCY2C, EGP40, EGP-2, EGP-4, CDL33, IFNAR1, DLL3, kappa light chain, TIM3, tEGFR, IL-22Ra, IL-2, ErbB3, ErbB4, MUC16, MAGE-A3, MAGE-A6, NKG2DL, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin (MSLN), IL-1Ra, PSCA, PRSS21, VEGFR2, Lewis-Y, CD24, PDGFR-β, SSEA-4, AFP, Folate receptor α, Her2/neu/ERBB2, MUC1, EGFR, CS1, CD138, NCAM, Claudin18.2, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6/E7, MAGE-A4, MART-1, WT-1, ETV6-AML, sperm protein 17, XAGE1, Tie2, MAD-CT-1, MAD-CT-2, Fos-related antigen 1, p53, p53 mutant, prostate-specific protein, survivin and telomerase, PCTA-1/Galectin 8, MelanA/MARTI, Ras mutant, hTERT, sarcoma translocation breakpoint, ML-IAP, TMPRSS2 ETS fusion gene/ERG, NA17, PAX3, androgen receptor, CyclinB1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL and NKG2D.

52. The viral particle according to claim 51, wherein the antigen is CD19.

53. The viral particle according to claim 51, wherein the antigen is one or more selected from the group consisting of CD19, CD20, BCMA, CD33, HER2 and CEA.

54. The viral particle according to any one of claims 49 to 53, wherein the extracellular antigen-binding domain comprises an antibody or antigen-binding fragment thereof and/or a ligand or receptor-binding fragment thereof, wherein the antibody or antigen-binding fragment thereof is at least one selected from the group consisting of an immunoglobulin (full-length antibody), a half-antibody, Fab, Fab', F(ab')2, an Fv fragment, a single-chain fragment variable (scFv), a disulfide-stabilized antibody (dsFv), a heavy chain variable region (VH) or a light chain variable region (VL) of an antibody, an Fd fragment consisting of a VH and a CH1 domain, a linear antibody and a single-domain antibody (nanobody).

55. The viral particle according to any one of claims 49 to 54, wherein the transmembrane domain is derived from a transmembrane domain of at least one of following proteins:
CD2, CD3, TCR, CD4, CD5, CD7, CD8, CD8α, CD8β, CD9, CD16, CD22, CD27, CD28, CD28H, CD30, CD33, CD37, CD40, CD45, CD64, CD80, CD84, CD154, CD166, CD226, CD244, 4-1BB, OX40, ICOS, ICAM-1, CTLA-4, PD-1, LAG-3, GITR, HVEM, DAP10, DAP12, TIM-1, LIGHT, ICOS, OX40, 2B4, BTLA, DNAM-1, DR3, FcERIγ, IL7, IL12, IL15, SLAM, KIR2DL4, KIR2DS1, KIR2DS2, NKG2C, NKG2D and CS1; and
preferably, the transmembrane domain is derived from the transmembrane domain of CD8α.

56. The viral particle according to any one of claims 49 to 55, wherein the intracellular signaling domain is derived from an intracellular signaling domain of at least one of following proteins:
CD3ε, CD3γ, CD3δ, CD3ζ, CD79a, CD79b, FcεRIγ, FcεRβ, FcγRIIa, bovine leukemia virus gp30, Epstein-Barr virus (EBV) LMP2A, simian immunodeficiency virus PBj14 Nef, DAP10, DAP12 and an intracellular signaling domain of other proteins comprising at least one ITAM in the intracellular signaling domain; and
preferably, the intracellular signaling domain is derived from the intracellular signaling domain of CD3ζ.

57. The viral particle according to any one of claims 49 to 56, wherein the chimeric antigen receptor further comprises a hinge region; and the hinge region sequentially links the extracellular antigen-binding domain and the transmembrane domain;
preferably, the hinge region is derived from a hinge region of at least one of following proteins:
CD28, CD8, CD8α, CD8β, CD3, CD45, Ig4, CD4, CD5, CD8, CD9, CD16, CD22, CD33, CD37, CD134, CD137, ICOS and CD154; and
more preferably, the hinge region is derived from the hinge region of CD8α.

58. The viral particle according to any one of claims 49 to 57, wherein the chimeric antigen receptor further comprises a costimulatory signaling domain;
preferably, the costimulatory signaling domain is derived from a costimulatory signaling domain of at least one of following proteins:
CD28, 4-1BB, CD27, CD2, CD7, CD8, CD8α, CD8β, OX40, CD226, DR3, SLAM, CDS, ICAM-1, NKG2D, NKG2C, B7-H3, 2B4, FcαR1γ, BTLA, GITR, HVEM, DAP10, DAP12, CD30, CD40, CD40L, TIM1, PD-1, LFA-1, LIGHT, JAML, CD244, CD100, ICOS, CD40 and MyD88; and
more preferably, the costimulatory signaling domain is derived from the costimulatory signaling domain of 4-1BB.

59. The viral particle according to any one of claims 49 to 58, wherein the CAR further comprises a signal peptide; and
preferably, the signal peptide is derived from a CD8α signal peptide.

60. The viral particle according to any one of claims 1 to 59, wherein an efficiency of transduction of non-activated T cells by the viral particle is improved relative to a control vector whose surface does not comprise the T cell activation primary signaling molecule and the T cell activation secondary signaling molecule.

61. The viral particle according to any one of claims 46 to 60, wherein after the viral particle contacts a non-activated T cell, an efficiency of expression of the exogenous payload gene in the T cell is improved relative to a control vector whose surface does not comprise the T cell activation primary signaling molecule and the T cell activation secondary signaling molecule.

62. The viral particle according to any one of claims 48 to 61, wherein after the viral particle contacts a non-activated T cell, an efficiency of membrane surface expression of the chimeric antigen receptor in the T cell is improved relative to a control vector whose surface does not comprise the T cell activation primary signaling molecule and the T cell activation secondary signaling molecule.

63. The viral particle according to any one of claims 48 to 62, wherein after the viral particle contacts a non-activated T cell, a killing efficiency of prepared CAR-T cells is enhanced relative to a control vector whose surface does not comprise the T cell activation primary signaling molecule and the T cell activation secondary signaling molecule.

64. The viral particle according to any one of claims 60 to 63, wherein the control vector comprises at least one T cell targeting molecule on a surface thereof, the T cell targeting molecule binding to a T cell endocytic receptor.

65. The viral particle according to claim 64, wherein the T cell endocytic receptor is at least one selected from the group consisting of CD5 and CD7.

66. The viral particle according to claim 65, wherein the T cell targeting molecule binds to CD7.

67. The viral particle according to claim 66, wherein the T cell targeting molecule is at least one selected from the group consisting of an anti-CD7 antibody or antigen-binding fragment thereof and a CD7 ligand or receptor-binding fragment thereof;
optionally, the anti-CD7 antibody or antigen-binding fragment thereof is an scFv derived from monoclonal antibody TH-69 (TH69-scFv); an amino acid sequence of the TH69-scFv is as set forth in SEQ ID NO: 37; and amino acid sequences of HCDR1-3 regions of the TH69-scFv are as set forth in SEQ ID NOs: 38-40, respectively, and amino acid sequences of LCDR1-3 regions of the TH69-scFv are as set forth in SEQ ID NOs: 41-43, respectively.

68. The viral particle according to any one of claims 60 to 63, wherein the control vector comprises only the T cell activation primary signaling molecule on a surface thereof, and does not comprise the T cell activation secondary signaling molecule;
optionally, the T cell activation primary signaling molecule binds to a TCR/CD3 complex or a TCR/CD3 complex subunit; the TCR/CD3 complex subunit is selected from CD3ε, CD3γ, CD3δ, TCRα and TCRβ; preferably, the T cell activation primary signaling molecule binds to a human TCR/CD3 complex or TCR/CD3 complex subunit; and more preferably, the T cell activation primary signaling molecule comprises an anti-human CD3 antibody or an antigen-binding fragment thereof.

69. An engineered T cell, **characterized in that** the engineered T cell expresses a chimeric antigen receptor, and the engineered T cell is prepared by contacting a T cell with the viral particle according to any one of claims 1 to 68 carrying a polynucleotide encoding the chimeric antigen receptor.

70. The engineered T cell according to claim 69, wherein the T cell comprises non-activated T cells and activated T cells.

71. The engineered T cell according to claim 69 or 70, wherein the contact occurs *in vivo* and/or *in vitro* in a subject; and the subject is an individual to which the engineered T cell and/or the viral particle according to any one of claims 1 to 68 carrying the polynucleotide encoding the chimeric antigen receptor is administered.

72. The engineered T cell according to claim 71, wherein the administration is at least one selected from the group consisting of oral, nasal, intravenous, intraperitoneal, intracerebral (intraparenchymal), intraventricular, intramuscular, intraocular, intra-arterial, portal vein, intralesional, sustained release system and implant device administration.

73. The engineered T cell according to any one of claims 69 to 72, wherein the T cells are non-activated T cells, and an efficiency of expression of CAR by the engineered T cell is improved; and
the improvement in the efficiency of expression is relative to an engineered T cell prepared by contacting a non-activated T cell with a control vector whose surface does not comprise the T cell activation primary signaling molecule and the T cell activation secondary signaling molecule.

74. The engineered T cell according to any one of claims 69 to 73, wherein the T cells are non-activated T cells, and a killing efficiency of the engineered T cell is improved; and
the improvement in the killing efficiency is relative to an engineered T cell prepared by contacting a non-activated T cell with a control vector whose surface does not comprise the T cell activation primary signaling molecule and the T cell activation secondary signaling molecule.

75. The engineered T cell according to claim 73 or 74, wherein the control vector comprises at least one T cell targeting molecule on a surface thereof, the T cell targeting molecule binding to a T cell endocytic receptor.

76. The engineered T cell according to claim 75, wherein the T cell endocytic receptor is at least one selected from the group consisting of CD5 and CD7.

77. The engineered T cell according to claim 76, wherein the T cell targeting molecule binds to CD7.

78. The engineered T cell according to claim 77, wherein the T cell targeting molecule is at least one selected from an anti-CD7 antibody or antigen-binding fragment thereof and a CD7 ligand or receptor-binding fragment thereof;
optionally, the anti-CD7 antibody or antigen-binding fragment thereof is an scFv derived from monoclonal antibody TH-69 (TH69-scFv); an amino acid sequence of the TH69-scFv is as set forth in SEQ ID NO: 37; and amino acid sequences of HCDR1-3 regions of the TH69-scFv are as set forth in SEQ ID NOs: 38-40, respectively, and amino acid sequences of LCDR1-3 regions of the TH69-scFv are as set forth in SEQ ID NOs: 41-43, respectively.

79. The engineered T cell according to claim 73 or 74, wherein the control vector comprises only the T cell activation primary signaling molecule on a surface thereof, and does not comprise the T cell activation secondary signaling molecule;
optionally, the T cell activation primary signaling molecule binds to a TCR/CD3 complex or a TCR/CD3 complex subunit; the TCR/CD3 complex subunit is selected from CD3ε, CD3γ, CD3δ, TCRα and TCRβ; preferably, the T cell activation primary signaling molecule binds to a human TCR/CD3 complex or TCR/CD3 complex subunit; more preferably, the T cell activation primary signaling molecule comprises an anti-human CD3 antibody or an antigen-binding fragment thereof.

80. A composition, **characterized in that** the composition comprises a pharmaceutically acceptable excipient or carrier and any one of following components: the viral particle according to any one of claims 1 to 68 and the engineered T cell according to any one of claims 69 to 79.

81. Use of the viral particle according to any one of claims 1 to 68, the engineered T cell according to any one of claims 69 to 79, or the composition according to claim 80, in a preparation of a medicament for prevention and/or treatment of a disease.

82. The use according to claim 81, wherein the disease is an autoimmune disease or cancer, the cancer comprising solid cancer and blood cancer.

83. The use according to claim 82, wherein the cancer is blood cancer.

84. The use according to claim 83, wherein the blood cancer is B lymphocyte cancer, B lymphocyte leukemia, Hodgkin lymphoma or multiple myeloma.

85. The use according to claim 83, wherein the blood cancer is one or more selected from the group consisting of non-Hodgkin lymphoma (NHL), acute B-cell lymphoblastic leukemia (B-ALL), chronic lymphocytic leukemia (CLL), large B-cell lymphoma (LBCL), transplant-ineligible LBCL, diffuse LBCL (DLBCL), high-grade B-cell lymphoma (HGBCL), primary mediastinal B-cell lymphoma (PMBCL), mantle cell lymphoma (MCL), follicular lymphoma (FL), marginal zone lymphoma (MZL), small lymphocytic lymphoma (SLL), precursor B-cell lymphoma/leukemia, Burkitt lymphoma (BL), multiple myeloma (MM), acute myeloid leukemia (AML), primary plasma cell leukemia (pPCL), peripheral T-cell lymphoma (PTCL-NHL), NK/T-cell lymphoma, anaplastic large cell lymphoma (ALCL), intestinal T-cell lymphoma, T-large granular lymphocyte leukemia (T-LGL) and germinal center T-cell lymphoma (FTCL).

86. The use according to claim 82, wherein the solid cancer is one or more selected from the group consisting of mesothelioma, pancreatic cancer, ovarian cancer, lung cancer, gastric cancer, breast cancer, colorectal cancer, bladder cancer, gastroesophageal junction cancer, biliary tract cancer and gastrointestinal cancer.

87. The use according to claim 86, wherein the cancer is one or more selected from the group consisting of CD19⁺ cancer, CD20⁺ cancer, BCMA⁺ cancer, CD33⁺ cancer, HER2⁺ cancer and CEA⁺ cancer.

88. A method for treating cancer in a subject or killing cancer cells in a subject, **characterized in that** the method comprises administering to the subject the viral particle according to any one of claims 1 to 68, the engineered T cell according to any one of claims 69 to 79, or the composition according to claim 80; and the viral particle or the composition further comprises an exogenous payload gene.

89. The method for treating cancer in a subject or killing cancer cells in a subject according to claim 88, wherein the exogenous payload gene encodes a therapeutic protein or polypeptide; the therapeutic protein or polypeptide is at least one selected from the group consisting of antibody-based drugs, chimeric antigen receptors, T cell receptors, cytokine receptors, cytokines, Fc fusion proteins, anticoagulants, blood factors, bone morphogenetic proteins, engineered protein scaffolds, enzymes, growth factors, hormones, interferons, interleukins and thrombolytic agents.

90. The method for treating cancer in a subject or killing cancer cells in a subject according to claim 89, wherein the exogenous payload gene encodes the chimeric antigen receptor.

91. The method according to any one of claims 88 to 90, wherein the administration is at least one selected from the group consisting of oral, nasal, intravenous, intraperitoneal, intracerebral (intraparenchymal), intraventricular, intramuscular, intraocular, intra-arterial, portal vein, intralesional, sustained release system and implant device administration.

92. A cell, **characterized in that** the cell is configured to produce the viral particle according to any one of claims 1 to 68.

93. The cell according to claim 92, wherein the cell is at least one selected from the group consisting of NS0 cells, Vero cells, HeLa cells, COS cells, CHO cells, HEK cells, BHK cells and MDCKII cells; and
preferably, the cell is an HEK-293T cell.

94. A method for preparing a viral particle, **characterized in that** the method comprises culturing the cell according to claim 92 or 93 to a degree sufficient to produce the viral particle.

95. A method for transducing a T cell, **characterized in that** the method comprises contacting a T cell with the viral particle according to any one of claims 1 to 68 carrying an exogenous payload gene.

96. The method according to claim 95, wherein the exogenous payload gene encodes a therapeutic protein or polypeptide; and the therapeutic protein or polypeptide is at least one selected from the group consisting of antibody-based drugs, chimeric antigen receptors, T cell receptors, cytokine receptors, cytokines, Fc fusion proteins, anticoagulants, blood factors, bone morphogenetic proteins, engineered protein scaffolds, enzymes, growth factors, hormones, interferons, interleukins and thrombolytic agents.

97. The method according to claim 96, wherein the exogenous payload gene encodes the chimeric antigen receptor.

98. The method according to any one of claims 95 to 97, wherein the T cell comprises activated T cells and non-activated T cells.

99. The method according to any one of claims 95 to 98, wherein the contact occurs *in vivo* and/or *in vitro* in a subject; and the subject is an individual to which T cells transduced by the method for transducing the T cells and/or the viral particle according to any one of claims 1 to 68 carrying the exogenous payload gene is administered.

100. The method according to claim 99, wherein the contact occurs *in vivo* in the subject, and the subject is an individual to which the viral particle according to any one of claims 1 to 68 carrying the exogenous payload gene is administered.

101. The method according to claim 100, wherein the viral particle is the viral particle according to any one of claims 48 to 68.

102. The method according to any one of claims 95 to 101, wherein the administration is at least one selected from the group consisting of oral, nasal, intravenous, intraperitoneal, intracerebral (intraparenchymal), intraventricular, intramuscular, intraocular, intra-arterial, portal vein, intralesional, sustained release system and implant device administration.
